# EUROPEAN PATENT SPECIFICATION

(11) **EP 4 185 701 B1**
(45) Date of publication and mention of the grant of the patent: **02.12.2026**
(21) Application number: 21845270.4
(22) Date of filing: 22.07.2021
(51) Int. Cl.: C12N 15/86, A61P 27/00, A61K 48/00

(54) **VARIANT TXNIP COMPOSITIONS AND METHODS OF USE THEREOF FOR THE TREATMENT OF DEGENERATIVE OCULAR DISEASES**
VARIANTEN VON TXNIP-ZUSAMMENSETZUNGEN UND VERFAHREN ZUR VERWENDUNG DAVON ZUR BEHANDLUNG VON DEGENERATIVEN AUGENERKRANKUNGEN
COMPOSITIONS DE VARIANTS TXNIP ET LEURS PROCÉDÉS D'UTILISATION POUR LE TRAITEMENT DE MALADIES OCULAIRES DÉGÉNÉRATIVES

(30) Priority: 23.07.2020 US 202063055413 P; 15.01.2021 US 202163137911 P
(43) Date of publication of application: 31.05.2023
(73) Proprietor: President and Fellows of Harvard College, Cambridge, MA 02138 (US)
(72) Inventor: XUE, Yunlu, Cambridge, MA 02138 (US); CEPKO, Constance L., Cambridge, MA 02138 (US)
(74) Representative: Carpmaels & Ransford LLP
(86) International application number: PCT/US2021/042697
(87) International publication number: WO 2022/020548

(56) References cited:
- WO-A1-2014/186160
- WO-A1-2014/186160
- WO-A1-2020/132040
- US-A1- 2015 259 395
- SEAN K. WANG ET AL: "Soluble CX3CL1 gene therapy improves cone survival and function in mouse models of retinitis pigmentosa", PROCEEDINGS OF THE NATIONAL ACADEMY OF SCIENCES, vol. 116, no. 20, 29 April 2019 (2019-04-29), pages 10140 - 10149, XP055729791, ISSN: 0027-8424, DOI: 10.1073/pnas.1901787116
- PATWARI PARTH ET AL: "Thioredoxin-independent Regulation of Metabolism by the [alpha]-Arrestin Proteins", JOURNAL OF BIOLOGICAL CHEMISTRY, vol. 284, no. 37, 1 September 2009 (2009-09-01), US, pages 24996 - 25003, XP055930244, ISSN: 0021-9258, DOI: 10.1074/jbc.M109.018093
- XUE YUNLU ET AL: "AAV-Txnip prolongs cone survival and vision in mouse models of retinitis pigmentosa", vol. 10, 13 April 2021 (2021-04-13), XP093099208, Retrieved from the Internet <URL:https://cdn.elifesciences.org/articles/66240/elife-66240-v2.xml> DOI: 10.7554/eLife.66240
- WANG ET AL.: "Soluble CX3CL1 gene therapy improves cone survival and function in mouse models of retinitis pigmentosa", PNAS, vol. 116, no. 20, 14 May 2019 (2019-05-14), pages 10140 - 10149, XP055729791, DOI: 10.1073/pnas.1901787116
- PATWARI ET AL.: "Thioredoxin-independent Regulation of Metabolism by the alpha-Arrestin Proteins", JOURNAL OF BIOLOGICAL CHEMISTRY, vol. 284, no. 37, 11 September 2009 (2009-09-11), pages 24996 - 25003, XP055930244

## Description

### RELATED APPLICATIONS

This application claims the benefit of priority to U.S. Provisional Application No. 63/055,413, filed on July 23, 2020, and U.S. Provisional Application No. 63/137,911, filed on January 15, 2021.

### SEQUENCE LISTING

The instant application contains a Sequence Listing which has been submitted electronically in ASCII format. Said ASCII copy, created on July 20, 2021, is named 117823_30520_SL.txt and is 313,849 bytes in size.

### BACKGROUND OF THE INVENTION

Retinitis pigmentosa (RP) is a disease of the eye that presents with progressive degeneration of rod and cone photoreceptors, the light-sensing cells of the retina (Hartong DT, et al. (2006) Lancet 368(9549):1795-1809). The disease can result from mutations in any of over 60 different genes and is the most common inherited form of blindness in the world, affecting an estimated 1 in 4000 individuals (Daiger SP, et al. (2013) Clin Genet 84(2): 132-141; Berson EL (1996) Proc Natl Acad Sci U S A 93(10):4526-8; Haim M (2002) Acta Ophthalmol Scand Suppl (233):1-34). One approach to treat this disease is gene therapy, e.g. using adeno-associated vectors (AAVs) to deliver a wild-type allele to complement a mutated gene (Ali RR, et al. (1996) Hum Mol Genet 5(5):591-4; Murata T, et al. (1997) Ophthalmic Res 29(5):242-251). While this approach has proven successful in other conditions, even leading to the approval of a gene therapy for RPE65-associated Leber's congenital amaurosis (Maguire AM, et al. (2008) N Engl J Med 358(21):2240-2248), it is difficult to implement for the majority of RP patients, given the extensive heterogeneity of genetic lesions (Daiger SP, et al. (2013) Clin Genet 84(2): 132-141). A broadly applicable gene therapy that is agnostic to the genetic lesion would provide a treatment option for a greater number of RP patients. Presently, there is no effective therapy of any kind for RP, and despite more than a dozen randomized clinical trials to date, none have been able to demonstrate an improvement in visual function (Sacchetti M, et al. 2015) J Ophthalmol 2015:737053).

In patients with RP, there is an initial loss of rods, the photoreceptors that mediate vision in dim light. Clinically, this results in the first manifestation of RP, poor or no night vision, which usually occurs between birth and adolescence (Hartong DT, et al. (2006) Lancet 368(9549):1795-1809). Daylight vision in RP is largely normal for decades, but eventually deteriorates beginning when most of the rods have died. This is due to dysfunction, and then death, of the cone photoreceptors, which are essential for high acuity and color vision. Loss of cone function is the major source of morbidity in the disease (Hartong DT, et al. (2006) Lancet 368(9549):1795-1809). Importantly, while the vast majority of genes implicated in RP are expressed in rods, few actually exhibit expression in cones, suggesting the existence of one or more common mechanisms by which diverse mutations in rods trigger non-autonomous cone degeneration (Narayan DS, et al. (2016) Acta Ophthalmol 94(8):748-754; Wang W, et al. (2016) Cell Rep 15(2):372-85; Komeima K, et al. (2006) Proc Natl Acad Sci U S A 103(30):11300-5). Attempts to elucidate these mechanisms have been made with the goal of developing therapies for RP that preserve cone vision regardless of the underlying mutation (Punzo C, et al. (2009) Nat Neurosci 12(1):44-52; Xiong W, et al. (2015) J Clin Invest 125(4):1433-1445; Venkatesh A, et al. (2015) J Clin Invest 125(4): 1446-58; Ait-Ali N, et al. (2015) Cell 161(4):817-832; Murakami Y, et al. (2012) Proc Natl Acad Sci 109(36):14598-14603*).*

Accordingly, there is a need in the art for therapies to treat and prevent vision loss that results from degenerative retinal diseases, such as RP.

### SUMMARY OF THE INVENTION

Any references to methods for treatment of the human or animal body by surgery or therapy are to be interpreted as references to the compounds, pharmaceutical compositions, and medicaments disclosed herein for use in those methods.

The present invention is based, at least in part on the discovery of mutation-independent compositions and methods of treatment for subjects having retinitis pigmentosa (RP).

More specifically, it has surprisingly been discovered that intraocular delivery of adeno-associated virus (AAV) comprising a thioredoxin interacting protein (TXNIP) variant that cannot bind thioredoxin (C247S) prolongs survival of cones in RP-mutant mice. Even more surprising, this C247S variant TXNIP-mediated effect was only observed when the TXNIP variant was specifically expressed in cones. In addition, it has been surprisingly discovered that a serine at amino acid residue 308 is required for this effect as replacing this residue with alanine abolishes the enhanced survival of cones resulting from the C247S variant. It has also surprisingly been discovered that overexpression of C247S variant TXNIP increases RP cone mitochondria size and function. Further, it has been supriringly discoved that overexpression of C247S variant TXNIP in combination with overexpression of nuclear factor erythroid 2-like 2 (Nrf2) prolongs survival of cones in RP-mutant mice further than overexpression of either C247S TXNIP or Nrf2 alone.

The present invention is defined by the appended claims.

In one aspect, the present invention provides a composition, comprising an adeno-associated virus (AAV) expression cassette, comprising a photoreceptor-specific (PR- specific) promoter, such as a cone-specific promoter, and a nucleic acid molecule encoding a C247S variant thioredoxin-interacting protein (TXNIP).

The PR-specific promoter is a human red opsin (hRedO) promoter.

In one embodiment, the hRedO promoter comprises nucleotides 452-2017 of SEQ ID NO:8 directly linked, *i.e.,* containing no intervening sequences, to nucleotides 4541-5032 of SEQ ID NO:8; or a nucleotide sequence having about 85%, 86%, 87%, 88%, 89%, 90%, 91%, 92%, 93%, 94%, 95%, 96%, 97%, 98%, or about 99% nucleotide sequence identity to the entire nucleotide sequence of nucleotides 452-2017 of SEQ ID NO:8 directly linked to nucleotides 4541-5032 of SEQ ID NO:8.

In another embodiment, the hRedO promoter comprises the nucleotide sequence of SEQ ID NO:16, or a nucleotide sequence having about 85%, 86%, 87%, 88%, 89%, 90%, 91%, 92%, 93%, 94%, 95%, 96%, 97%, 98%, or about 99% nucleotide sequence identity to the entire nucleotide sequence of SEQ ID NO:16.

In one embodiment, the hRedO promoter comprises nucleotides 457-2514 of SEQ ID NO:26, or a nucleotide sequence having about 85%, 86%, 87%, 88%, 89%, 90%, 91%, 92%, 93%, 94%, 95%, 96%, 97%, 98%, or about 99% nucleotide sequence identity to the entire nucleotide sequence of nucleotides 457-2514 of SEQ ID NO:26.

In another embodiment, the hRedO promoter comprises nucleotides 457-2514 of SEQ ID NO: 49, or a nucleotide sequence having about 85%, 86%, 87%, 88%, 89%, 90%, 91%, 92%, 93%, 94%, 95%, 96%, 97%, 98%, or about 99% nucleotide sequence identity to the entire nucleotide sequence of nucleotides 457-2514 of SEQ ID NO: 49.

In another embodiment, the hRedO promoter comprises the nucleotide sequence of SEQ ID NO:119, or a nucleotide sequence having about 85%, 86%, 87%, 88%, 89%, 90%, 91%, 92%, 93%, 94%, 95%, 96%, 97%, 98%, or about 99% nucleotide sequence identity to the entire nucleotide sequence of SEQ ID NO:119.

In one embodiment, the nucleic acid molecule encoding the C247S variant TXNIP comprises nucleotides 366-1541 of SEQ ID NO:111; or a nucleotide sequence having about 85%, 86%, 87%, 88%, 89%, 90%, 91%, 92%, 93%, 94%, 95%, 96%, 97%, 98%, or about 99% nucleotide sequence identity to the entire nucleotide sequence of nucleotides 366-1541 of SEQ ID NO:111.

In another embodiment, the nucleic acid molecule encoding the C247S variant TXNIP comprises nucleotides 162-1172 of SEQ ID NO:112, or a nucleotide sequence having about 85%, 86%, 87%, 88%, 89%, 90%, 91%, 92%, 93%, 94%, 95%, 96%, 97%, 98%, or about 99% nucleotide sequence identity to the entire nucleotide sequence of nucleotides 162-1172 of SEQ ID NO:112.

In yet another embodiment, the nucleic acid molecule encoding the C247S variant TXNIP comprises nucleotides 280-1473 of SEQ ID NO:113; or a nucleotide sequence having about 85%, 86%, 87%, 88%, 89%, 90%, 91%, 92%, 93%, 94%, 95%, 96%, 97%, 98%, or about 99% nucleotide sequence identity to the entire nucleotide sequence of nucleotides 280-1473 of SEQ ID NO:113.

In one embodiment, the nucleic acid molecule encoding the C247S variant TXNIP comprises nucleotides 280-1470 of SEQ ID NO:114, or a nucleotide sequence having about 85%, 86%, 87%, 88%, 89%, 90%, 91%, 92%, 93%, 94%, 95%, 96%, 97%, 98%, or about 99% nucleotide sequence identity to the entire nucleotide sequence of nucleotides 280-1470 of SEQ ID NO:114.

In one embodiment, the nucleic acid molecule encoding TXNIP comprises SEQ ID NO: 120; or a nucleotide sequence having about 85%, 86%, 87%, 88%, 89%, 90%, 91%, 92%, 93%, 94%, 95%, 96%, 97%, 98%, or about 99% nucleotide sequence identity to the entire nucleotide sequence of SEQ ID NO:120.

In one embodiment, the nucleic acid molecule encodes a C247S .LL351.352AA variant thioredoxin-interacting 5 protein (TXNIP).

In one embodiment, the nucleic acid molecule encoding TXNIP comprises SEQ ID NO: 115; or a nucleotide sequence having about 85%, 86%, 87%, 88%, 89%, 90%, 91%, 92%, 93%, 94%, 95%, 96%, 97%, 98%, or about 99% nucleotide sequence identity to the entire nucleotide sequence of SEQ ID NO:115.

In one embodiment, the nucleic acid molecule encoding TXNIP comprises SEQ ID NO: 121; or a nucleotide sequence having about 85%, 86%, 87%, 88%, 89%, 90%, 91%, 92%, 93%, 94%, 95%, 96%, 97%, 98%, or about 99% nucleotide sequence identity to the entire nucleotide sequence of SEQ ID NO:121.

In one embodiment, the expression cassette further comprises a linker, such as a 2A linker.

In one embodiment, the expression cassette further comprises an intron.

In one embodiment, the expression cassette further comprises a post-transcriptional regulatory region.

In another embodiment, the expression cassette further comprises a Woodchuck hepatitis virus posttranscriptional regulatory element (WPRE).

In one embodiment, the expression cassette further comprises a polyadenylation signal.

In one embodiment, the polyadenylation signal is a bovine growth hormone polyadenylation signal or an SV40 polyadenylation signal.

In one embodiment, the expression cassette is present in a vector.

In one embodiment, the vector is an AAV vector selected from the group consisting of AAV2, AAV 8, AAV2/5, and AAV 2/8.

The present invention also provides AAV vector particles and pharmaceutical compositions comprising the AAV compositions of the invention and isolated cells comprising the AAV particles of the invention.

In one embodiment, the pharmaceutical compositions of the invention further comprise a viscosity inducing agent.

In one embodiment, the pharmaceutical compositions of the invention are for intraocular administration.

In one embodiment, the intraocular administration is selected from the group consisting of intravitreal or subretinal, subvitreal, subconjuctival, sub-tenon, periocular, retrobulbar, suprachoroidal, and/or intrascleral administration.

In one aspect, the compositions of the present invention are for use in a method for prolonging the viability of a photoreceptor cell compromised by a degenerative ocular disorder. The method includes contacting the cell with any one or more of the AAV composition or the pharmaceutical composition of the invention, or the AAV viral particle of the invention, thereby prolonging the viability of the photoreceptor cell compromised by the degenerative ocular disorder.

In another aspect, the compositions of the present invention are for use in a method for treating or preventing a degenerative ocular disorder in a subject. The methods includes administering to the subject a therapeutically effective amount of any one or more of the AAV composition or the pharmaceutical composition of the invention, or the AAV viral particle of the invention, thereby treating or preventing said degenerative ocular disorder.

In another aspect, the compositions of the present invention are for use in a method for delaying loss of functional vision in a subject having a degenerative ocular disorder. The methods includes administering to the subject a therapeutically effective amount of any one or more of the AAV composition or the pharmaceutical composition of the invention, or the AAV viral particle of the invention, thereby treating or preventing said degenerative ocular disorder.

In one embodiment, the degenerative ocular disorder is associated with decreased viability of cone cells and/or decreased viability of rod cells.

In one embodiment, the degenerative ocular disorder is selected from the group consisting of retinitis pigmentosa, age related macular degeneration, cone rod dystrophy, and rod cone dystrophy.

In one embodiment, the degenerative ocular disorder is a genetic disorder.

In one embodiment, the degenerative ocular disorder is not associated with blood vessel leakage and/or growth.

In one embodiment, the degenerative ocular disorder is retinitis pigmentosa.

In one aspect, the compositions of the present invention are for use in a method for treating or preventing retinitis pigmentosa in a subject. The methods includes administering to the subject a therapeutically effective amount of the composition of any one or more of the AAV composition or the pharmaceutical composition of the invention, or the AAV viral particle of the invention, thereby treating or preventing retinitis pigmentosa in said subject.

In one embodiment, the methods further comprise administering to the subject a therapeutically effective amount of a composition comprising an adeno-associated virus (AAV) expression cassette, the expression cassette comprising a human bestrophin 1 (hBest1) promoter, a chimeric intron, and a nucleic acid molecule encoding nuclear factor erythroid 2-like 2 (Nrf2), or a pharmaceutical comprising an adeno-associated virus (AAV) expression cassette, the expression cassette comprising a human red opsin (hRedO) promoter and a nucleic acid molecule encoding transforming growth factor beta 1 (Tgfb1).

Other features and advantages of the invention will be apparent from the following detailed description and claims.

### BRIEF DESCRIPTION OF THE DRAWINGS

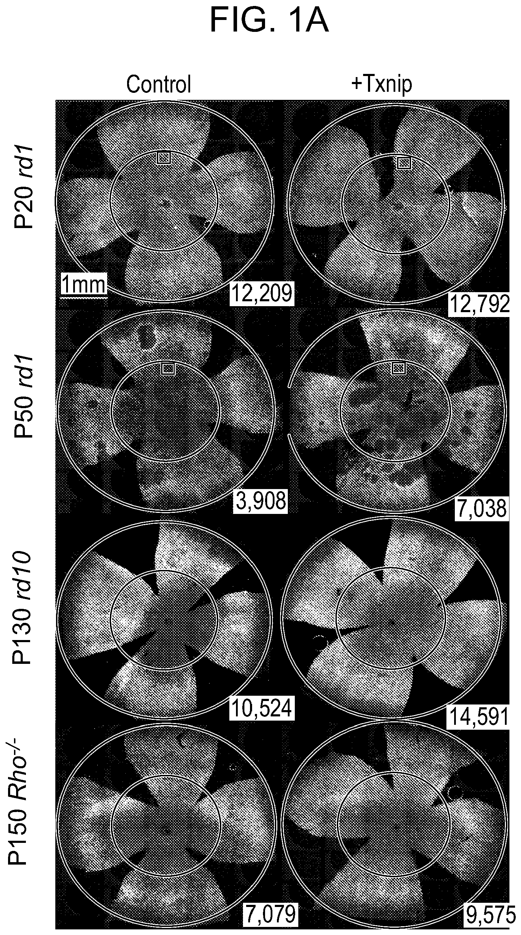

**FIGS. 1A-1C** show that Txnip expression enhances cone survival and delays the deterioration of cone-mediated vision in RP mice.

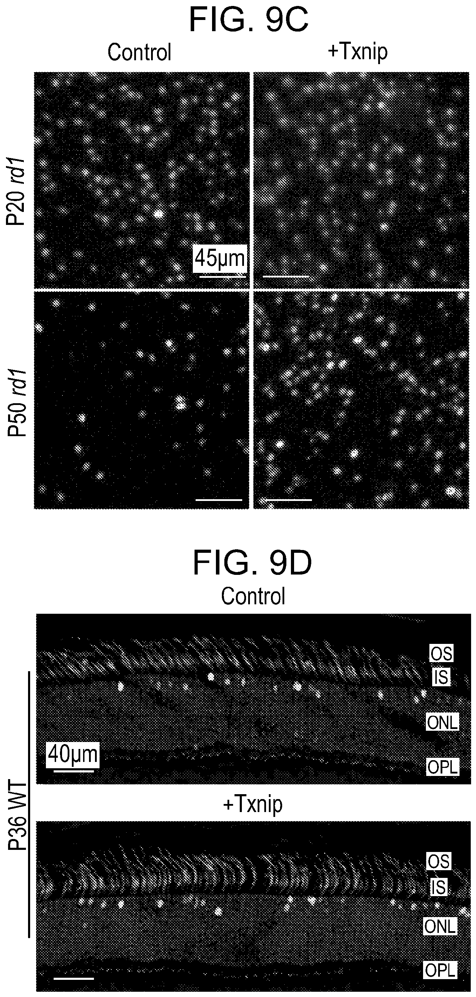

**FIG. 1A** are representative images from P20 and P50 *rd1,* P130 *rd10* and P150 *rho*^{*-*/*-*} flat-mounted retinas, in which cones are labeled with H2BGFP, treated with Txnip or control *(i.e.* H2BGFP and vehicle only, same applies to all other figures). The outer circle was drawn to mark the full extent of the retina, and the inner circle was drawn by using half of the radius of the outer circle. The small box in the top four panels are zoomed in with pixels recognized as cones by a MATLAB automated-counting program **(****FIG. 9C****).** The number at lower right corner is the count of cells within the half radius of each image (same applies to all other figures).
**FIG. 1B** are graphs depicting the quantification of H2BGFP-positive cones within the inner half of the retina at different groups. Error bar: standard deviation. The number in round brackets "( )" indicates the sample size, *i.e.* the number of eyes/retinas within each group (same applies to all other figures).
**FIG. 1C** are graphs depicting the visual acuity of *rd10* and P140 *rho*^{*-*/*-*} mice with Txnip or control treatment in each eye measured with optomotor assays. Error bar: SEM. NS: not significant, p > 0.05. ** p <* 0.05. ** *p* < 0.01. *** *p* < 0.001. **** *p* < or << 0.0001.
**FIGS. 2A-2B** show the results of expression of Txnip alleles on cone survival.

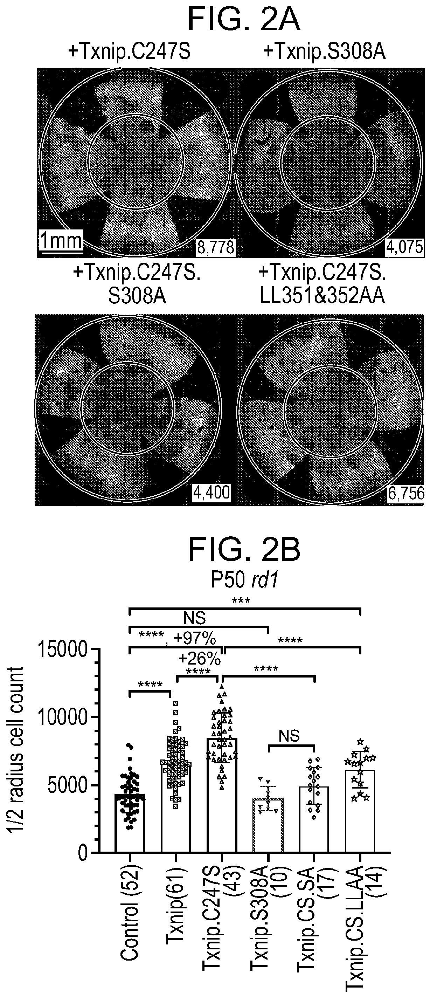

**FIG. 2A** are representative P50 *rd1* flat-mounted retinas with H2BGFP (gray) labeled cones treated with one of four different Txnip alleles.
**FIG. 2B** is a graph depicting the quantification of H2BGFP-positive cones within the half radius of P50 *rd1* retinas treated with wildtype (wt) Txnip, Txnip alleles, and control. Error bar: standard deviation. Abbreviations: Txnip.CS.SA = Txnip.C247S.S308A; Txnip.CS.LLAA = Txnip.C247S.LL351&352AA. NS: not significant, p *>* 0.05. ** p <* 0.05. *** p <* 0.01. **** p <* 0.001. **** *p* < or << 0.0001.

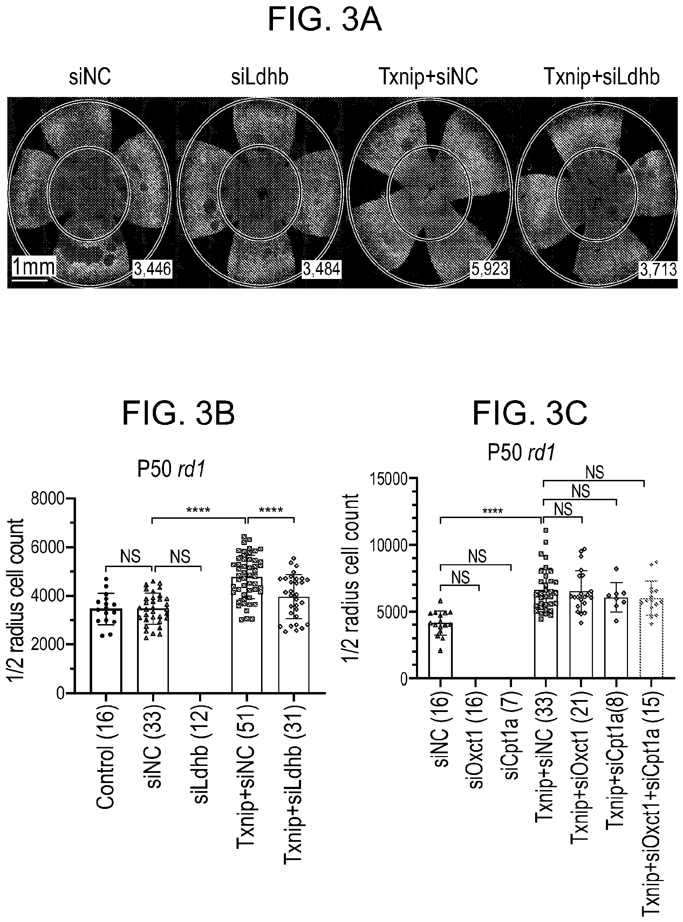

**FIGS. 3A-3C** show that Ldhb expression is necessary for Txnip-induced rescue of RP cones *in vivo.*
**FIG. 3A** are representative P50 *rd1* flat-mounted retinas with H2BGFP (gray) labeled cones treated with siNC (non-targeting scrambled control shRNA), siLdhb^{(#2)} (Ldhb shRNA), Txnip + siNC, or Txnip + siLdhb^{(#2)}.
**FIG. 3B** is a graph depicting the quantification of H2BGFP-positive cones within the half radius of P50 *rd1* retinas treated with control, siNC control, Txnip + siLdhb^{(#2)} or siNC control.
**FIG. 3C** is a graph depicting quantification of H2BGFP-positive cones within the half radius of P50 *rd1* retinas treated with Txnip + siOxct1^{(#c)}, Txnip + siCpt1a^{(#c)}, Txnip + siOxct1^{(#c)} + siCpt1a^{(#c)}, or siNC control. Error bar: standard deviation. NS: not significant, *p >* 0.05. *** p < 0.01.* *** p < 0.001. **** p < or << 0.0001.

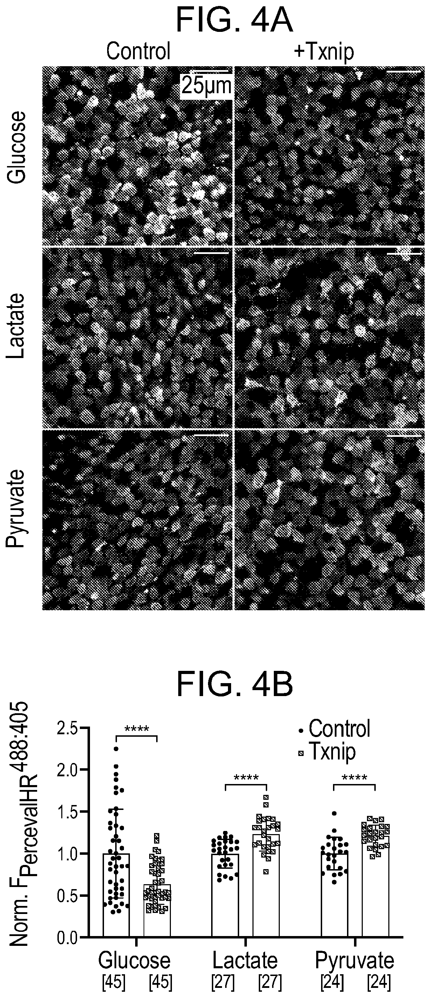

**FIGS. 4A-4E** show that Txnip expression increases ATP:ADP levels in RP cones in lactate medium.
**FIG. 4A** are representative *ex vivo* live images of PercevalHR labeled cones in P20 *rd1* retinas cultured with high-glucose, lactate-only, or pyruvate-only medium and treated with Txnip or control. (Medium gray: fluorescence by 405 nm excitation, indicating low-ATP:ADP. Light gray: fluorescence by 488 nm excitation, indicating high-ATP:ADP.)
**FIG. 4B** is agraph depicting the quantification of normalized PercevalHR fluorescence intensity ratio (F_{PereevalHR}^{ex488nm : ex405nm}, proportional to ATP:ADP ratio) in cones from P20 *rd1* retinas in different conditions. The number in the bracket "[ ]" indicates the sample size, *i.e.* the number of images taken from regions of interest of multiple retinas (≈3 images per retina), in each condition (same applies to all other figures).

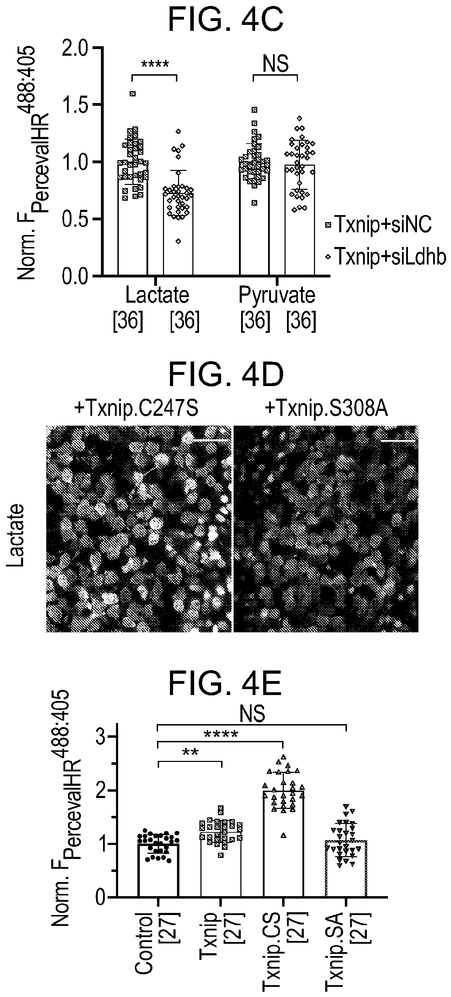

**FIG. 4C** is a graph depicting the quantification of normalized PercevalHR fluorescence intensity of Txnip + siLdhb^{(#2)} and Txnip + siNC in cones from P20 *rd1* retina in lactate-only or pyruvate-only medium.
**FIG. 4D** are representative *ex vivo* live images of PercevalHR labeled cones in P20 *rd1* retinas cultured in lactate-only medium, following treatment with Txnip.C247S or Txnip.S308A. (Medium gray: fluorescence by 405 nm excitation, indicating low-ATP:ADP. Light gray: fluorescence by 488 nm excitation, indicating high-ATP:ADP.)
**FIG. 4E** is a graph depicting the quantification of normalized PercevalHR fluorescence intensity following treatment by Txnip, Txnip alleles, and control cones in *P20 rd1* retinas cultured in lactate-only medium. Abbreviations: Txnip.CS = Txnip.C247S; Txnip.SA = Txnip.S308A. Error bar: standard deviation. NS: not significant, p *>* 0.05. *** p <* 0.01. **** p <* 0.001. ***** p* < or << 0.0001.

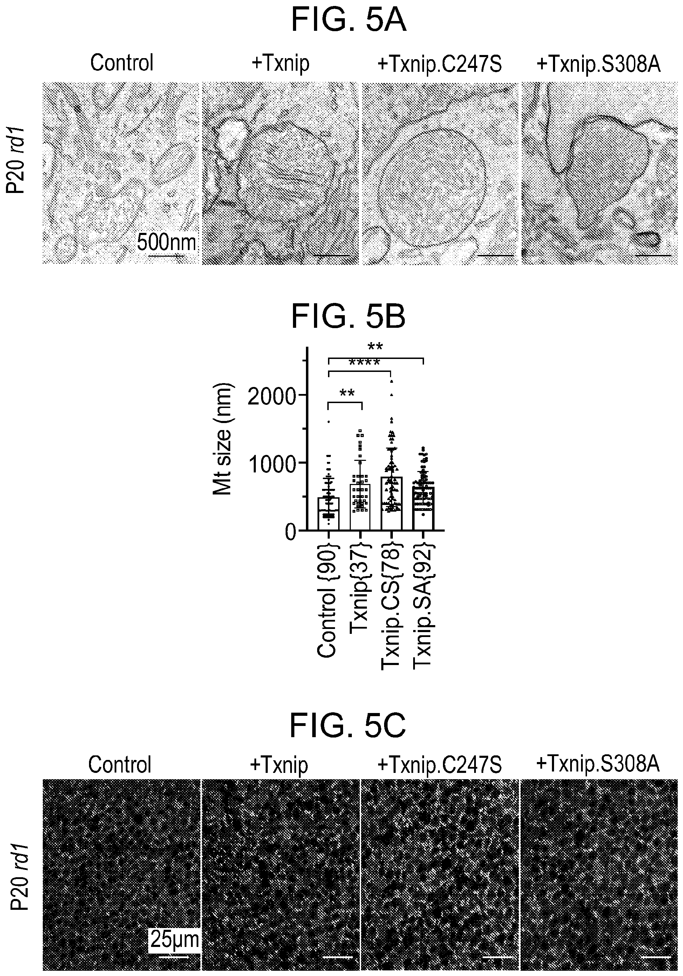

**FIGS. 5A-5J** shows that Txnip expression enhances RP cone mitochondrial size and function.
**FIG. 5A** are representative EM images of RP cones from P20 *rd1* cones treated with Txnip, Txnip.C247S, Txnip.S308A, and control.
**FIG. 5B** is a graph depicting the quantification of mitochondrial diameters from control, Txnip, Txnip.C247S and Txnip.S308A treated cones. The number in the curly bracket "{ }" indicates the sample size, *i.e.* the number of mitochondria from multiple cones of ≥ one retina for each condition (5 retinas for control, 4 for Txnip, 2 for Txnip.C247S, and 1 for Txnip.S308A).
**FIG. 5C** are images of JC-1 dye staining (indicator of ETC function) in live cones of P20 *rd1* central retina at different conditions. (Magenta: J-aggregate, indicating high ETC function. Green: JC-1 monomer, for self-normalization. H2BGFP channel, the tracer of AAV infected area, is not shown.)

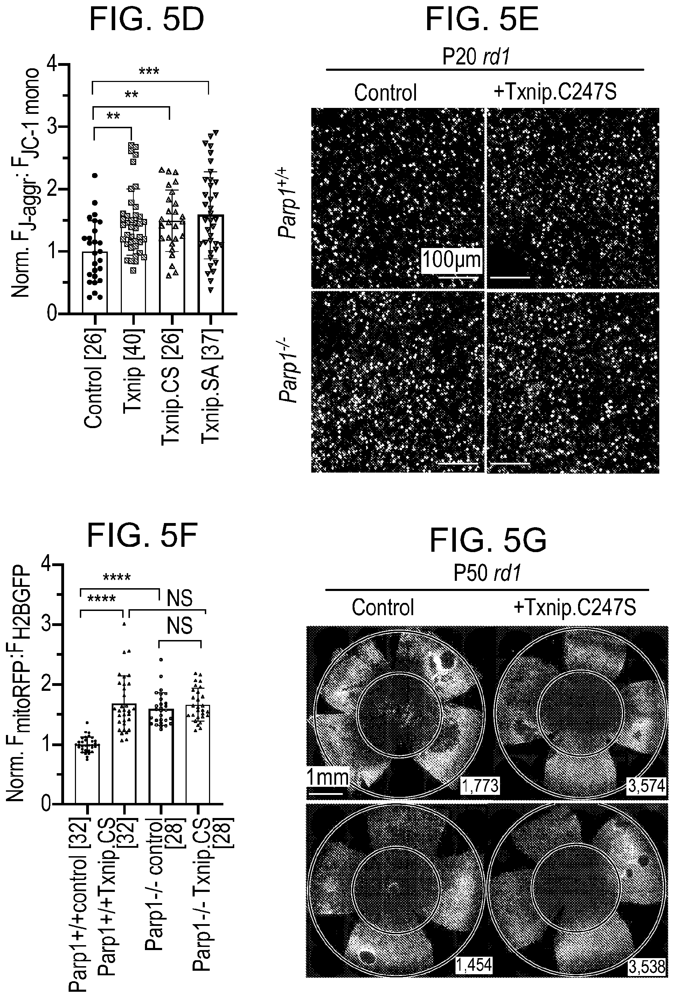

**FIG. 5D** is a graph depicting the quantification of normalized cone JC-1 dye staining (fluorescence intensity of J-aggregate:JC-1 monomer) from live cones in P20 *rd1* retinas in different conditions (3 - 4 images per retina).
**FIG. 5E** is images of mitoRFP staining (reflecting mitochondrial function) in Txnip.C247S and control cones from fixed P20 *parp1*^{*+*/}*⁺ rd1* and *parp1*^{*-*/}*⁻ rd1* retinas near the optic nerve head. (Medium gray: mitoRFP. Light grayray: H2BGFP, for mitoRPF normalization.)
**FIG. 5F** is a graph depicting the quantification of normalized mito-RFP:H2BGFP intensity in different conditions of P20 *parp1 rd1* retinas (4 images per retina, near optical nerve head).
**FIG. 5G** are images of P50 *parp1*^{*+*/}*⁺ rd1* and *parpl*^{*-*/}*⁻ rd1* retinas with H2BGFP (gray) labeled cones treated with Txnip.C247S or control. *Rd1* cone degeneration seems to be faster after being crossed to *parp1* mice (on 129S background) due to unknown reason(s).

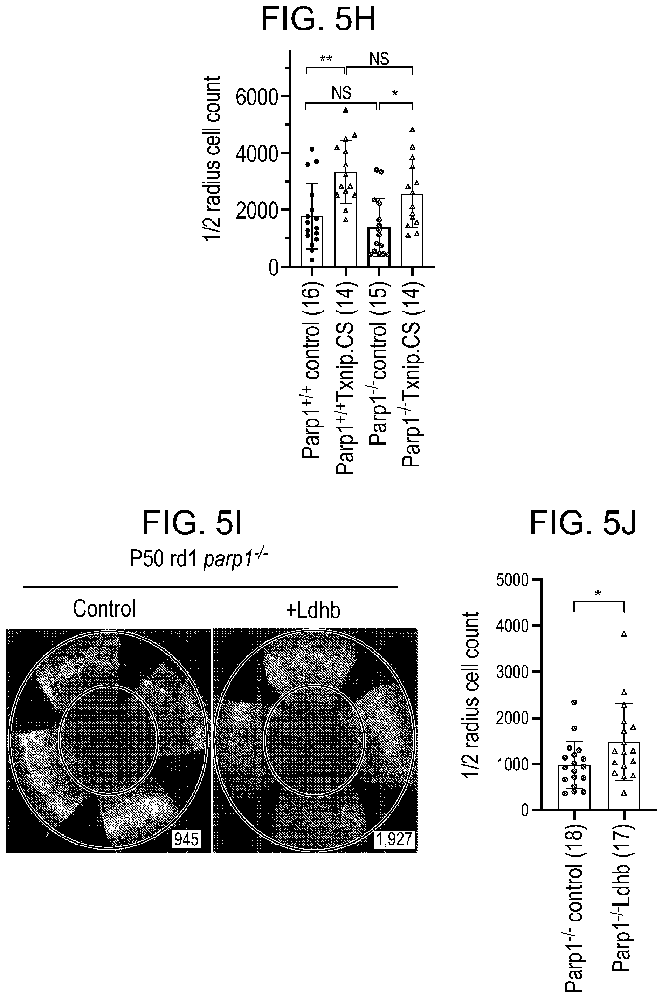

**FIG. 5H** is a graph depicting the quantification of H2BGFP-positive cones within the half radius of P50 *parp1*^{*+*/}*⁺ rd1* and *parp1*^{*-*/}*⁻ rd1* retinas treated with Txnip.C247S or control.
**FIG. 5I** are images of P50 *parp1*^{*-*/}*⁻ rd1* retinas with H2BGFP (gray) labeled cones treated with Ldhb or H2BGFP only.
**FIG. 5J** is a graph depicting the quantification of H2BGFP-positive cones within the half radius of P50 *parp1*^{*-*/}*⁻ rd1* retinas treated with Ldhb or H2BGFP only. Error bar: standard deviation. NS: not significant, p > 0.05. * *p* < 0.05. *** p* < 0.01. *** p < 0.001. **** p < or << 0.0001. Abbreviations: Txnip.SA = Txnip; Txnip.SA = Txnip.S308A.

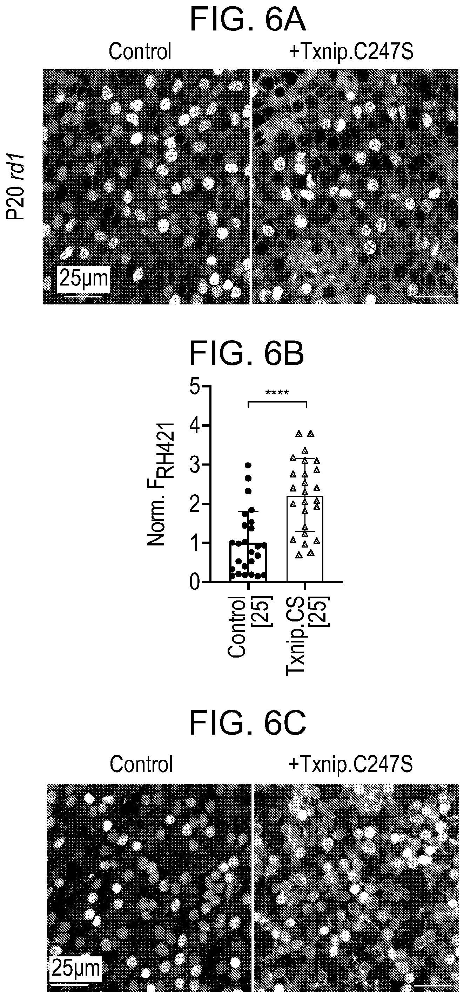

**FIGS. 6A-6C** show that Txnip expression enhances Na⁺/K⁺ ATPase pump function and cone opsin expression in RP cones.
**FIG. 6A** are images of live *ex vivo* RH421 stained cones in P20 *rd1* retinas treated with Txnip.C247S or control and cultured in lactate-only medium. (Magenta: RH421 fluorescence, proportional to Na⁺/K⁺ ATPase function. Gray: H2BGFP, tracer of infection).
**FIG. 6B** is a graph depicting the quantification of normalized RH421 fluorescence intensity from Txnip.C247S treated cones relative to control in P20 *rd1* retinas cultured in lactate-only medium (5 images per retina). Abbreviation: Txnip.CS = Txnip.C247S.
**FIG. 6C** are immunohistochemical staining (IHC) images with anti-s-opsin plus anti-m-opsin antibodies near the half radius of P50 *rd1* retinas treated with Txnip.C247S or control. (Medium gray: cone-opsins. Light gray: H2BGFP, tracer of infection).

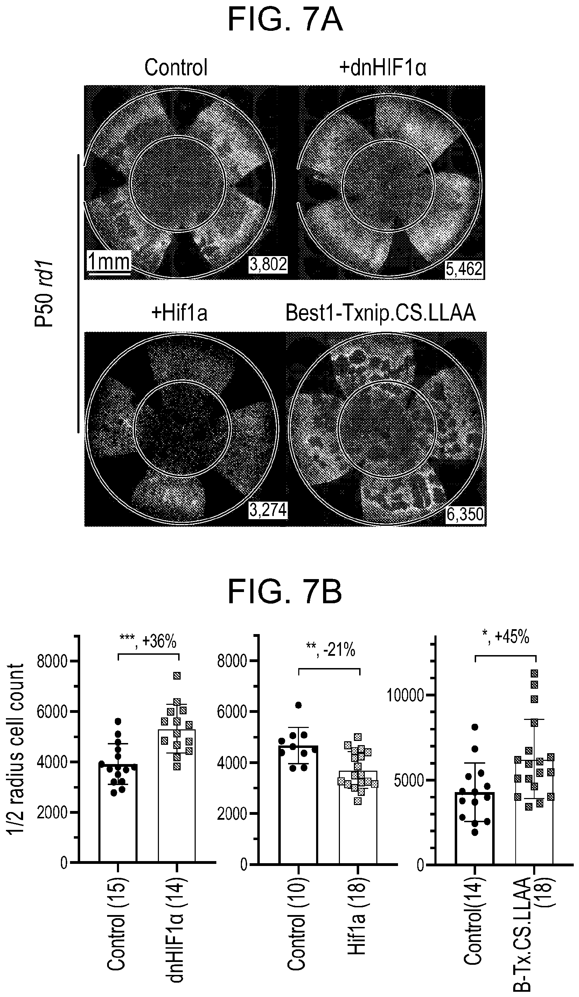

**FIGS. 7A-7B** show that Best1 Txnip.C247S.LL351&352AA and dominant negative HIF1α enhance RP cone survival.
**FIG. 7A** are simages of P50 *rd1* retinas with H2BGFP (gray) labeled cones treated with dnHIF1α, Hifla, Best1-Txnip.C247S.LL351&352AA (Txnip.CS.LLAA, driven by an RPE-specific promoter) or control. Note that Best1-Txnip.C247S.LL351&352AA amplified the FVB-specific retinal craters, while dnHIF1α decreased them.
**FIG. 7B** are graphs depicting the quantification of H2BGFP-positive cones within the half radius of P50 *rd1* retinas treated with dnHIF1α, Hifla, Best1-Txnip.C247S.LL351&352AA or control. Abbreviation: B-Tx.CS.LLAA = Best1-Txnip.C247S.LL351&352AA. Error bar: standard deviation. * *p <* 0.05. *** p* < 0.01. *** *p* < 0.001.

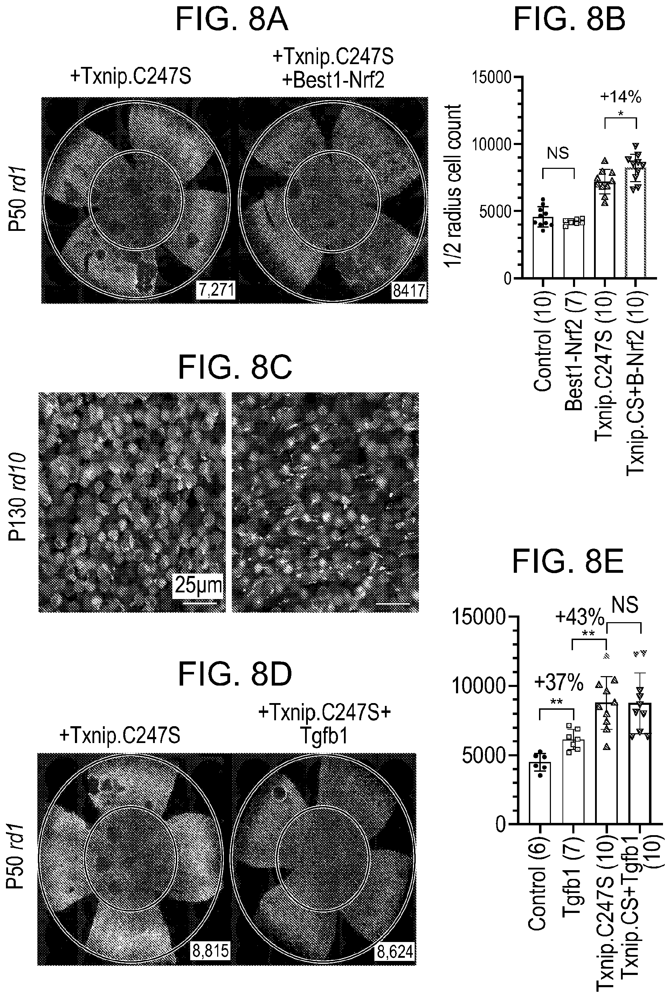

**FIGS. 8A-8E** shows that the combination of expression of Txnip.C247S with Best1-Nrf2 or RedO-Tgfb1 provides an additive effect.
**FIG. 8A** are images of P50 rd1 retinas with H2BGFP (light gray) labeled cones treated with Txnip.C247S or Txnip.C247S + Best1-Nrf2.
**FIG. 8B** is a graph depicting the quantification of H2BGFP-positive cones within the half radius of P50 *rd1* retinas treated with Txnip.C247S or Txnip.C247S + Best1-Nrf2. Abbreviation: B-Nrf2 = Best1-Nrf2.
**FIG. 8C** are IHC images with anti-s-opsin plus anti-m-opsin antibodies near the half radius of P130 *rd10* retinas treated with Txnip.C247S (left panel) or Txnip.C247S + Best1-Nrf2 (right panel). (Green: cone-opsins. Gray: H2BGFP, tracer of infection).
**FIG. 8D** are images of P50 rd1 retinas with H2BGFP (gray) labeled cones treated with Txnip.C247S or Txnip.C247S + Tgfbl.
**FIG. 8E****.** is a graph depicting the quantification of H2BGFP-labeled cones within the half radius of P50 *rd1* retinas treated with control, Tgfb1, Txnip.C247S, or Txnip.C247S + Tgfb1. Error bar: standard deviation. NS: not significant, p > 0.05. ** p <* 0.05. ** *p* < 0.01.

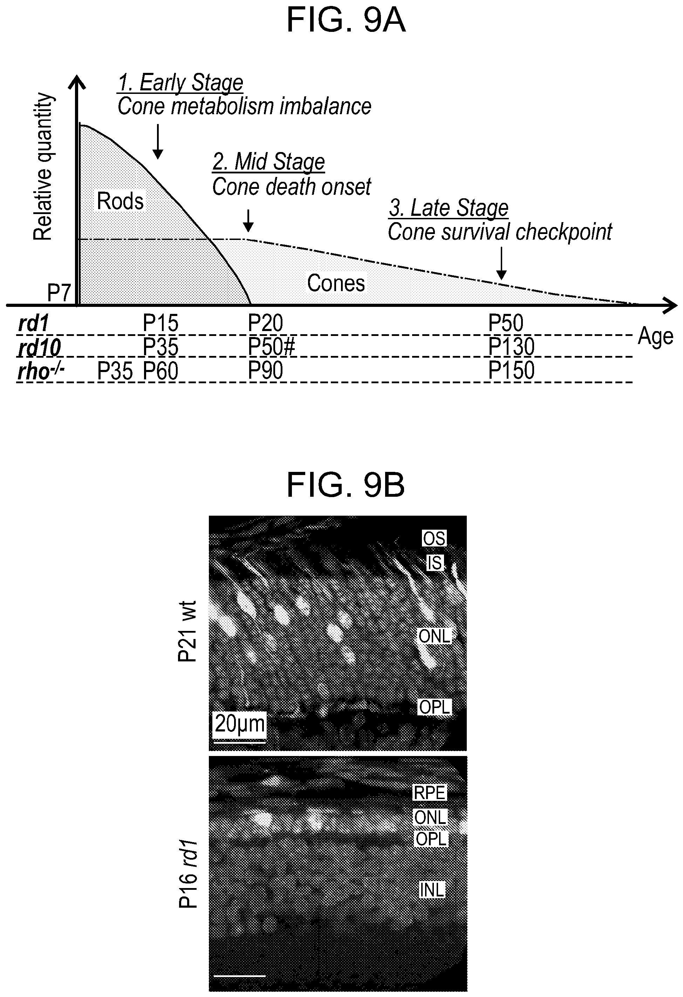

**FIGS. 9A-9E** are related to **FIGS. 1A-1C****.**
**FIG. 9A** is a schematic depicting photoreceptor degeneration in RP mice. # rd10 mid stage varies due to light-dependent rod degeneration (Chang *et al.,* 2007).
**FIG. 9B** are images depicting AAV8-RO1.7-GFP.Txnip expression in P21 wt (BALB/c) and P16 rd1 retina. (Light gray: GFP. Medium gray: PNA for cone extracellular matrix. Gray: DAPI.) Abbreviations. OS: outer segment, IS: inner segment, ONL: outer nuclear layer, OPL: outer plexiform layer, INL: inner nuclear layer, RPE: retinal pigmented epithelium.
**FIG. 9C** are images depicting pixels recognized as cones by a MATLAB automated-counting program zoomed in from the small boxes in the top four panels (Fig. 1a). (Gray: H2BGFP labeled cones. Medium gray: center of one labeled cell recognized by MATLAP program.)
**FIG. 9D** are images of P36 wildtype (C57BL/6J) retinal cross-section with PNA staining injected with control or 2E9 vg/eye RedO-Txnip, indicating RedO-Txnip is not toxic to the wildtype cones. 3E8 vg/eye RedO-H2BGFP was co-injected to track infection. (Medium gray: PNA. Light gray: H2BGFP. Gray: DAPI.)

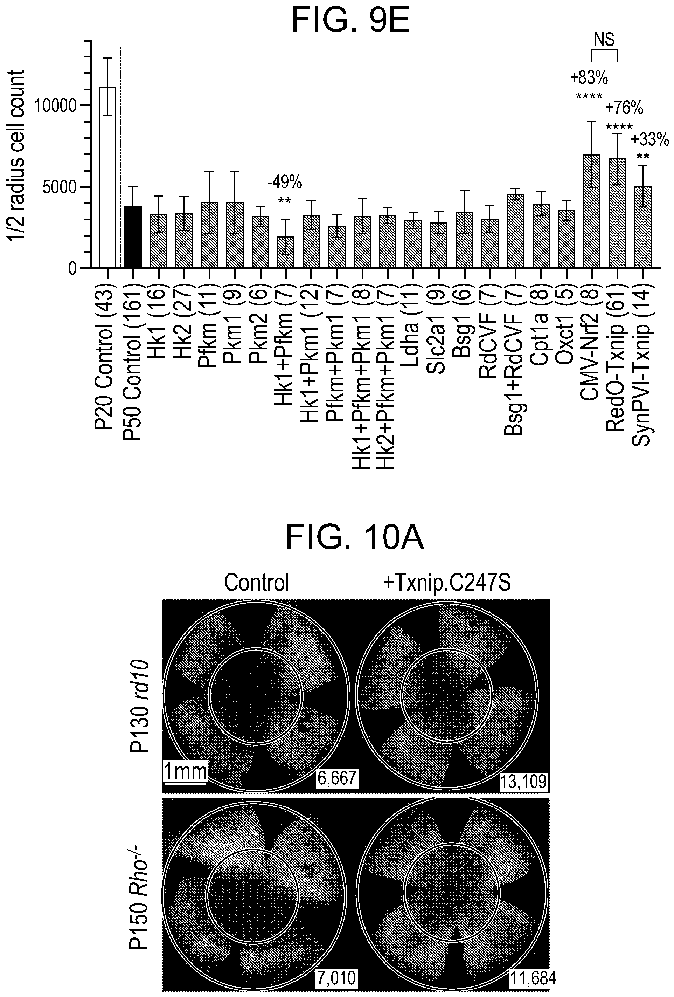

**FIG. 9E** is a graph depicting the quantification of H2BGFP-positive cones within the half radius of P20 rd1 control retinas, and P50 rd1 retinas treated with 20 different vectors and combinations or control. (Please note: dark-reared rd10 was not used for testing the RdCVF vector, and our AAV capsid and promoter were different from the original study (Byrne *et al.,* 2015). Error bar: standard deviation. NS: not significant, p > 0.05. * p < 0.05. ** p < 0.01. *** p < 0.001. **** p < or << 0.0001. (Same applies to the rest of the figures below.)
**FIGS. 10A-10D** are related to **FIGS. 2A-2B****.**
**FIG. 10A** are images of representative P130 rd10 and P150 rho-/- flat-mounted retinas with H2BGFP (gray) labeled cones treated with Txnip.C247S or control.

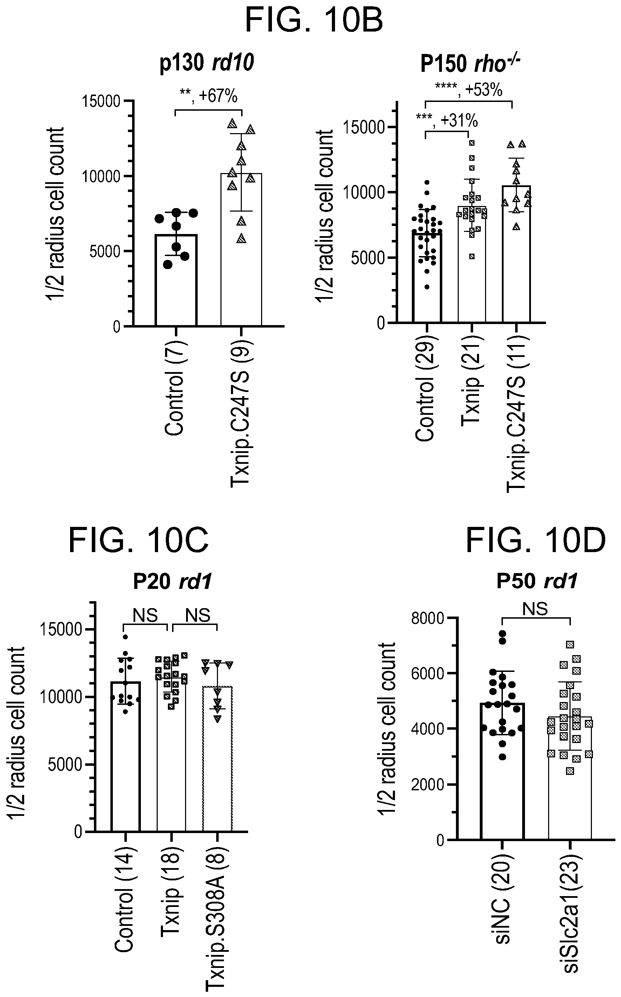

**FIG. 10B** are graphs depicting the quantification of H2BGFP-positive cones within the half radius of P130 rd10 and P150 rho-/- retinas treated with Txnip.C247S or control.
**FIG. 10C** is a graph depicting the quantification of H2BGFP-positive cones within the half radius of P20 rd1 retinas treated with Txnip, Txnip.S308A or control.
**FIG. 10D** is a graph depicting the quantification of H2BGFP-positive cones within the half radius of P50 rd1 retinas treated with siNC (non-targeting scrambled control shRNA) or S1c2a1/Glut1 shRNA.

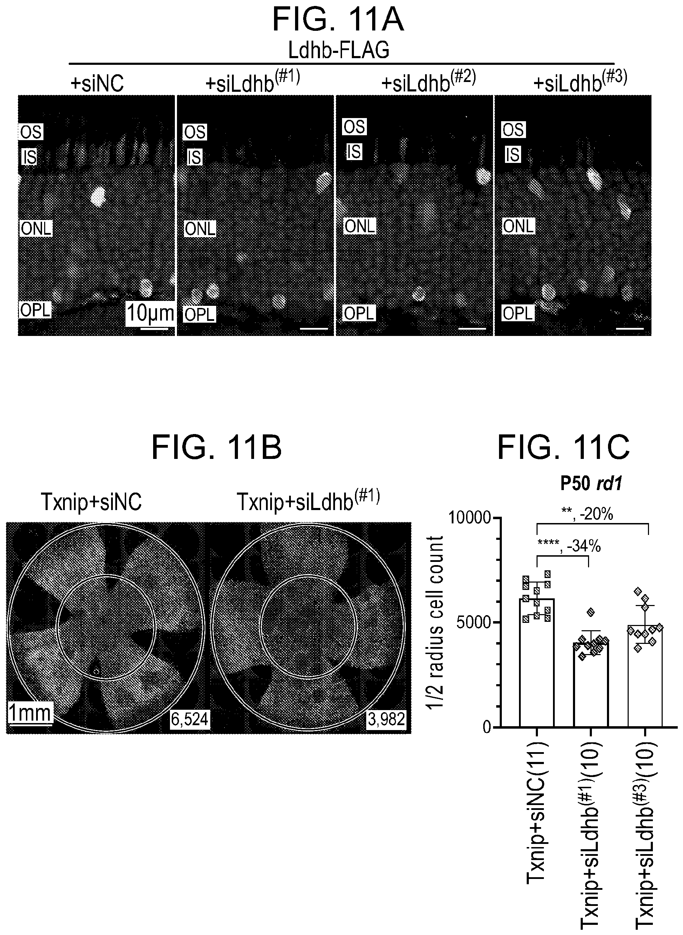

**FIGS. 11A-11E** are related to **FIGS. 3A-3C****.**
**FIG. 11A** are images of AAV8-RO1.7-Ldhb-FLAG with siNC control or *Ldhb* shRNAs in P21 wildtype (CD1) retina plus RedO-H2BGFP to track the infection. (Magenta: anti-FLAG. Green: anti-GFP. Gray: DAPI.)
**FIG. 11B** are images of representative P50 *rd1* flat-mounted retinas with H2BGFP (gray) labeled cones treated with Txnip + siNC, Txnip + siLdhb^{(#1)}, or Txnip + siLdhb^{(#3)}.
**FIG. 11C** is a graph depicting the quantification of H2BGFP-positive cones within the half radius of P50 *rd1* retinas treated with Txnip + siNC, Txnip + siLdhb^{(#1)} or Txnip + siLdhb^{(#3)}.

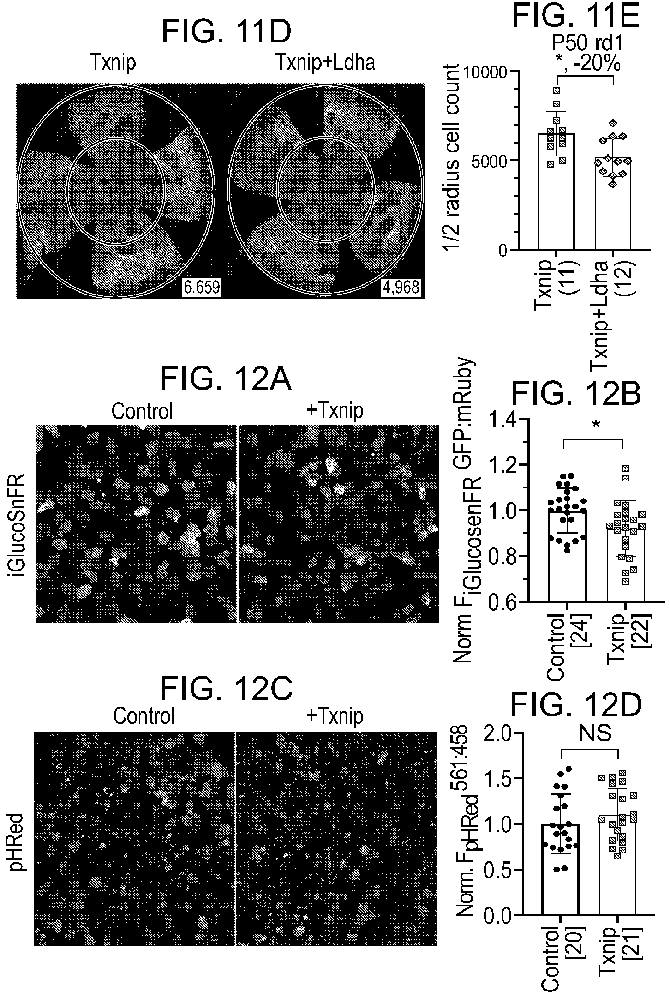

**FIG. 11D** are images of representative P50 *rd1* flat-mounted retinas with H2BGFP (gray) labeled cones treated with Txnip or Txnip + Ldha.
**FIG. 11E** is a graph depicting the quantification of H2BGFP-positive cones within the half radius of P50 *rd1* retinas treated with Txnip or Txnip + Ldha.
**FIGS. 12A-12D** are related to **FIGS. 4A-4E****.**
**FIG. 12A** are representative *ex vivo* live images of iGlucoSnFR labeled cones in P20 rd1 retinas cultured with high-glucose medium treated Txnip or control. (Green: glucose sensing GFP. Magenta: mRuby for self-normalization.)
**FIG. 12B** is a graph depicting the quantification of normalized iGlucoSnFR fluorescence intensity (FiGlucoSnFRGFP : mRuby, proportional to glucose level) in cones from P20 rd1 retinas treated with Txnip or control (≈3 images per retina).
**FIG. 12C** are *ex vivo* live images of pHRed labeled cones in P20 rd1 retinas cultured with high-glucose medium treated Txnip or control. (Magenta: fluorescence by 561 nm excitation, indicating a lower pH. Green: fluorescence by 458 nm excitation, indicating a higher pH.)
**FIG. 12D** is a graph depicting the quantification of normalized pHRed fluorescence intensity (FpHRedx561nm : 458nm, inversely proportional to pH value) in cones from P20 rd1 retinas treated with Txnip or control (≈3 images per retina).

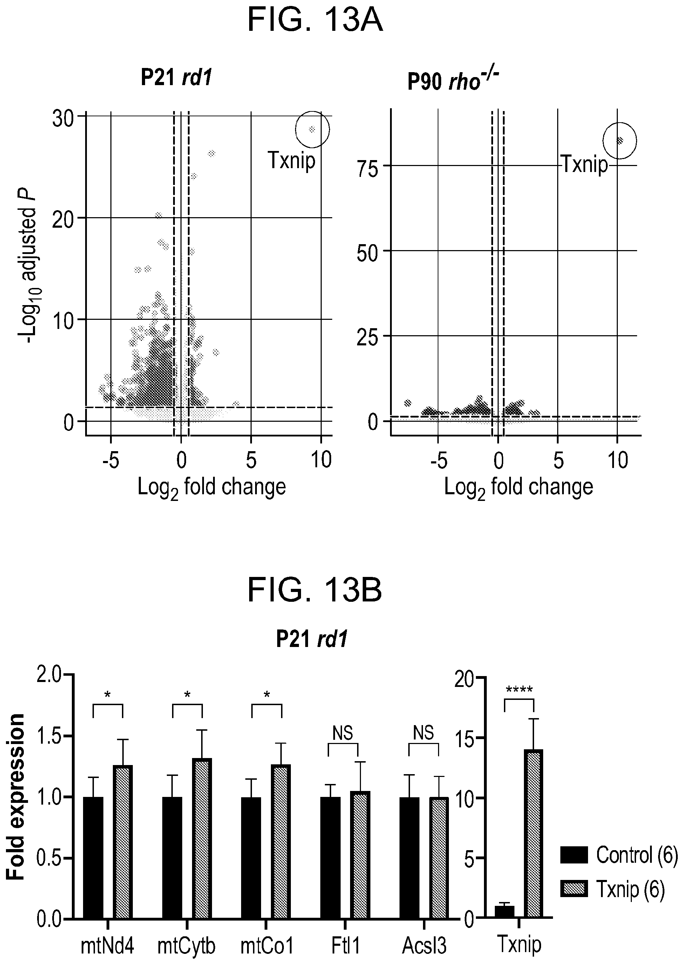

**FIGS. 13A-13I** are related to **FIGS. 5A-5J****.**
**FIG. 13A** are volcano plots of differentially expressed genes in RP cones FACS sorted from P21 rd1 retinas (left panel; +Txnip, n = 3, relative to control, n = 6) and P90 rho-/- retinas (right panel; +Txnip, n = 4, relative to control, n = 4). Dotted lines indicate adjusted p < 0.05 and log2 fold change > 0.5.
**FIG. 13B** is a graph depicting the ddPCR fold-changes of commonly upregulated mitochondrial ETC genes and genes not confirmed *(i.e.* Acsl3 and Ftl1) by Txnip overexpression in FACS sorted P21 rd1 cones.

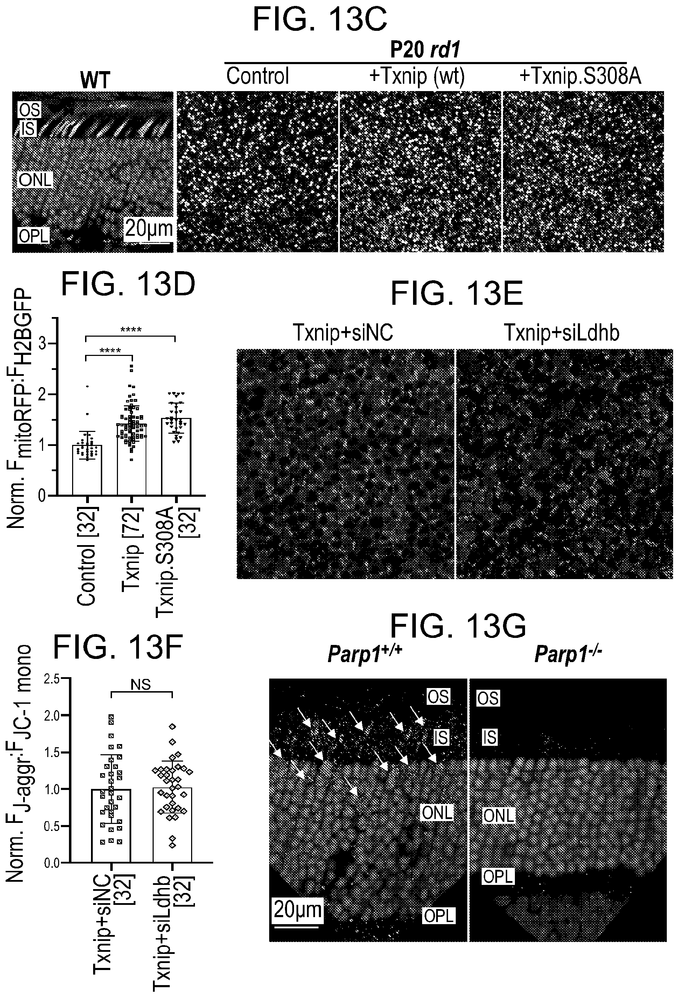

**FIG. 13C** are images of AAV8-SynP136-mitoRFP expression in P26 wildtype (BALB/c) retina cross-section in the most left panel (Medium gray: mitoRFP. Light gray: PNA. Gray: DAPI.) Other three panels show representative mitoRFP images from the control, Txnip and Txnip.S308A of fixed P20 rd1 retina flat-mounts near optic nerve head, reflecting the mitochondrial function. (Medium gray: mitoRFP. Gray: H2BGFP, for infection normalization.)
**FIG. 13D** is a graph depicting the quantification of normalized mito-RFP:H2BGFP intensity of P20 *parp1* retinas treated with control, Txnip or Txnip.S308A (4 images per retina).
**FIG. 13E** are representative JC-1 dye staining images from live cones in P20 rd1 retina treated with Txnip + siNC or + siLdhb(#2). (Medium gray: J-aggregate, indicating high ETC function. Light gray: JC-1 monomer, for self-normalization. H2BGFP channel, the tracer of AAV infected area, is not shown.)
**FIG. 13F** is a graph depicting the quantification of normalized cone JC-1 dye staining (fluorescence intensity of J-aggregate:JC-1 monomer) from live cones in P20 rd1 retinas treated with Txnip + siNC or siLdhb(#2) (4 images per retina).
**FIG. 13G** are images if Parp1 antibody staining of parp1+/+ (C57BL/6J) or parp1-/- (on 129S background) retina. (Magenta: Parp1. Gray: DAPI. Arrow heads: Parp1 staining from inner segments and cone nuclei).

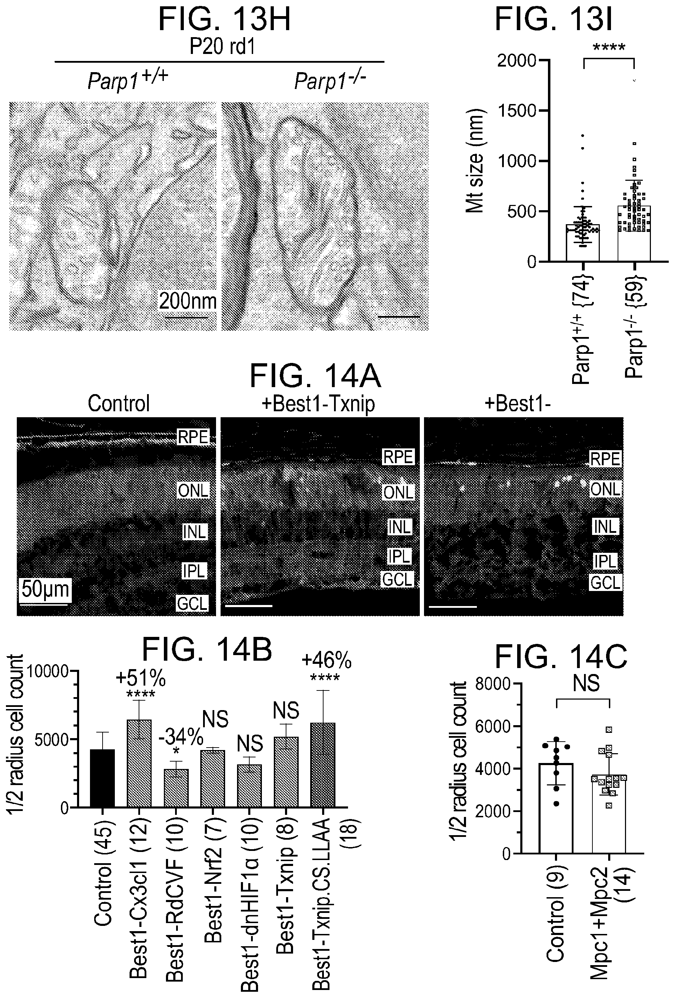

**FIG. 13H** are representative mitochondria EM images from P20 parp1+/+ or parp1-/- rd1 cones.
**FIG. 13I** is a graph depicting the quantification of mitochondrial diameters from P20 parp1+/+ or parp1-/- rd1 cones from one retina per condition.
**FIGS. 14A-14F** are related to **FIGS. 5A-5J****, 7A-7B** and **8A-8E.**
**FIG. 14A** are images of Glut1 expression in P37 wildtype (C57BL/6J) eyes treated with control, AAV8-Best1-Txnip or AAV8-Best1-Txnip.C247S.LL351&352AA (Medium gray: Glut1. Light gray: RedO-H2BGFP for infection tracing, leaky expression in RPE due to recombination with Best1-vector due to unclear mechanism. Gray: DAPI.)
**FIG. 14B** is a graph depicting the quantification of H2BGFP-positive cones within the half radius of P50 rd1 retinas treated with 6 different Best1-vectors or control)
**FIG. 14C** is a graph depicting the quantification of H2BGFP-positive cones within the half radius of P50 rd1 retinas treated with Mpc1 + Mpc2 or control.

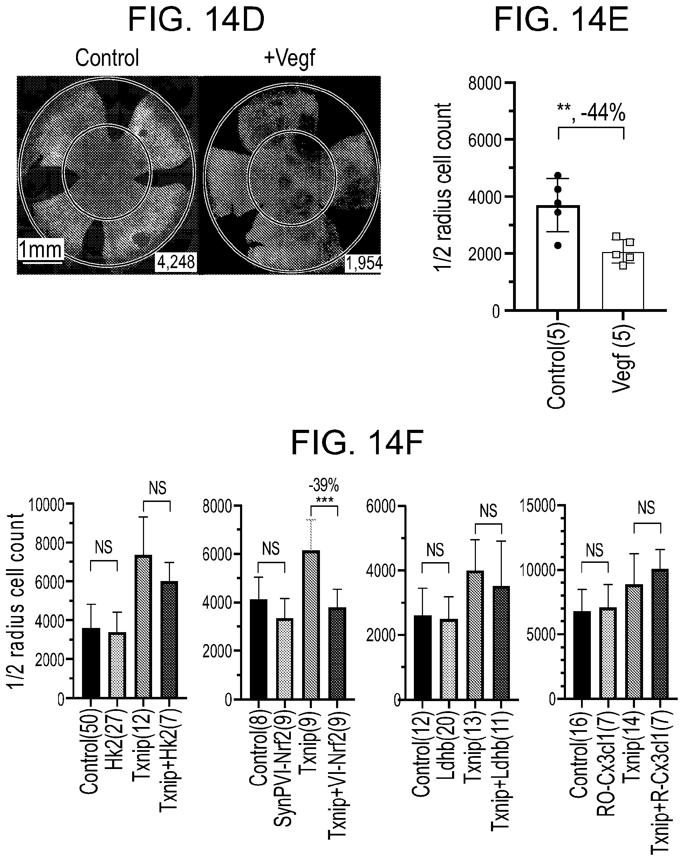

**FIG. 14D** are representative P50 rd1 flat-mounted retinas with H2BGFP (gray) labeled cones treated with Vegf164 and control. Abbreviation: Txnip.CS.LLAA = Txnip.C247S.LL351&352AA.
**FIG. 14E** is a graph depicting the quantification of H2BGFP-positive cones within the half radius of P50 rd1 retinas treated with Vegf164 or control.
**FIG. 14F** are graphs depicting the quantification of H2BGFP-positive cones within the half radius of P50 rd1 retinas treated with control, SynPVI-Hk2, SynPVI-Nrf2, RedO-Ldhb, RedO-Cx3cl1, RedO-Txnip and combinations with RedO-Txnip. Abbreviation: VI = SynPVI. RO- or R- = RedO-.

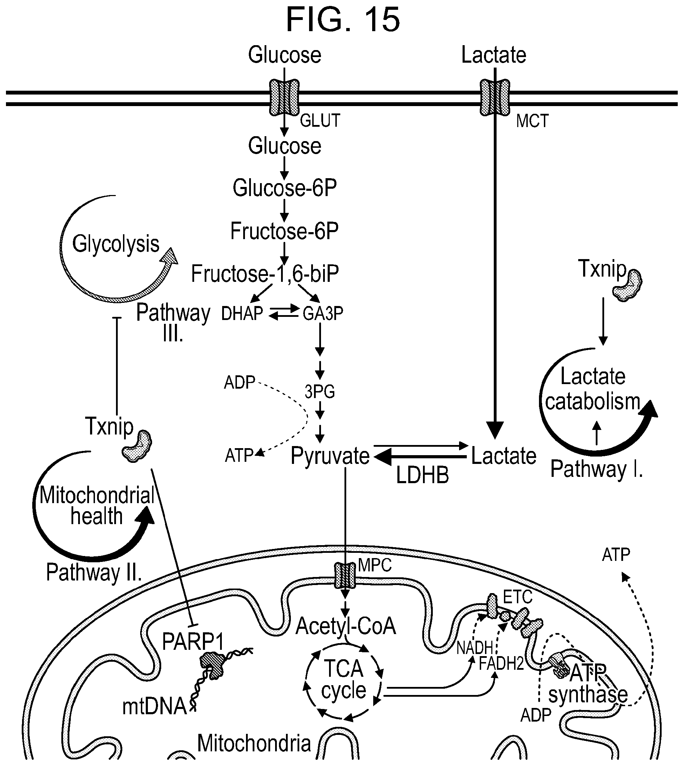

**FIG. 15** is a schematic of a proposed Txnip working mechanism.

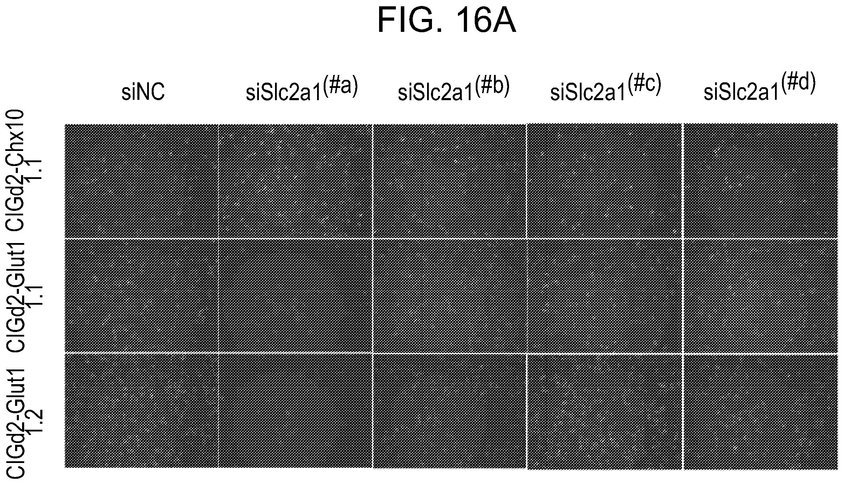

**FIGS. 16A-16B** show Slc2a1/Glut1 shRNA *in vitro* screening and screened out siSlc2a1^{(#a)} for *in vivo* experiments.
**FIG. 16A** are images of GFP signals from overnight transfected HEK293T cells labeled with CAG-Slc2a1-IRES-GFPd2 (CIGd2-Glut1) or CIGd2-Chx10 (negative control group) plus siSlc2a1(#a, b, c, d) or siNC at 1:1 or 1:2 ratios.

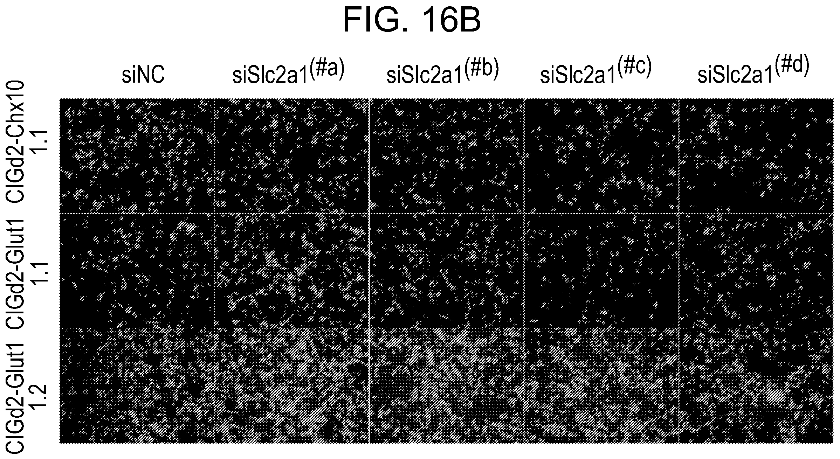

**FIG. 16B** are images of mCherry signals (positive-control for transfection) from the same imaging regions as in **FIG. 16A** above.

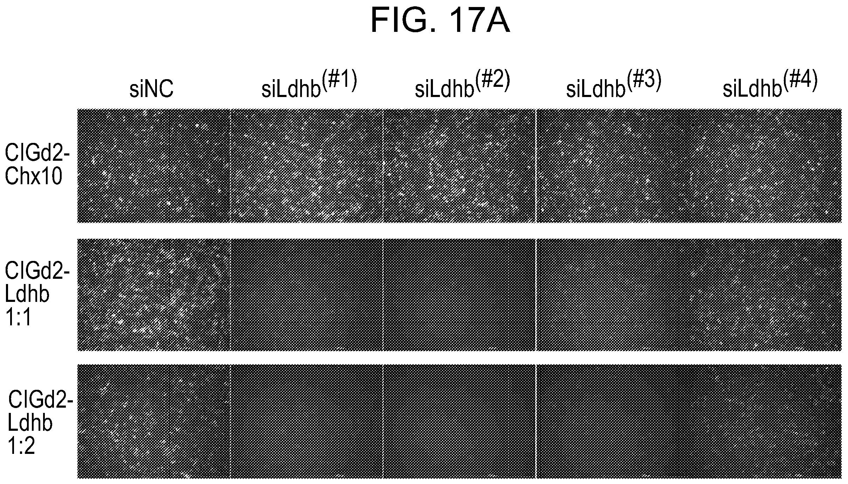

**FIGS. 17A-17B** show Ldhb shRNA *in vitro* screening and screened out siLdhb^{(#2), (#1)&(#3)} for *in vivo* experiments.
**FIG. 17A** are images of GFP signals from overnight transfected HEK293T cells labeled with CAG-Ldhb-IRES-GFPd2 (CIGd2-Ldhb) or CIGd2-Chx10 (negative control group) plus siLdhb(#1, 2, 3, 4) or siNC at 1:1 or 1:2 ratios.

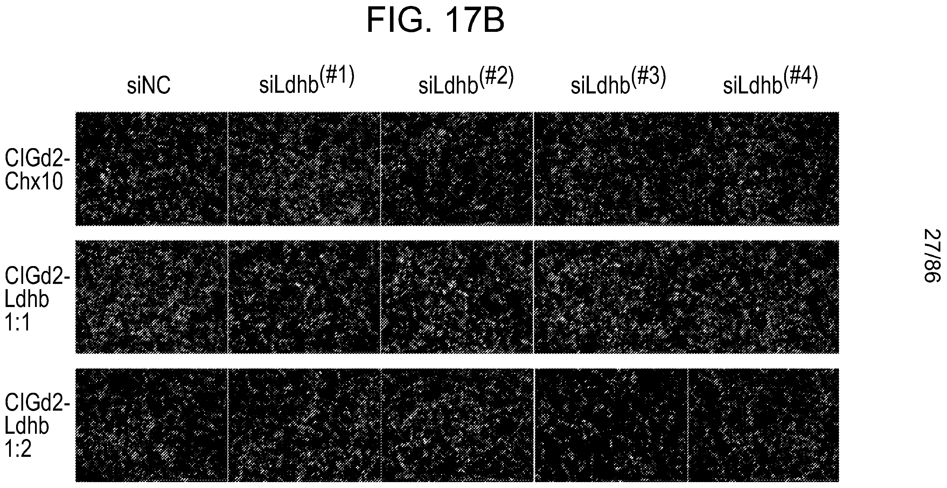

**FIG. 17B** are images of mCherry signals (positive-control for transfection) from the same imaging regions as in **FIG. 17A** above.

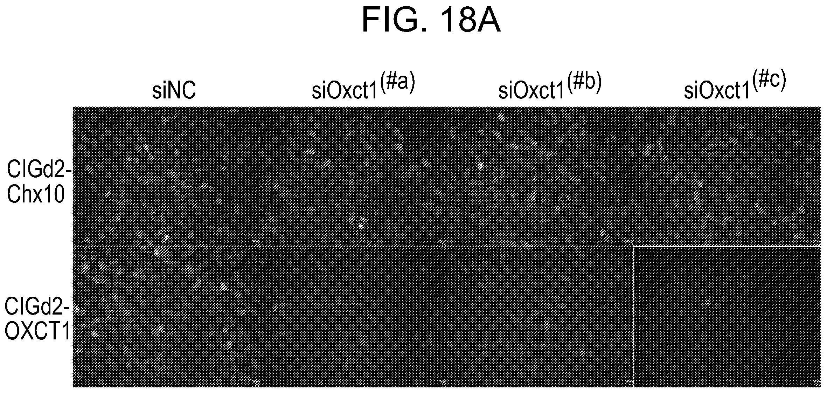

**FIGS. 18A-18B** show Oxct1 shRNA *in vitro* screening and screened out siOxct1^{(#c)} for *in vivo* experiments.
**FIG. 18A** are images of GFP signals from overnight transfected HEK293T cells labeled with CAG-Oxct1-IRES-GFPd2 (CIGd2-OXCT1) or CIGd2-Chx10 (negative control group) plus siOxct1(#a, b, c) or siNC at 1:2 ratios.

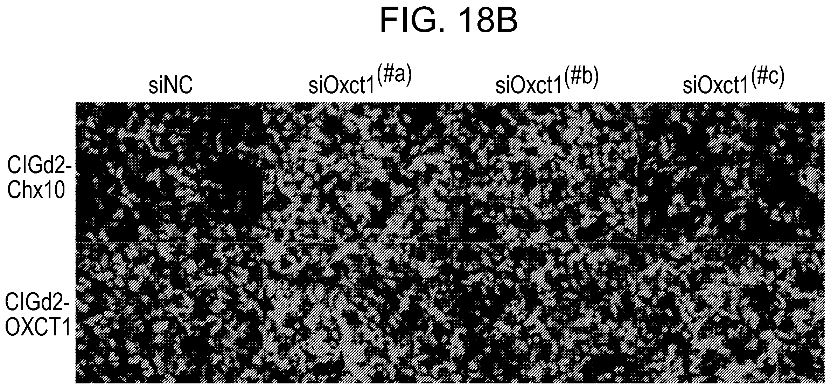

**FIG. 18B** are images of mCherry signals (positive-control for transfection) from the same imaging regions as in **FIG. 18A** above.

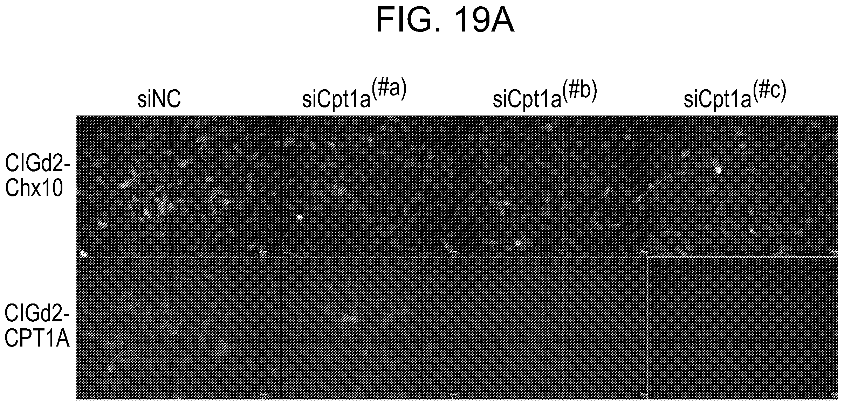

**FIGS. 19A-19B** show Cpt1a shRNA *in vitro* screening and screened out siCpt1a^{(#c)} for *in vivo* experiments.
**FIG. 19A** are images of GFP signals from overnight transfected HEK293T cells labeled with CAG-Cptla-IRES-GFPd2 (CIGd2-CPT1A) or CIGd2-Chx10 (negative control group) plus siCpt1a(#a, b, c) or siNC at 1:2 ratios.

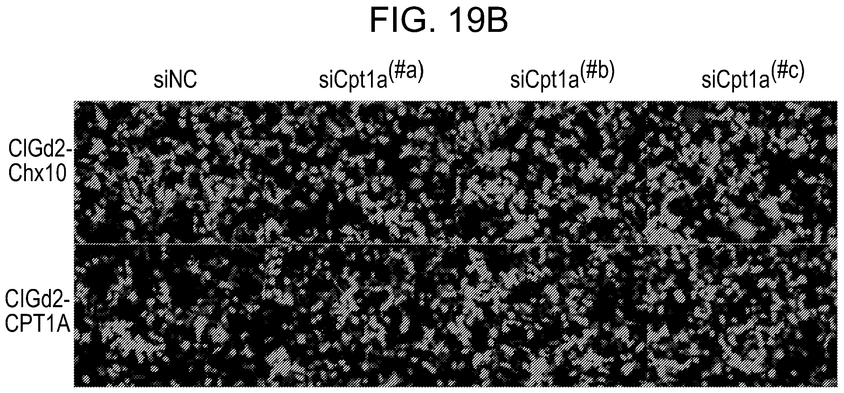

**FIG. 19B** are images of mCherry signals (positive-control for transfection) from the same imaging regions as in **FIG. 19A** above.

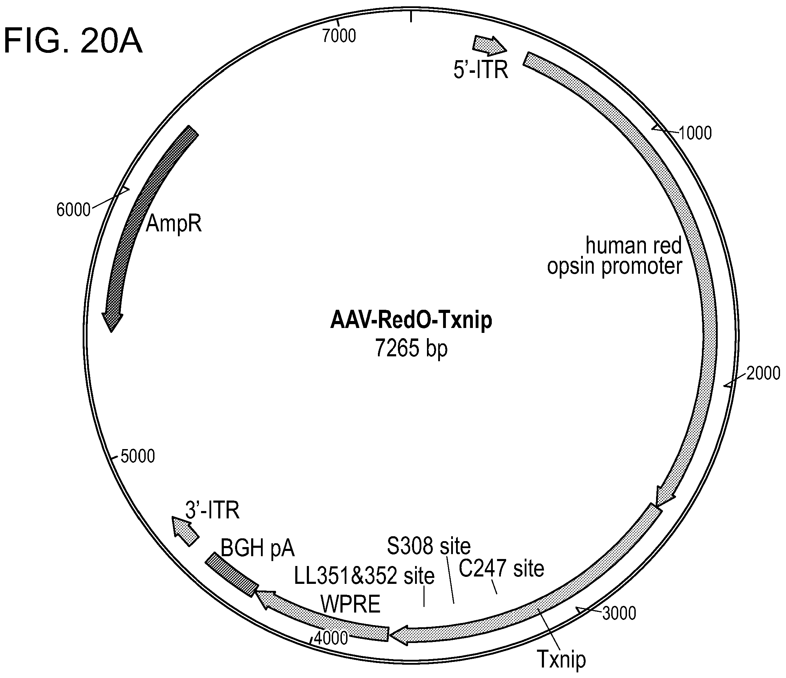

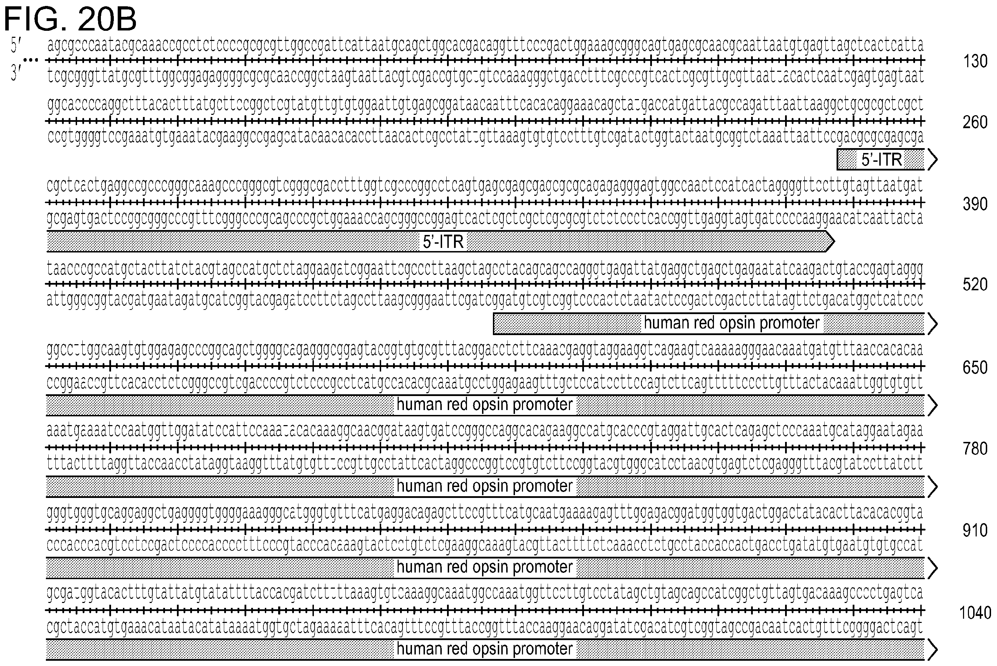

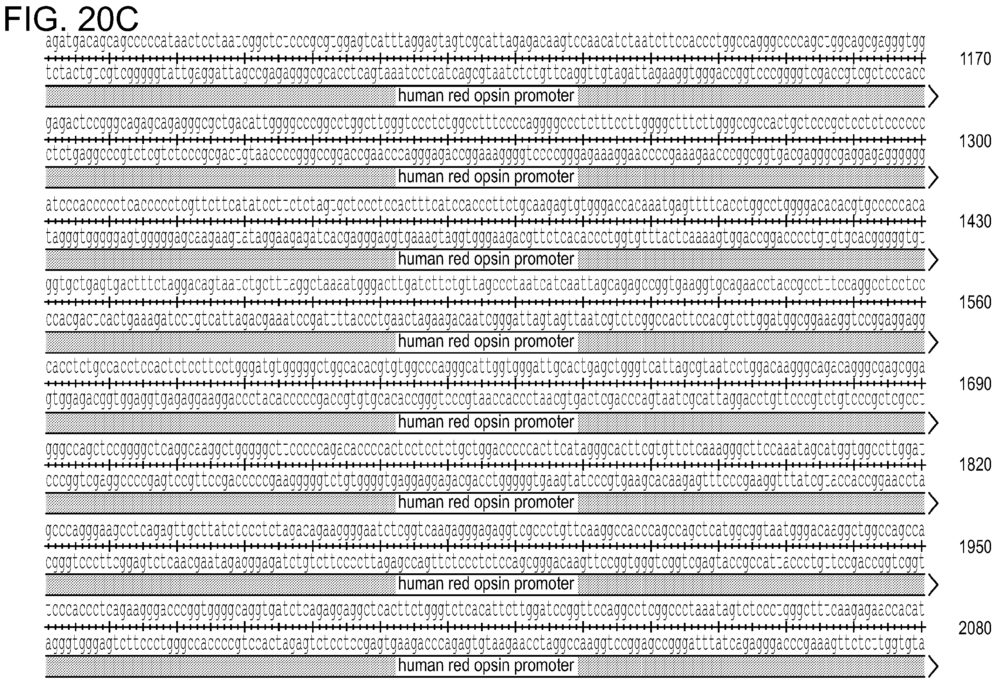

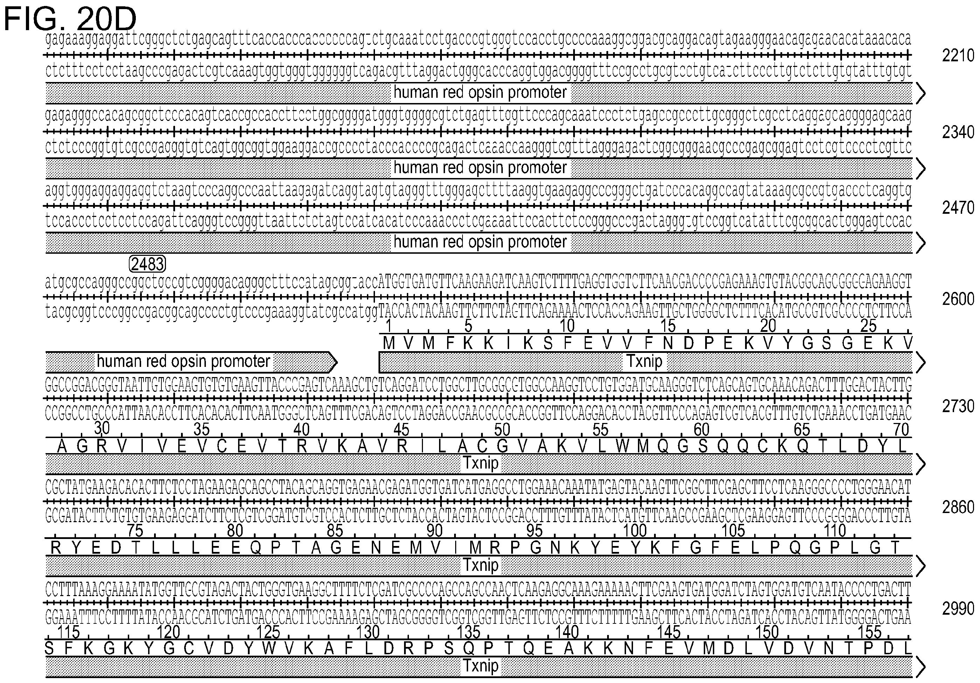

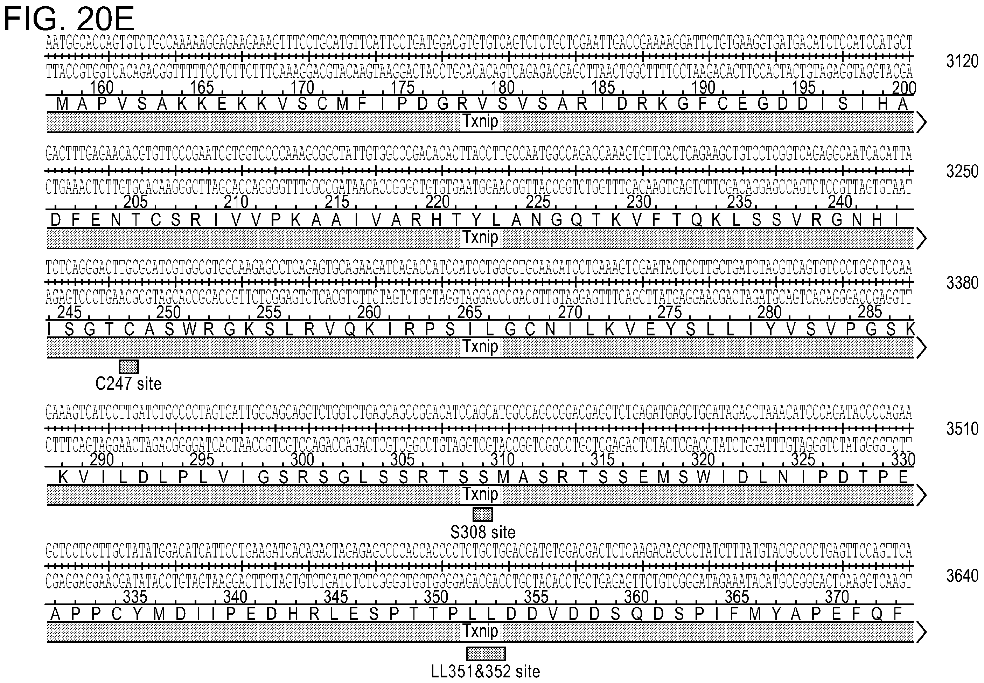

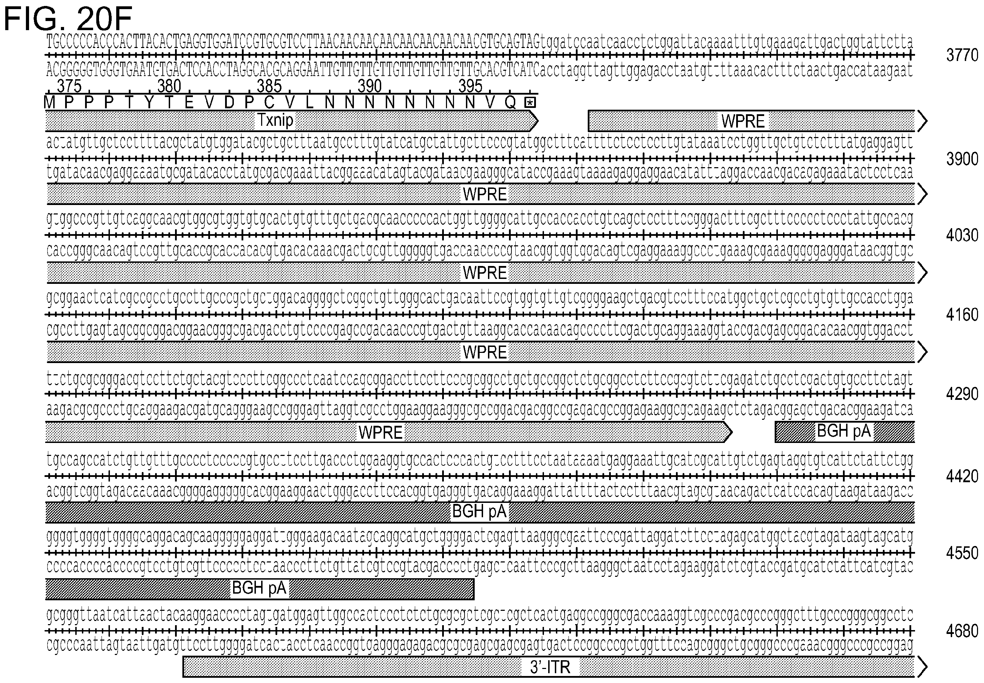

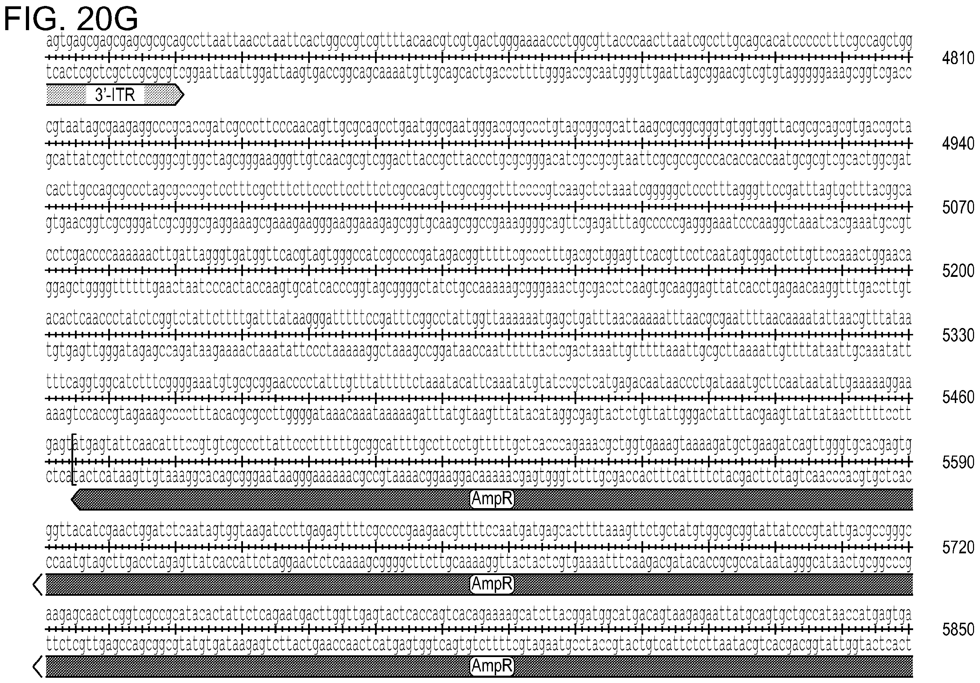

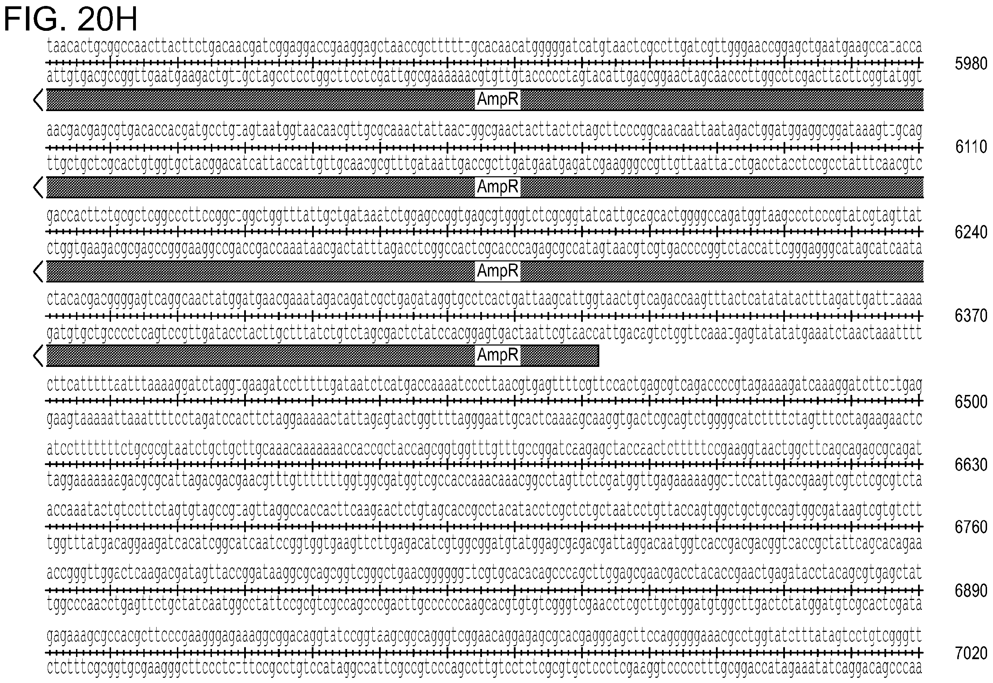

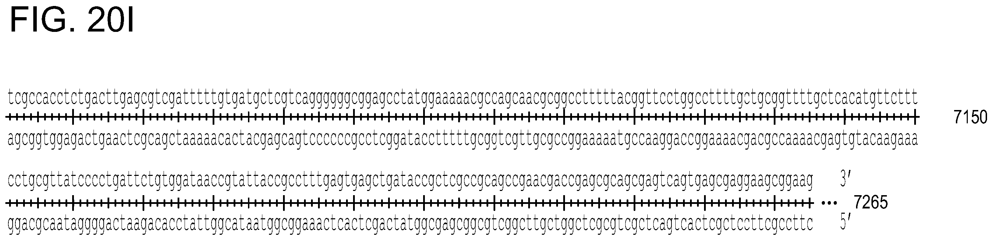

**FIGS. 20A-20I** depict an exemplary vector map of an exemplary AAV vector of the invention comprising a RedO promoter and a nucleic acid molecule encoding wild-type thioredoxin-interacting protein (TXNIP). **FIG. 20E** depicts the position of the TXNIP C247 variant, the TXNIP S308A variant, and the TXNIP LL351-352AA variant. FIGS. 20B-I disclose SEQ ID NOS 146 and 168, respectively in order of appearance.

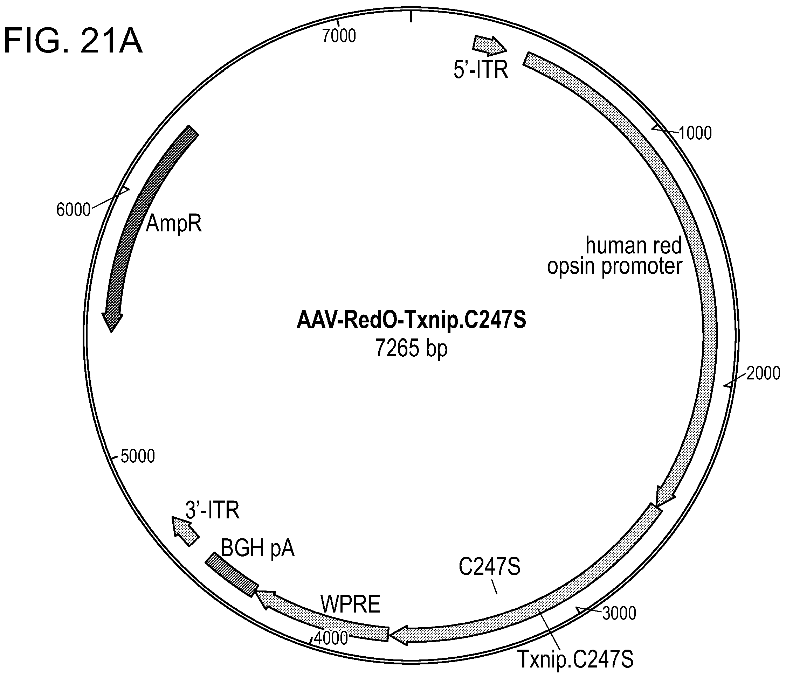

**FIGS. 21A-21B****, 22A-22B, 23A-23B, and 24A-24B** are the portion of the vector map in FIG. 20E depicting the TXNIP variants described herein. For use in the working examples of the application, the AAV vectors comprising the TXNIP variants described herein were as depicted in FIGS. 20A-20I with the exception of variant TXNIP.

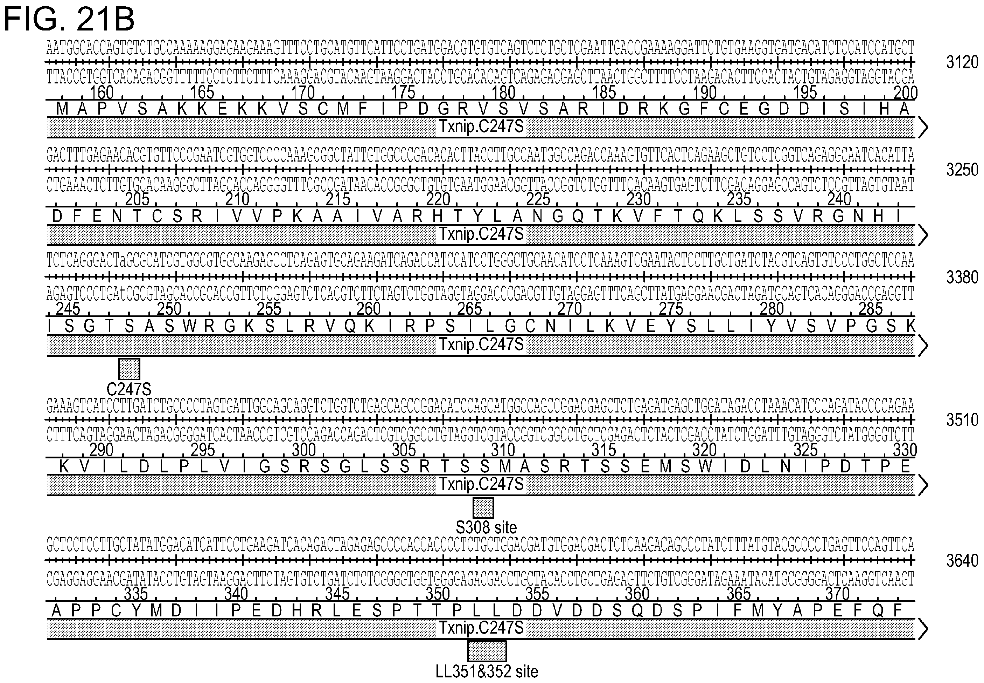

**FIGS. 21A-21B** depict an exemplary vector map of the portion of the exemplary AAV vector of the invention in FIGs. 20A-20I comprising a RedO promoter and a nucleic acid molecule encoding C247S variant thioredoxin-interacting protein (TXNIP). FIG. 21B discloses SEQ ID NOS 147-148, respectively, in order of appearance.

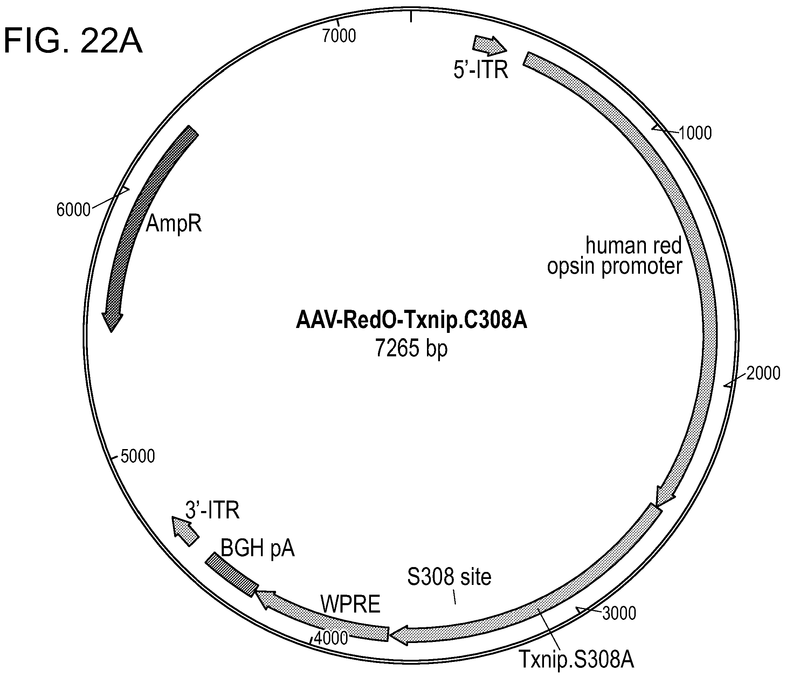

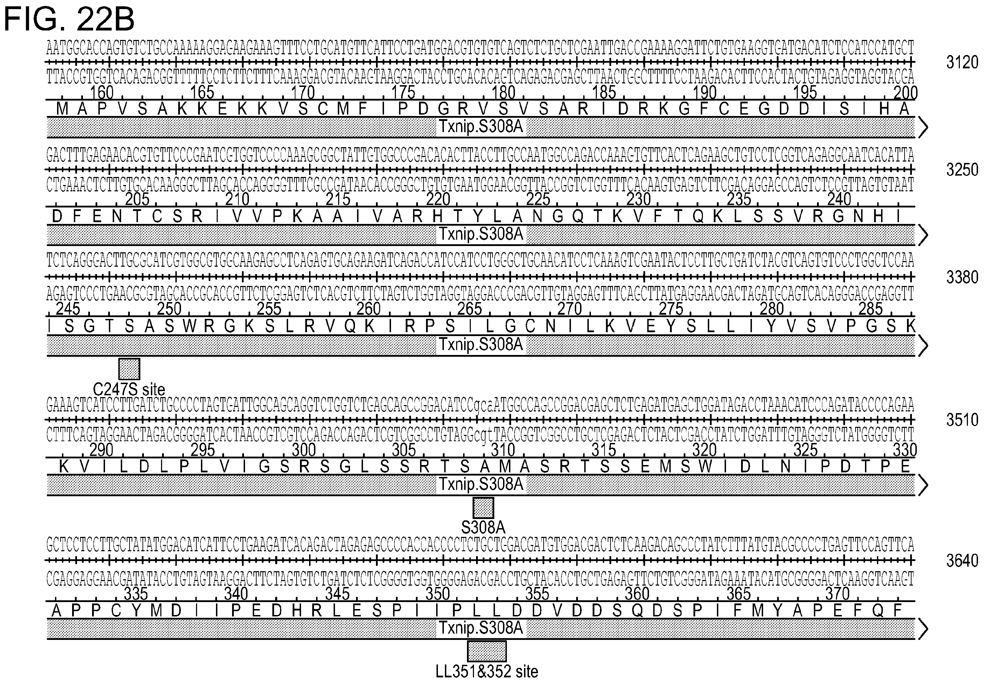

**FIGS. 22A-22B** depict an exemplary vector map of the portion of the exemplary AAV vector of the invention in FIGS. 20A-20I comprising a RedO promoter and a nucleic acid molecule encoding S308A variant thioredoxin-interacting protein (TXNIP). FIG. 22B discloses SEQ ID NOS 149-150, respectively, in order of appearance.

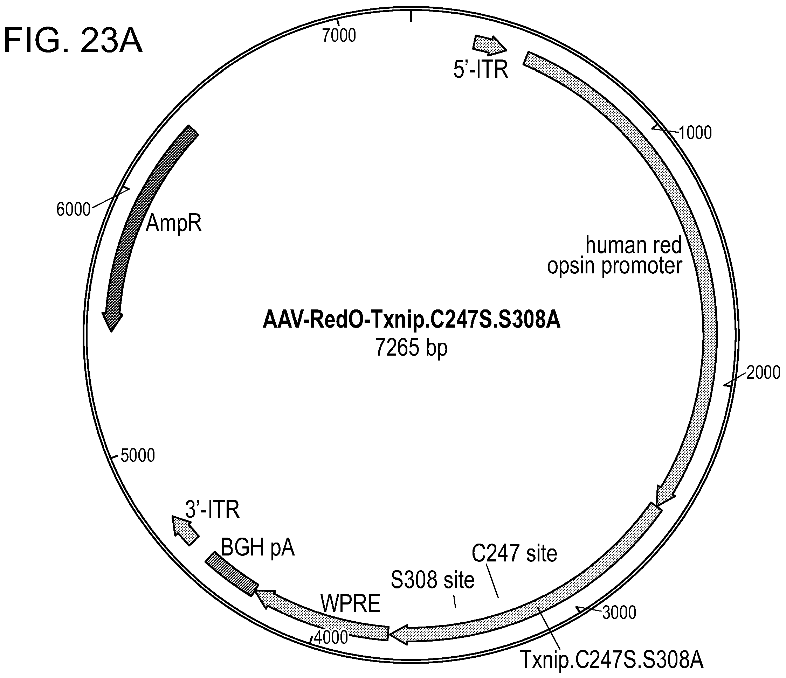

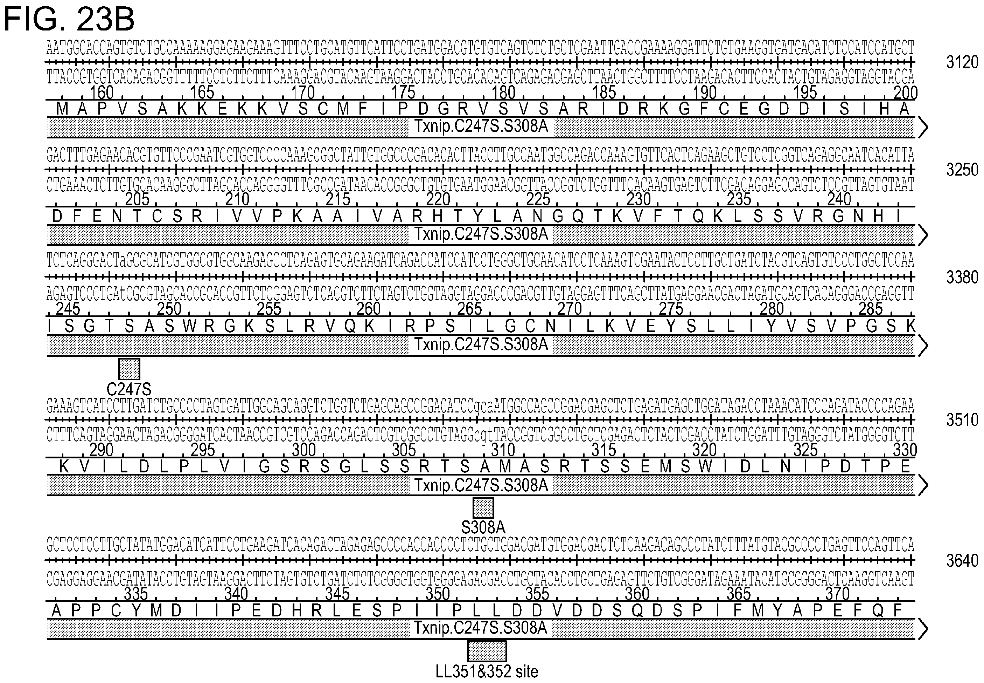

**FIGS. 23A-23B** depict an exemplary vector map of the portion of the exemplary AAV vector of the invention in FIGS. 20A-20I comprising a RedO promoter and a nucleic acid molecule encoding C247S and S308A variant thioredoxin-interacting protein (TXNIP). FIG. 23B discloses SEQ ID NOS 151-152, respectively, in order of appearance.

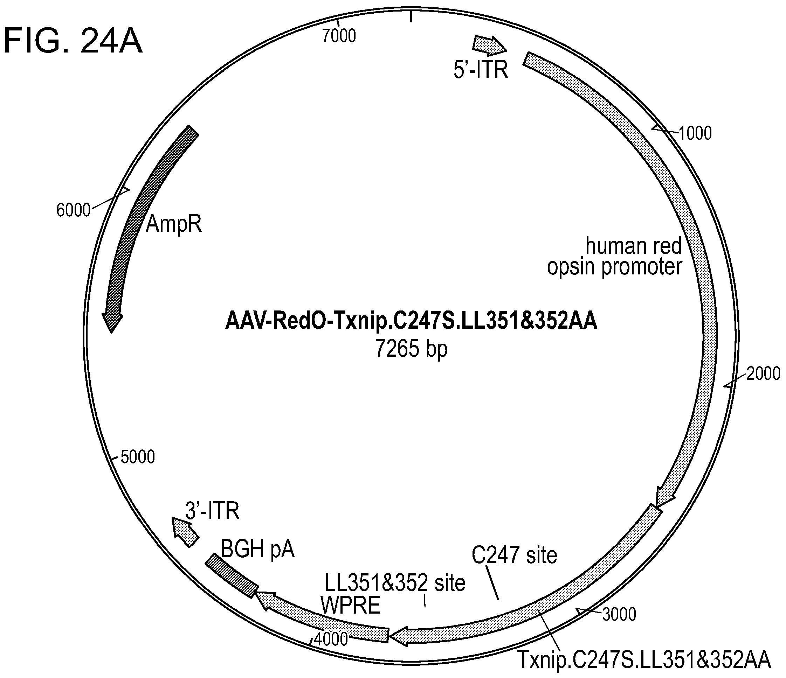

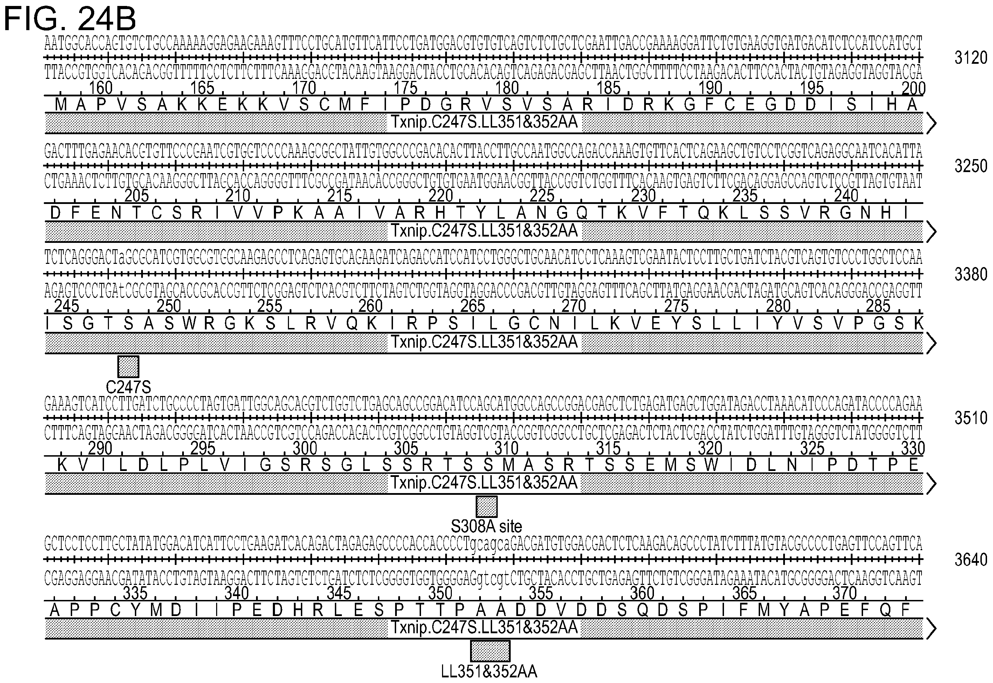

**FIGS. 24A-24B** depict an exemplary vector map of the portion of the exemplary AAV vector of the invention in FIGS. 20A-20I comprising a RedO promoter and a nucleic acid molecule encoding LL351-352AA variant thioredoxin-interacting protein (TXNIP). FIG. 24B discloses SEQ ID NOS 153-154, respectively, in order of appearance.

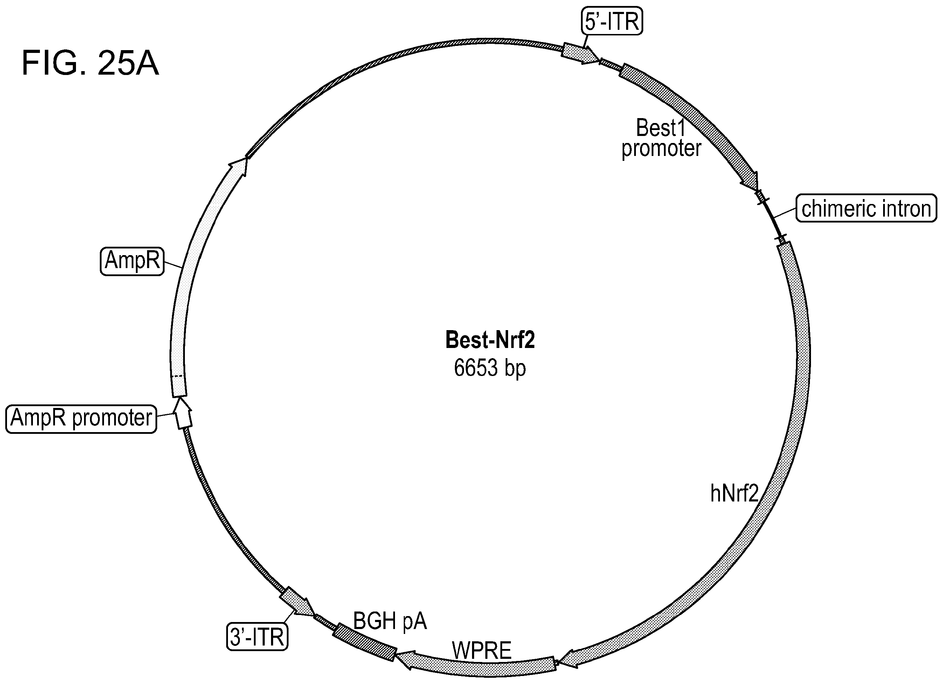

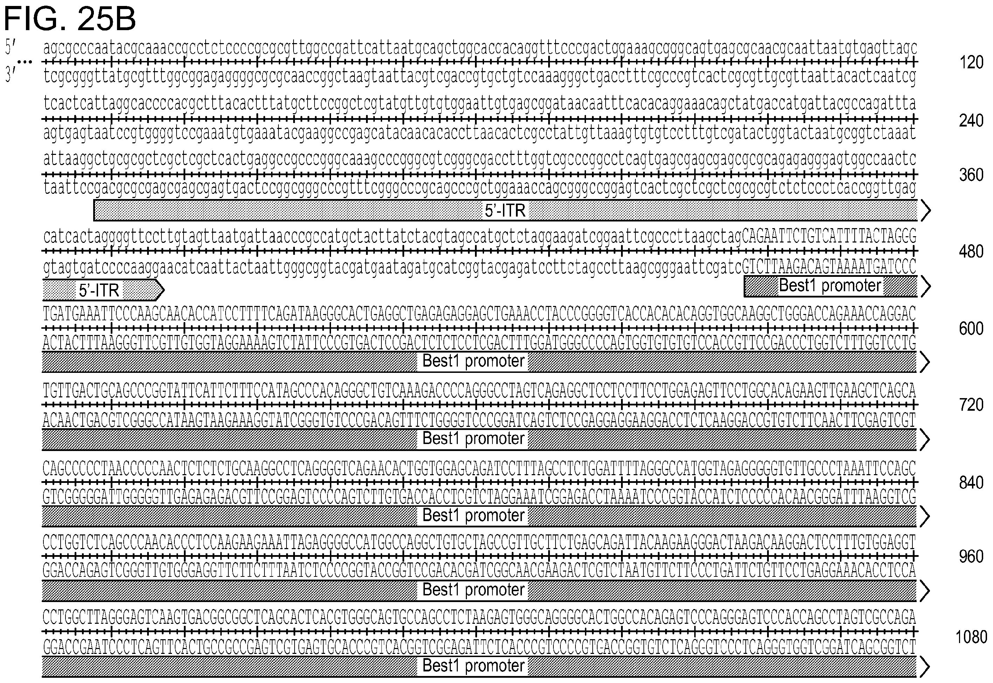

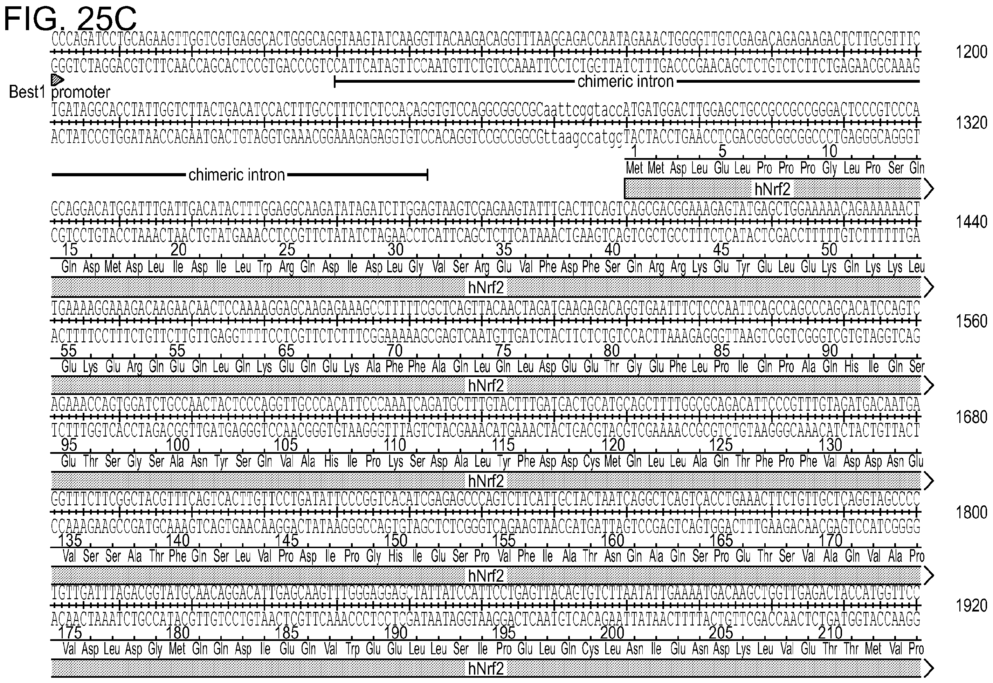

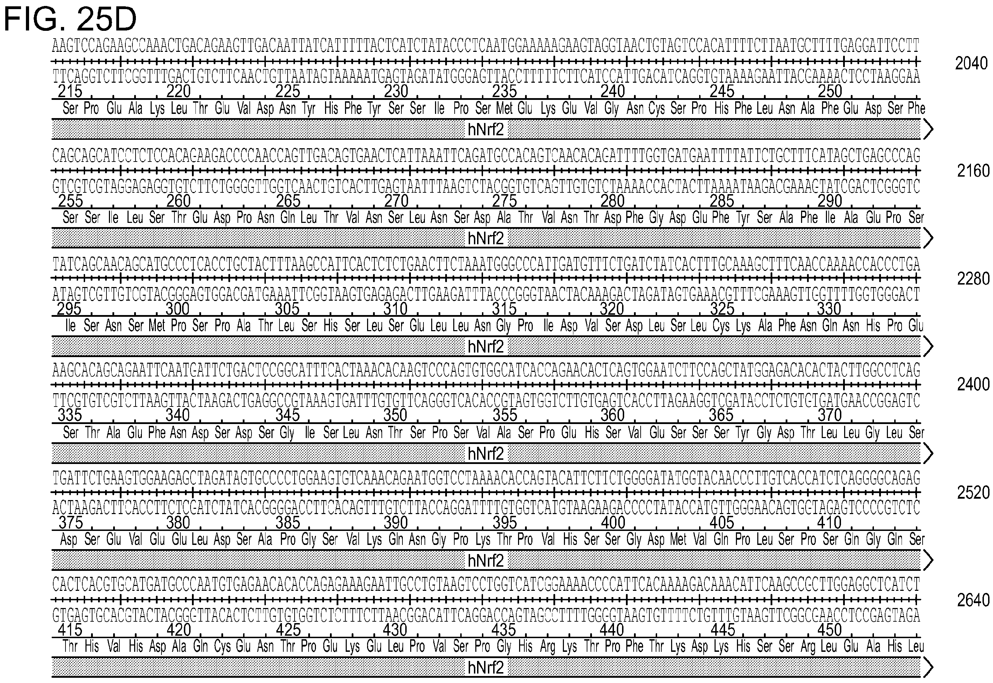

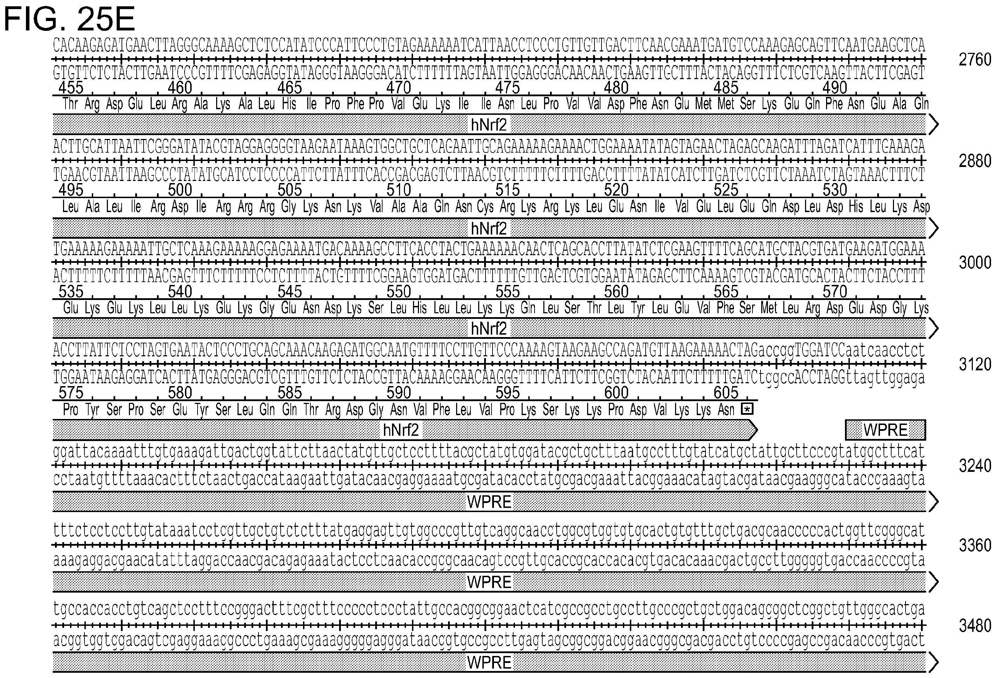

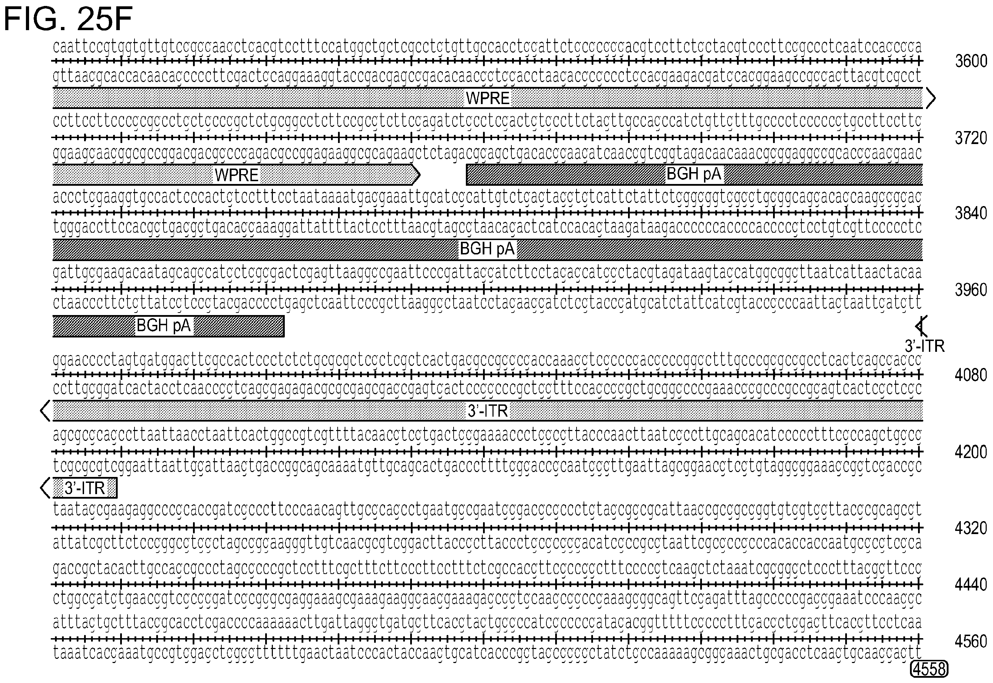

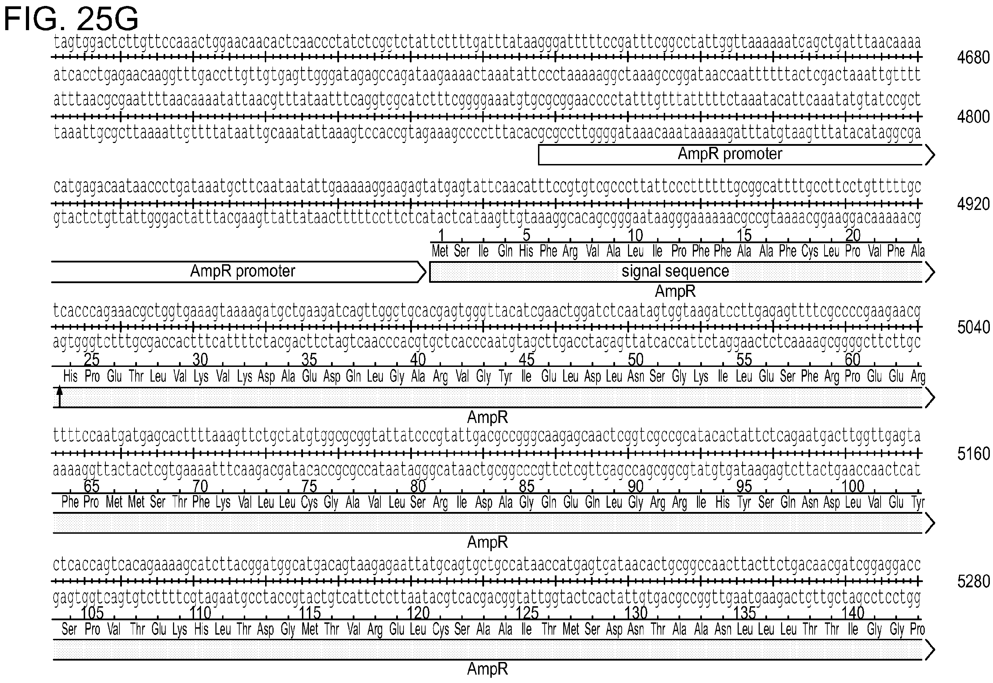

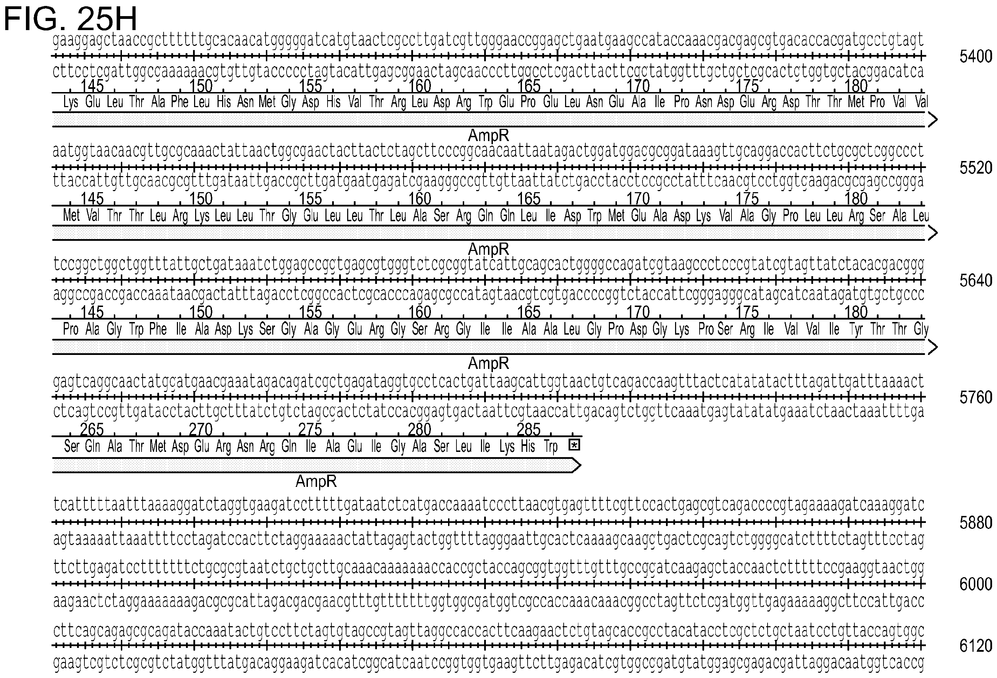

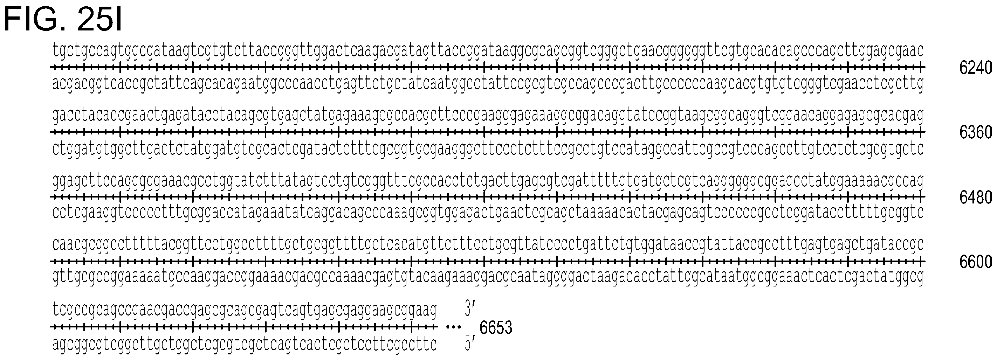

**FIGS. 25A-25I** depict an exemplary vector map of an exemplary AAV vector of the invention comprising a human bestrophin 1 (hBest1) promoter and a nucleic acid molecule encoding nuclear factor erythroid 2-like 2 (Nrf2). FIGS. 25B-I disclose SEQ ID NOS 155-156 and 35, respectively, in order of appearance.

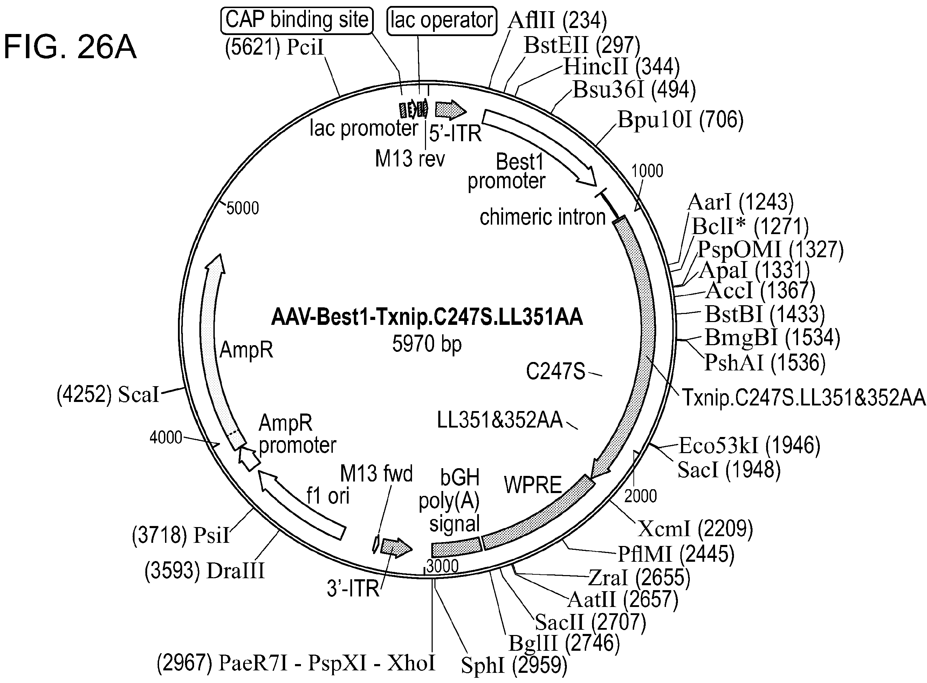

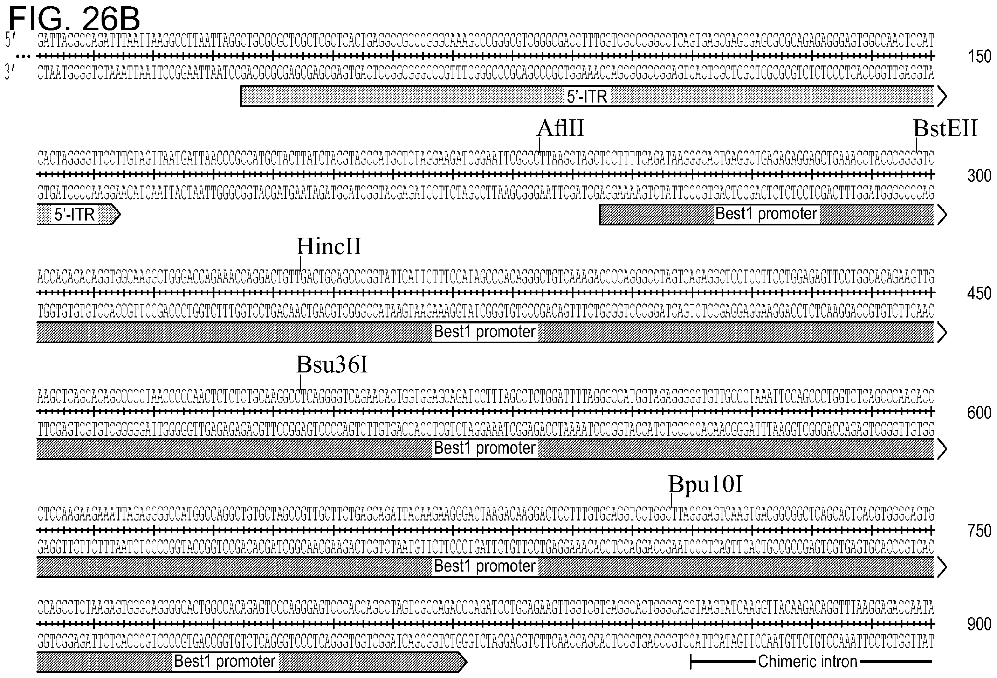

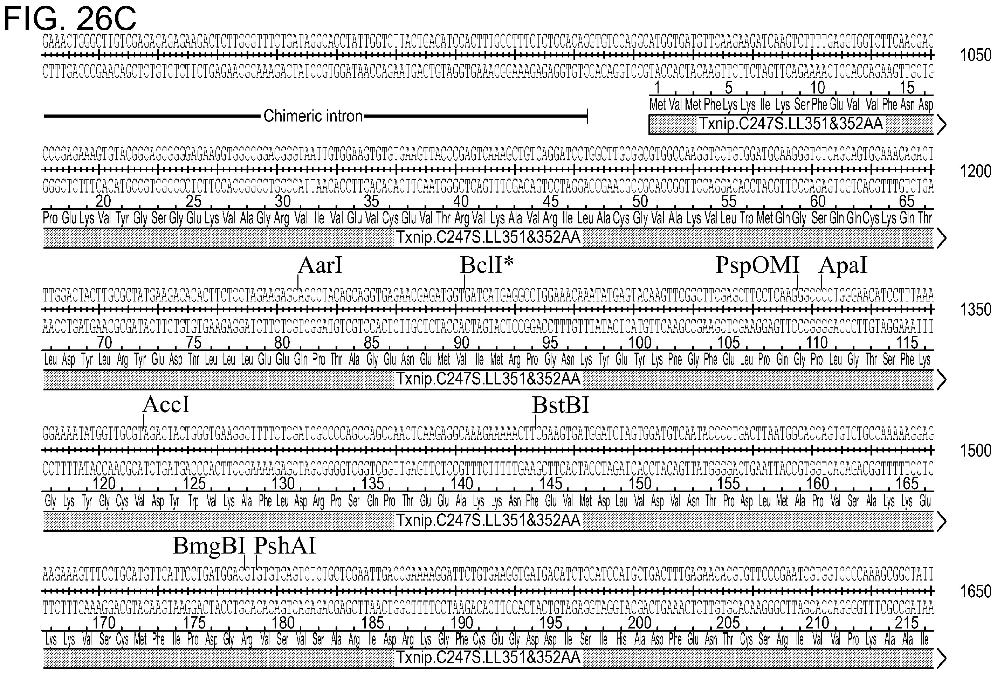

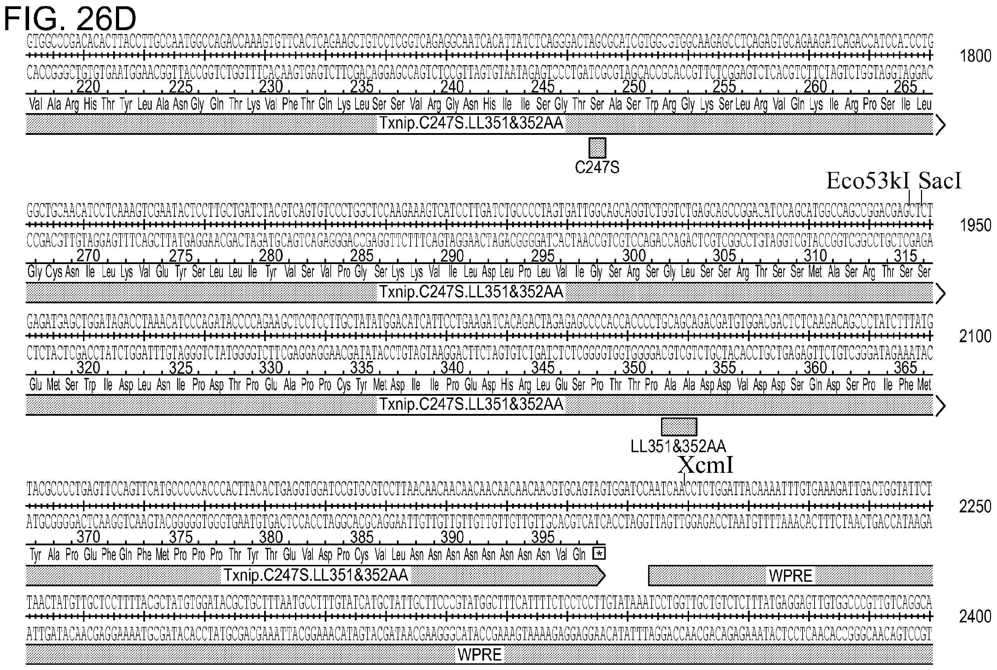

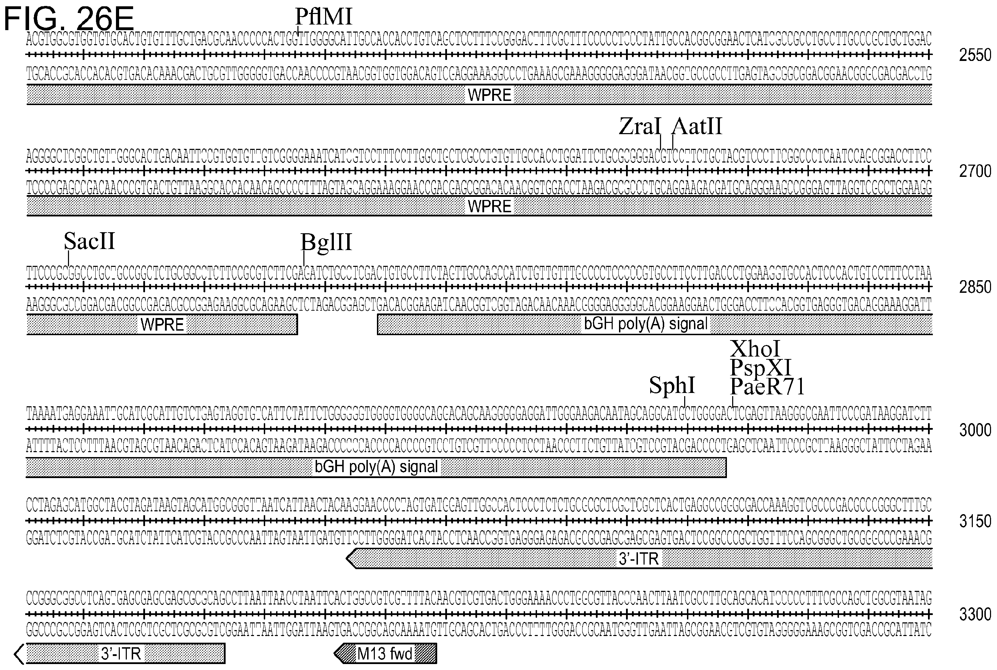

**FIGS. 26A-26E** depict an exemplary vector map of an exemplary AAV vector of the invention comprising a human bestrophin 1 (hBest1) promoter and a nucleic acid molecule encoding C247S.LL351-352AA variant thioredoxin-interacting protein (TXNIP). FIGS. 26B-E disclose SEQ ID NOS 158-159, respectively, in order of appearance.

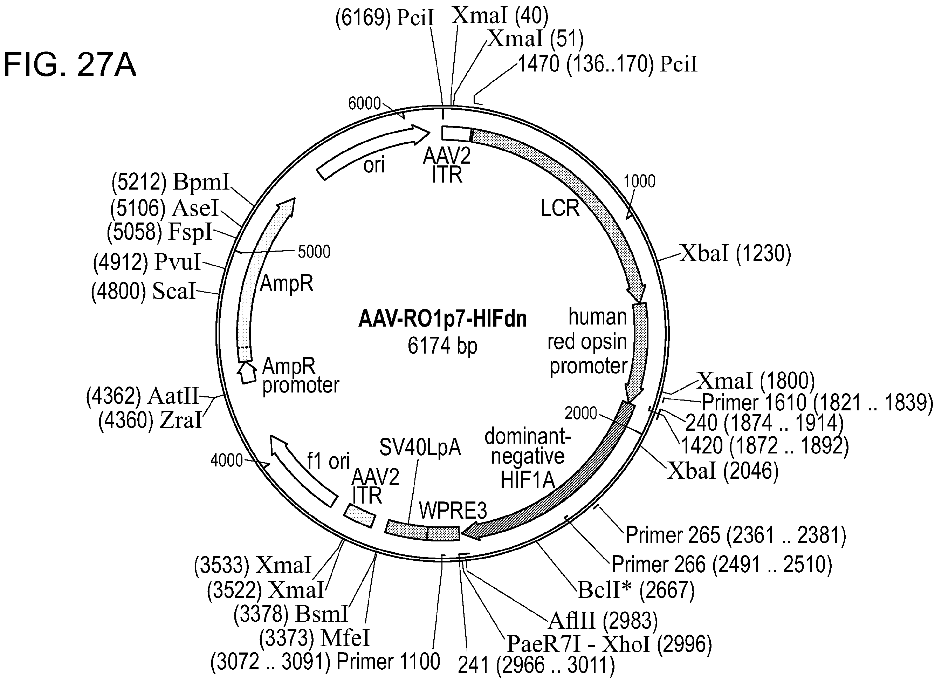

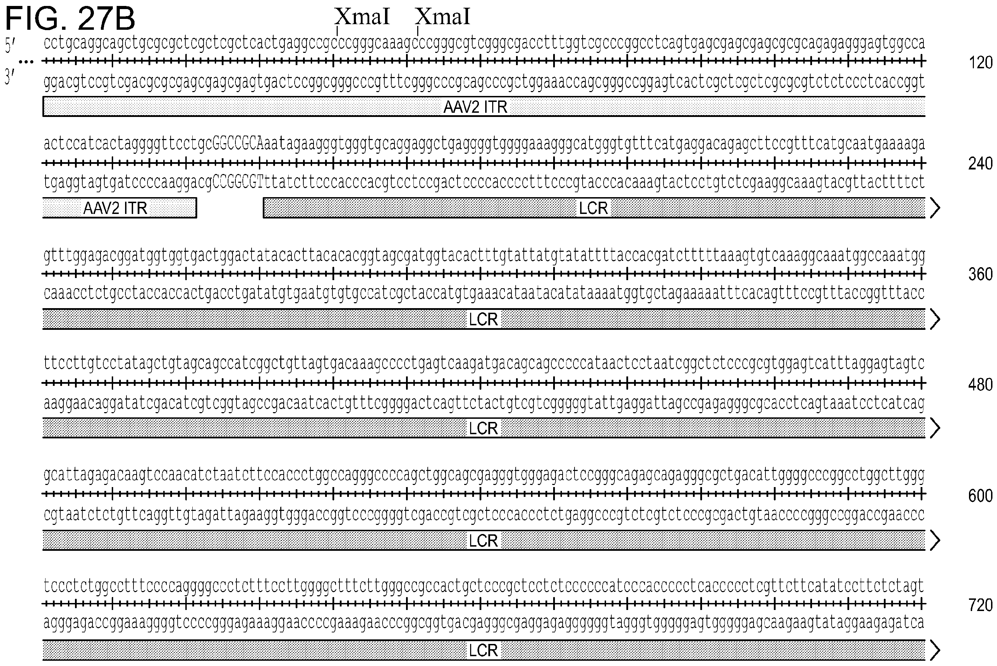

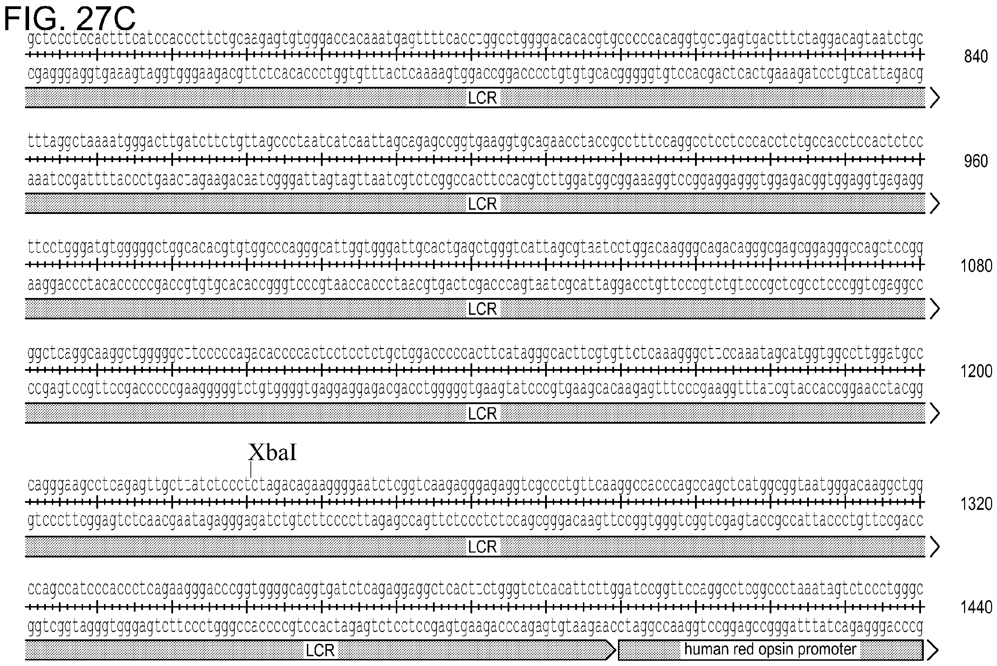

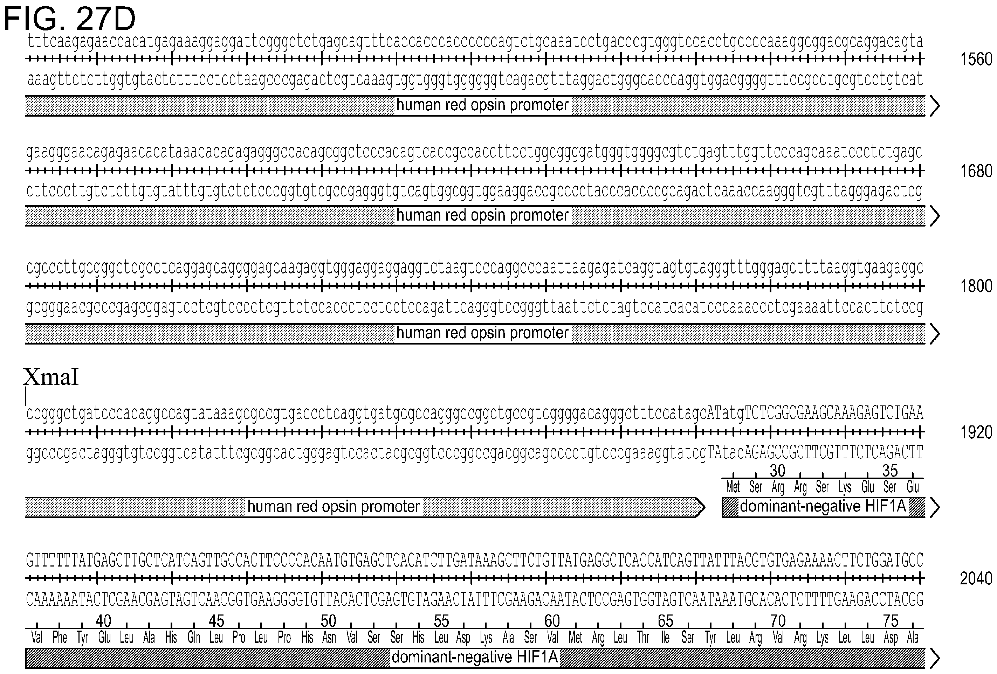

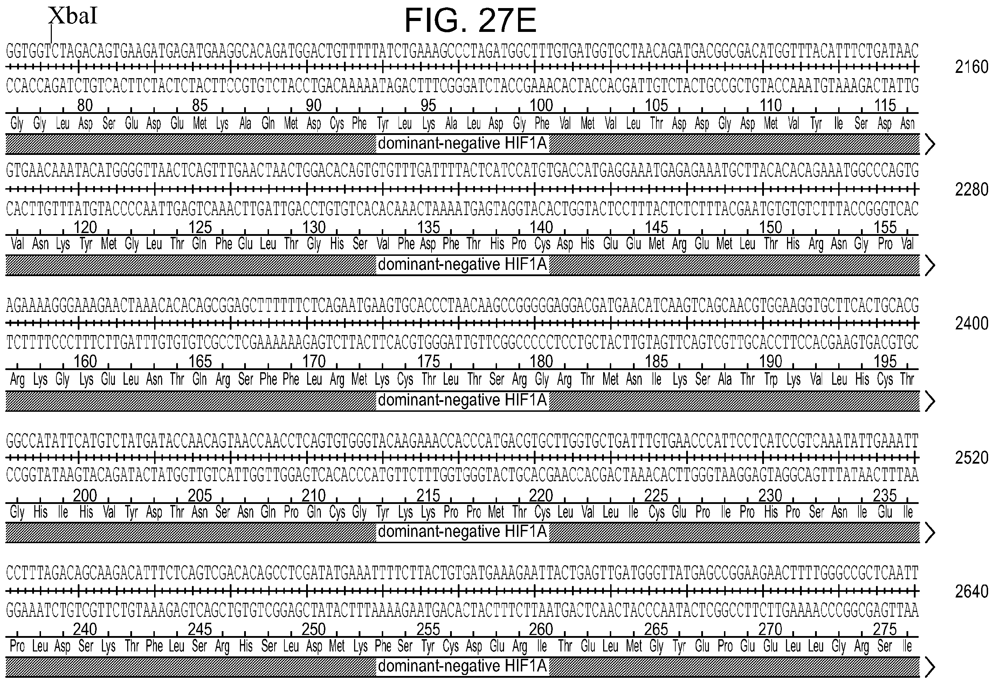

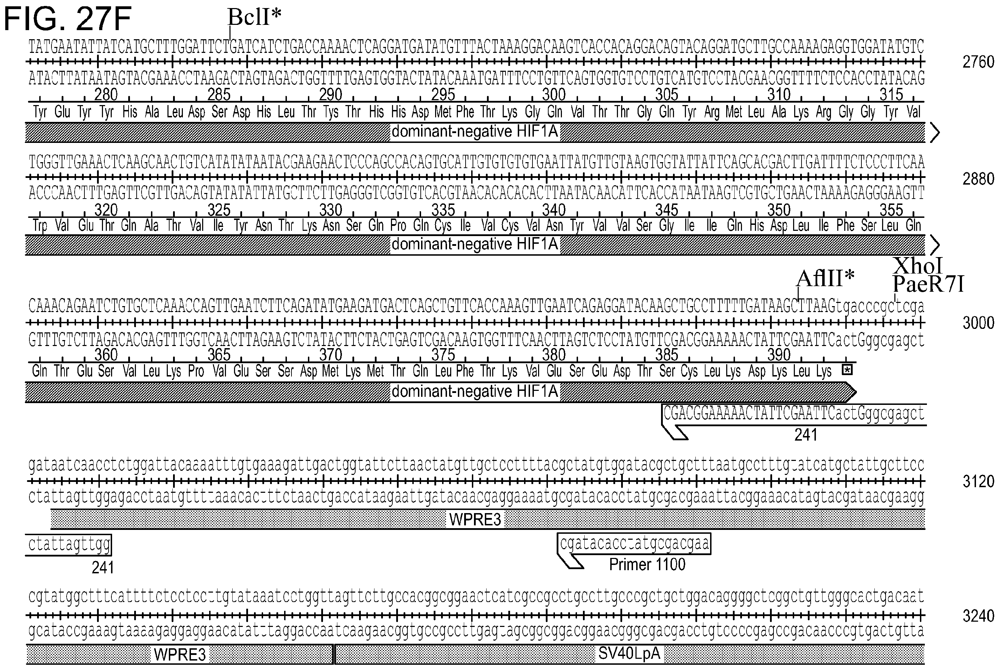

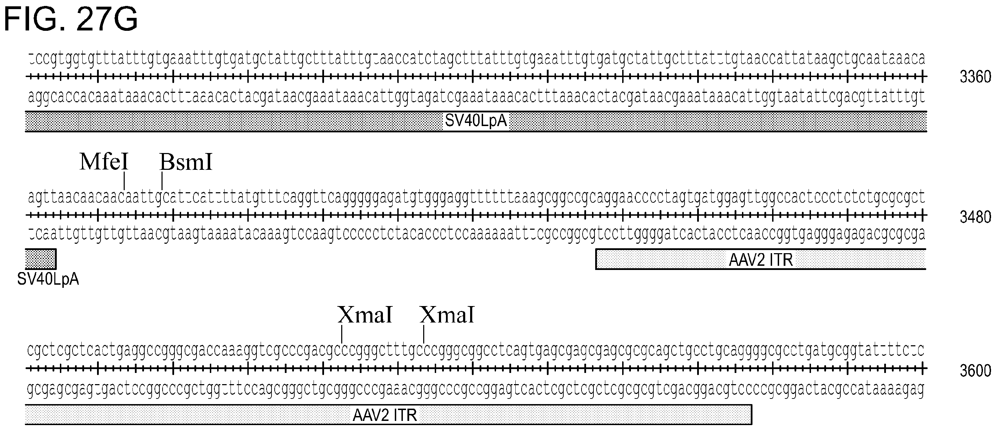

**FIGS. 27A-27G** depict an exemplary vector map of an exemplary AAV vector of the invention comprising RedO promoter and a nucleic acid molecule encoding dominant negative hypoxia inducible factor 1 subunit alpha (HIF1α). FIGS. 27B-G disclose SEQ ID NOS 160-162 and 48, respectively, in order of appearance.

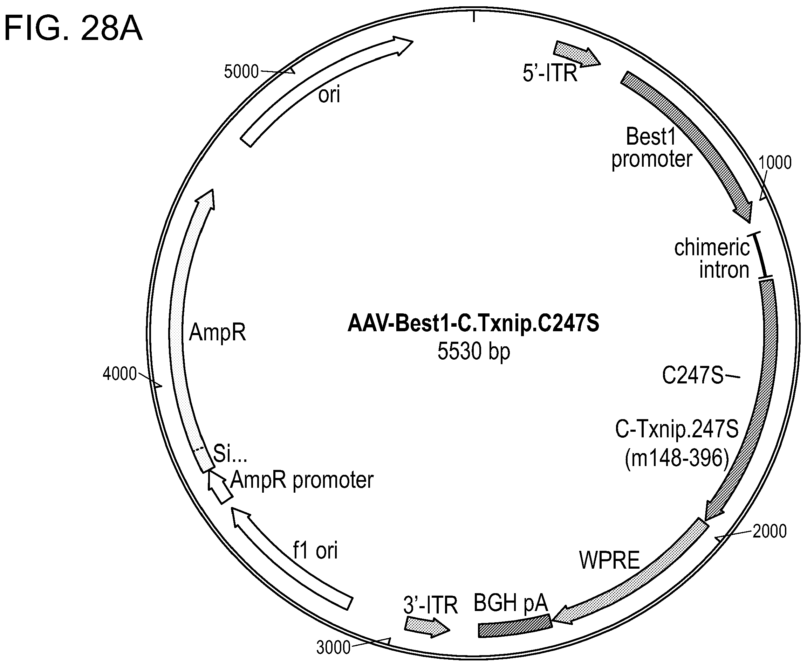

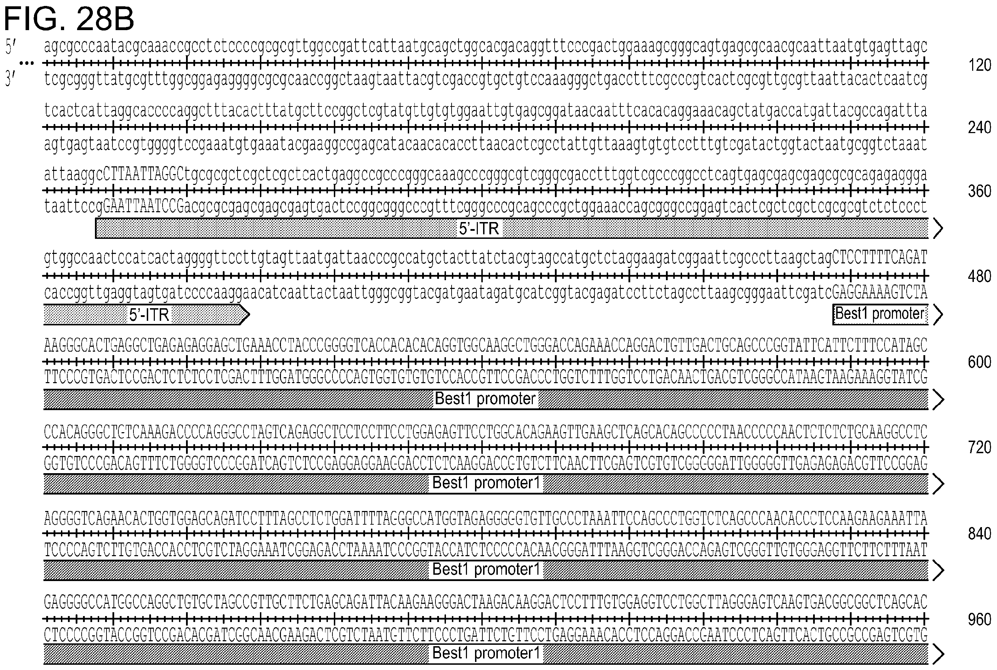

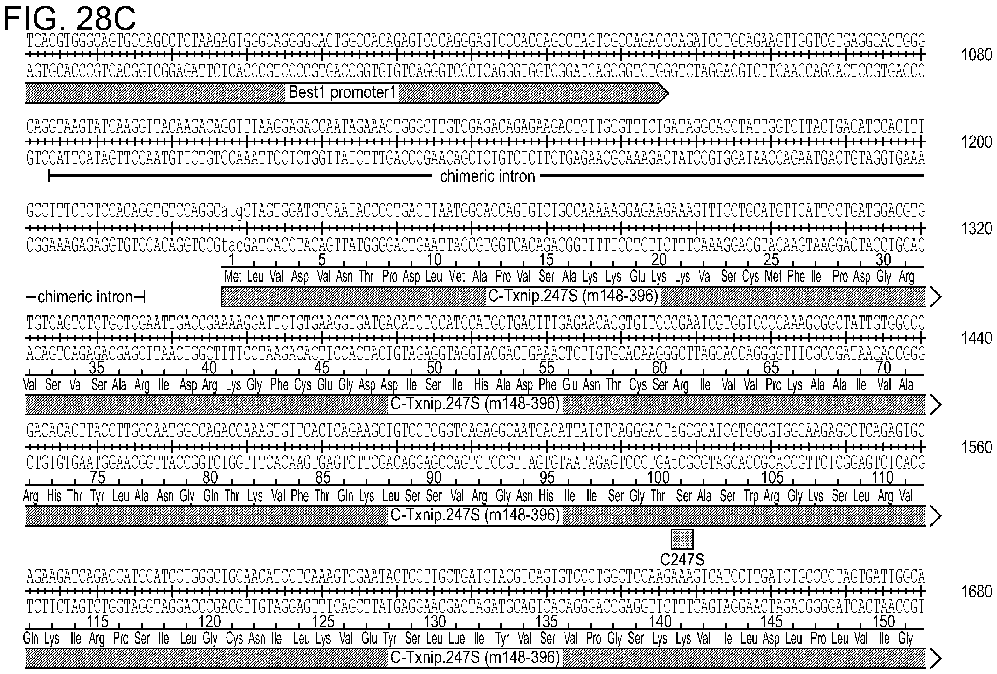

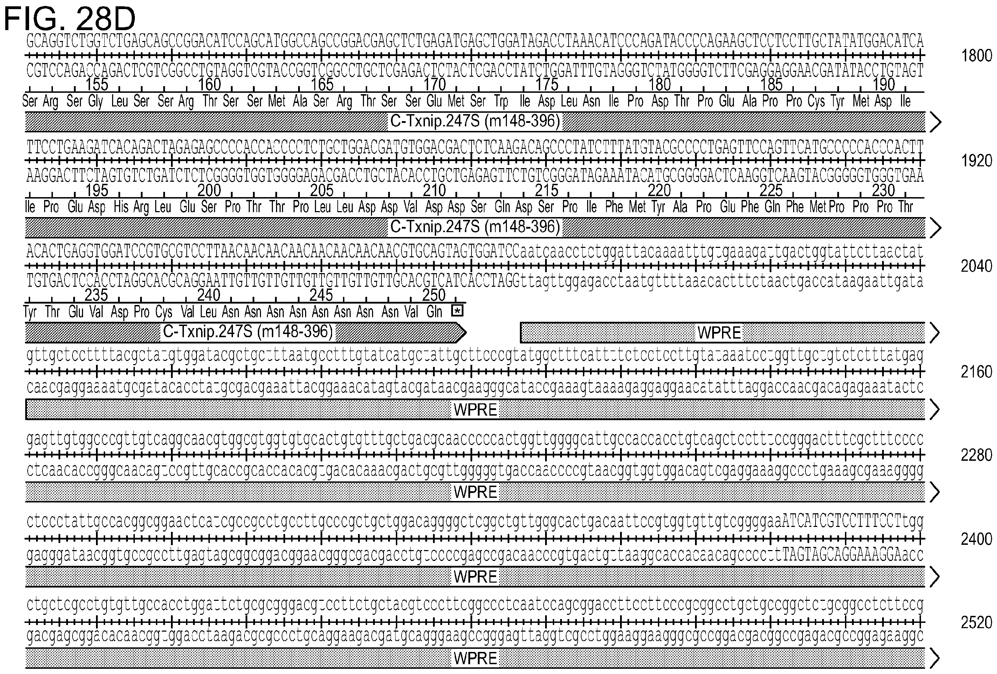

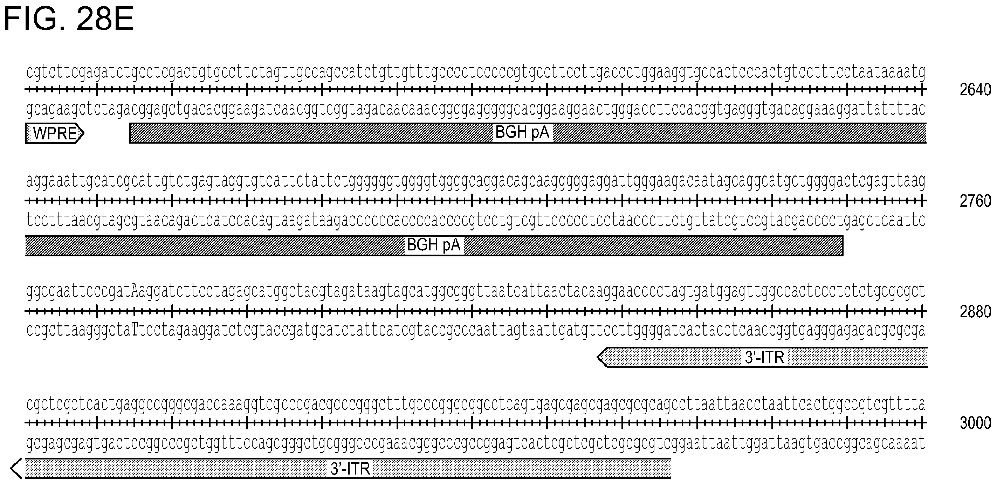

**FIGS. 28A-28E** depict an exemplary vector map of an exemplary AAV vector of the invention comprising a human bestrophin 1 (hBest1) promoter and a nucleic acid molecule encoding C247S variant thioredoxin-interacting protein (TXNIP). FIGS. 28B-E disclose SEQ ID NOS 163-164, respectively, in order of appearance.

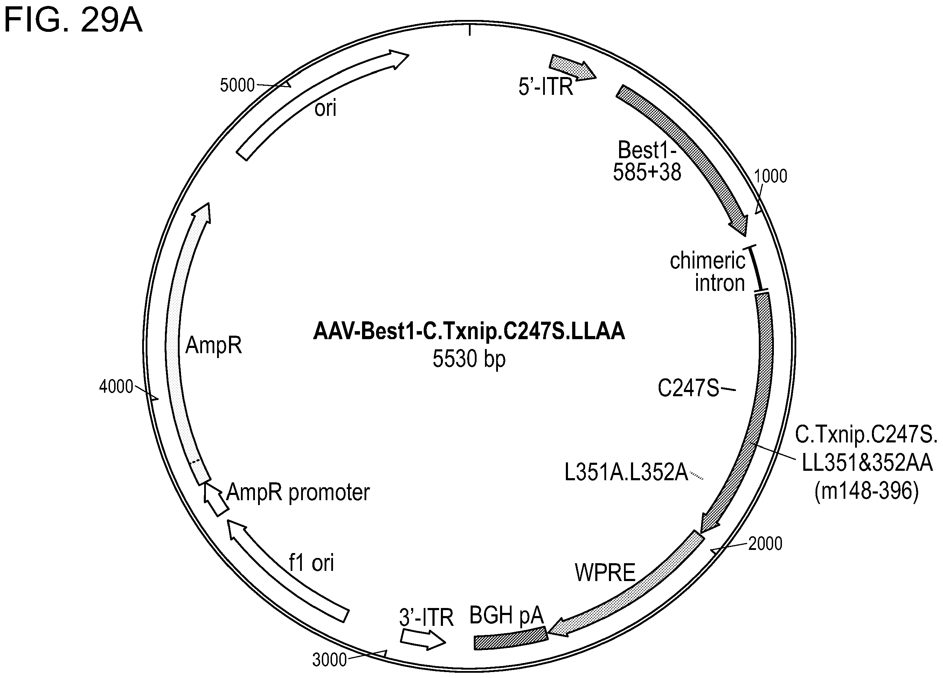

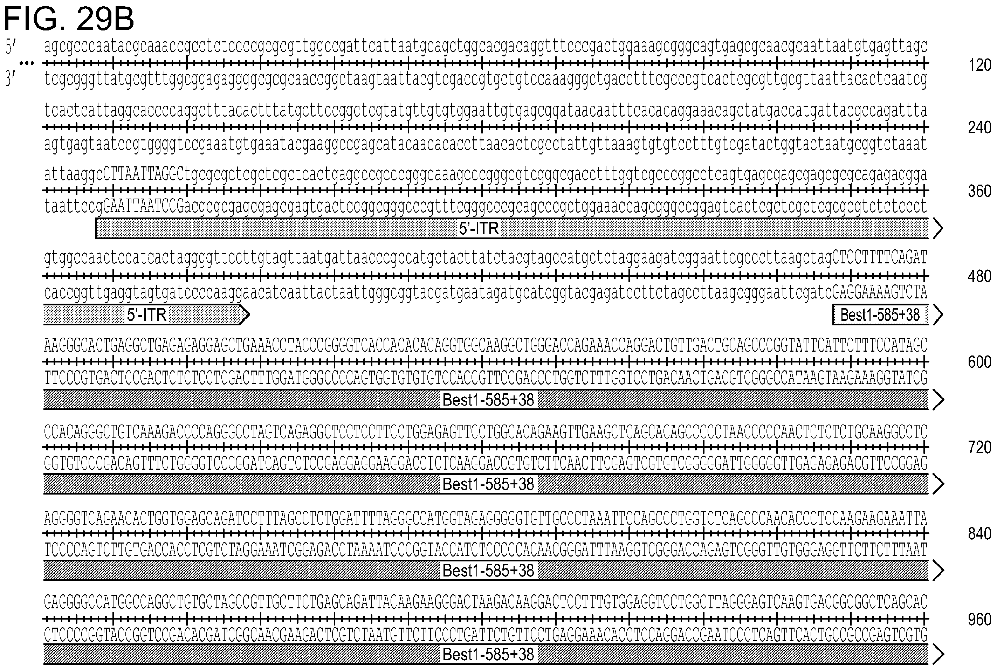

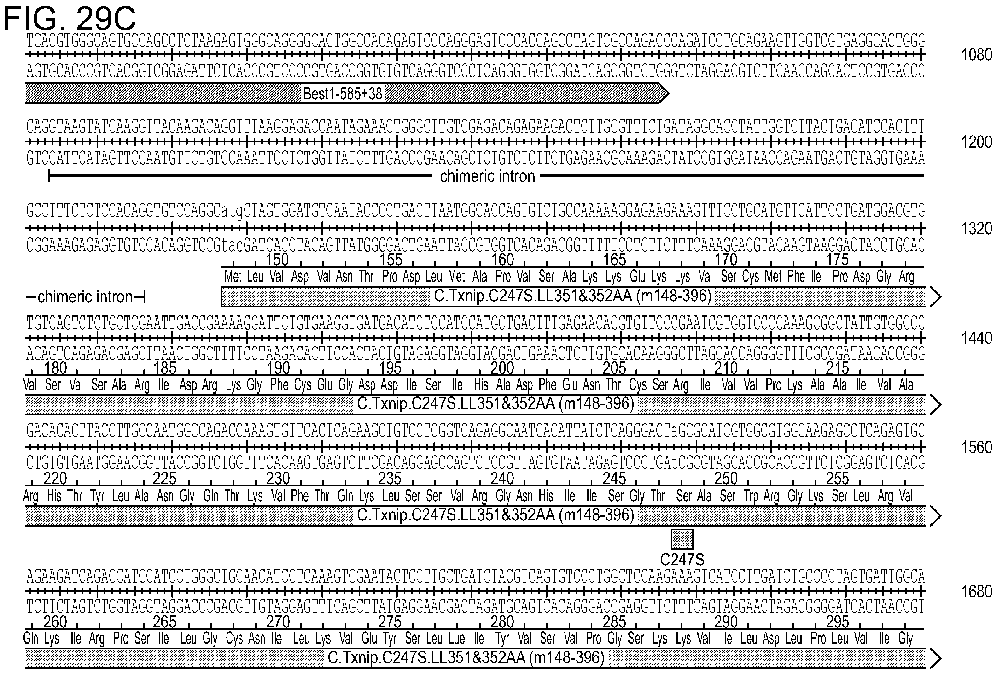

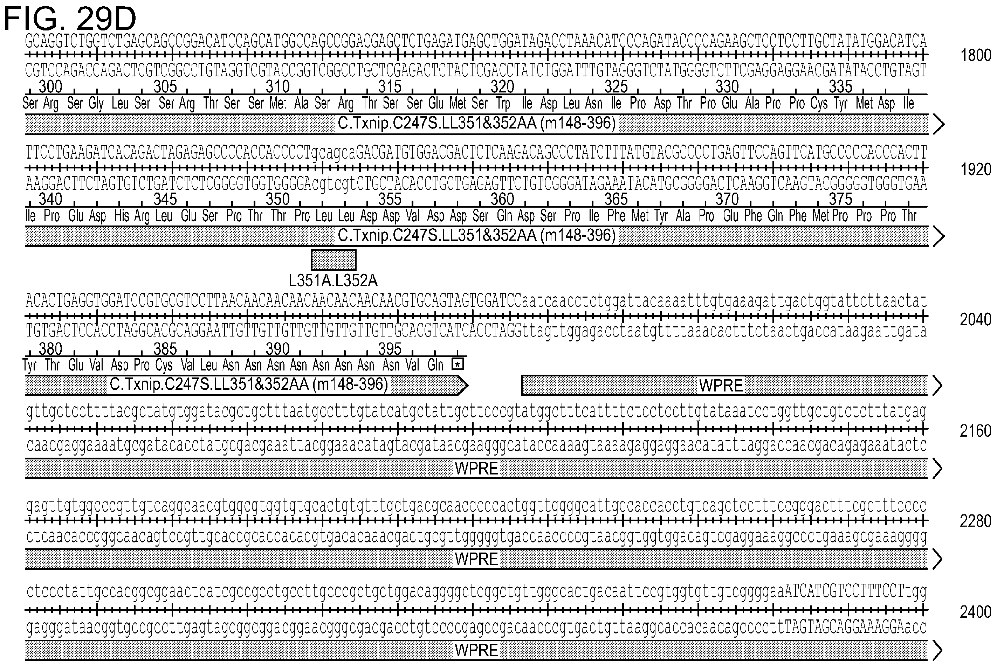

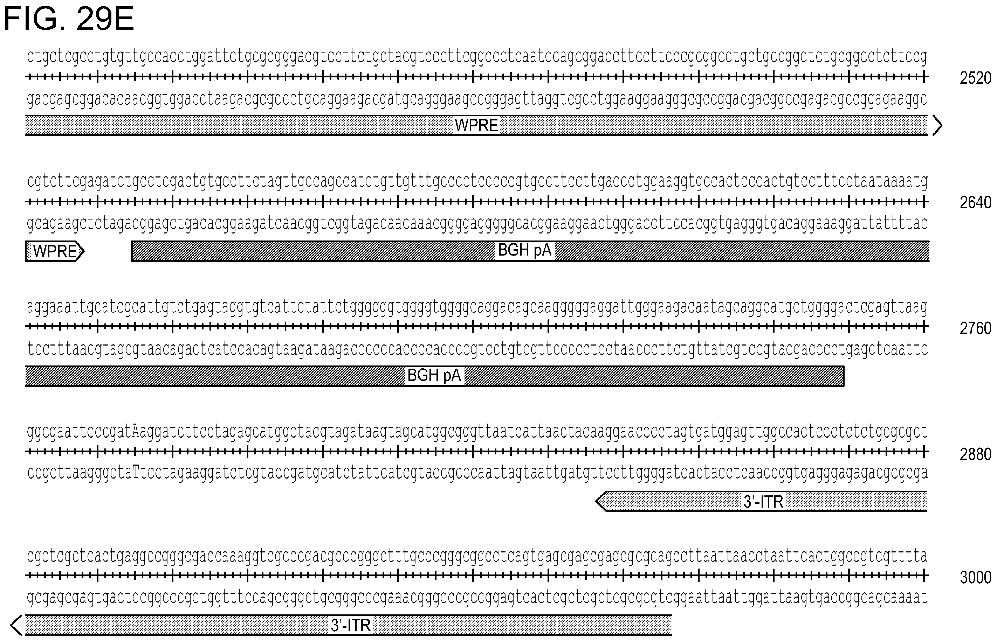

**FIGS. 29A-29E** depict an exemplary vector map of an exemplary AAV vector of the invention comprising a human bestrophin 1 (hBest1) promoter and a nucleic acid molecule encoding C247S.LL351-352AA variant thioredoxin-interacting protein (TXNIP). FIGS. 29B-E disclose SEQ ID NOS 165-166, respectively, in order of appearance.

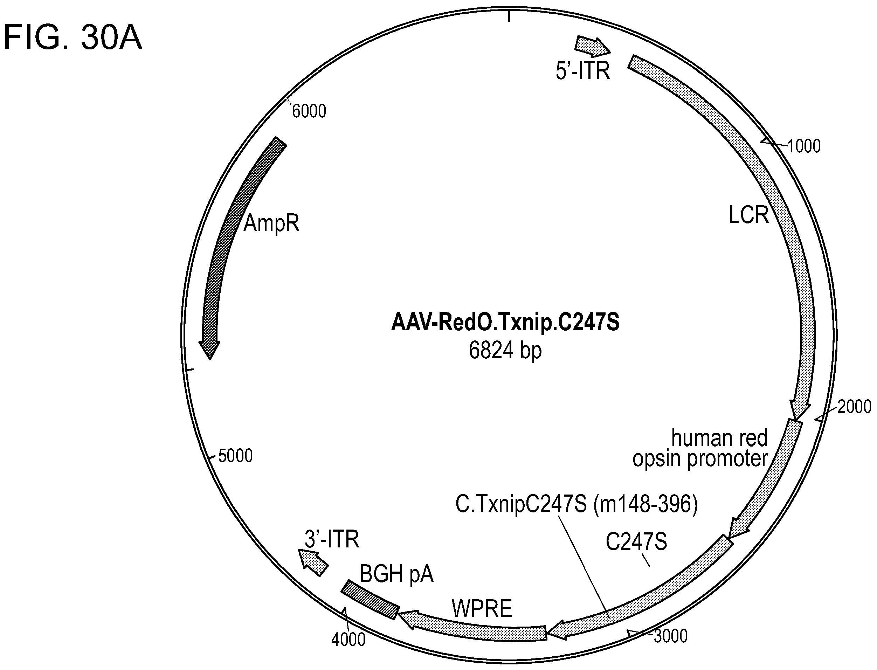

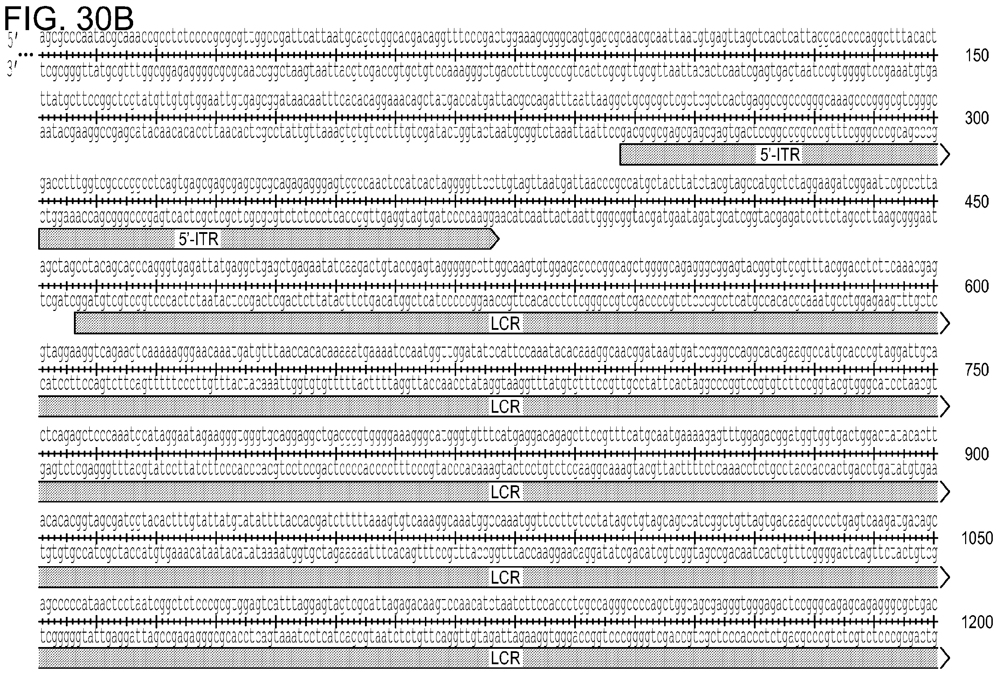

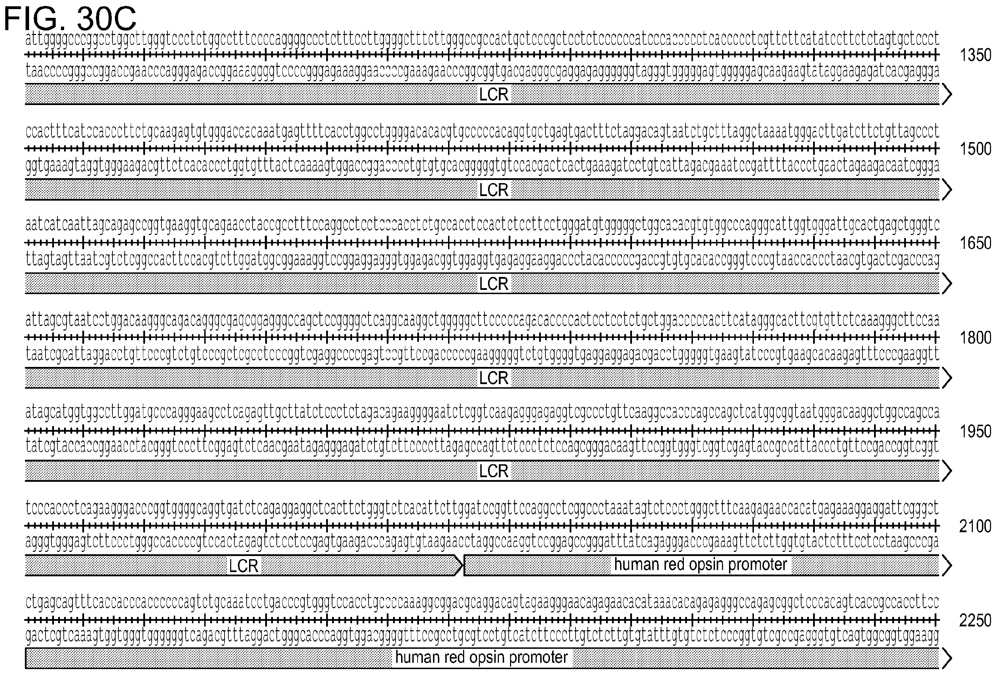

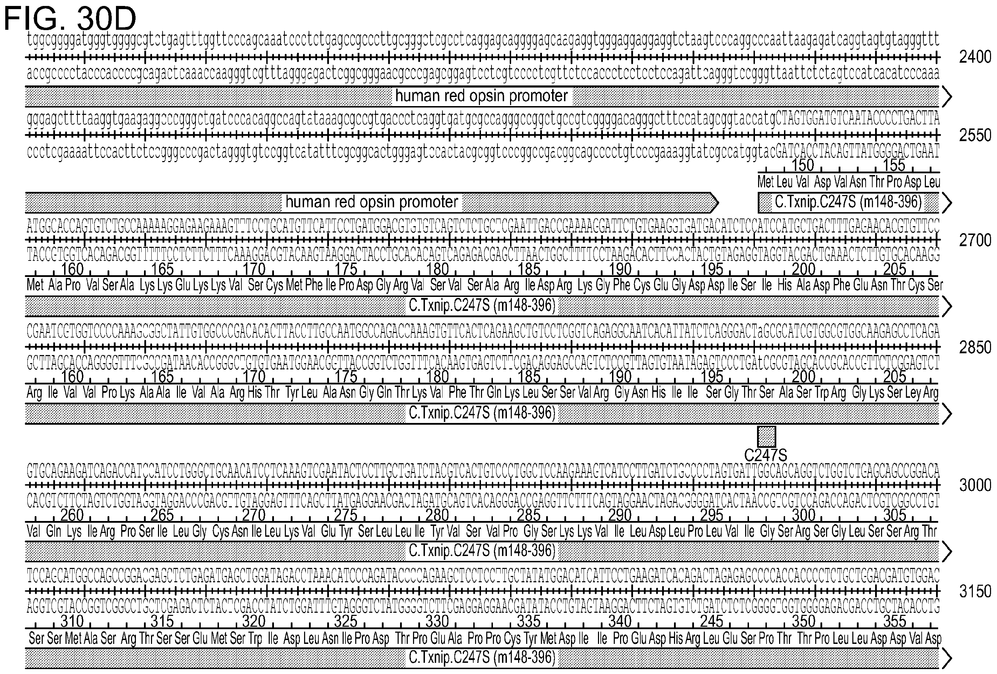

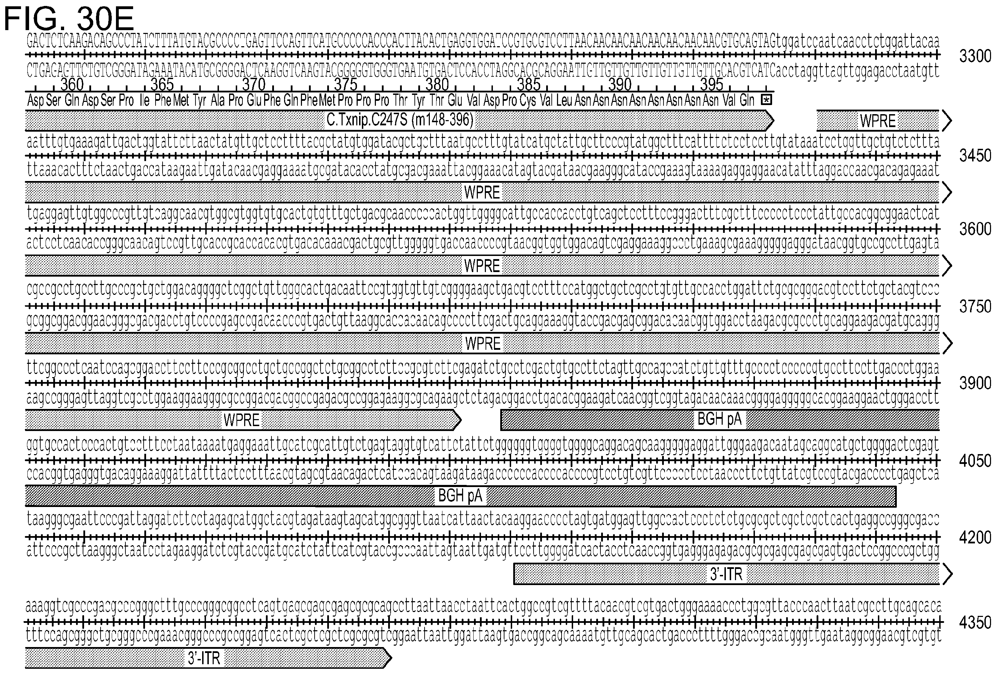

**FIGS. 30A-30E** depict an exemplary vector map of an exemplary AAV vector of the invention comprising a RedO promoter and a nucleic acid molecule encoding C247S variant thioredoxin-interacting protein (TXNIP). FIGS. 30B-E disclose SEQ ID NOS 167 and 157, respectively, in order of appearance.

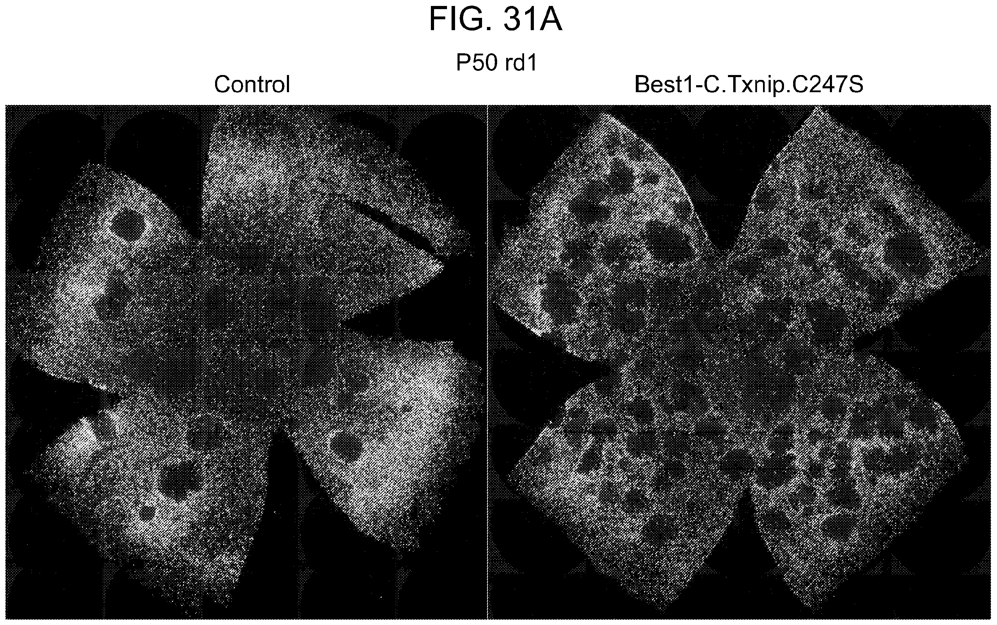

**FIG. 31A** are images of P50 *rd1* flat-mounted retinas with H2BGFP labeled cones treated with control or AAV8-Best1-C.Txnip C247S.

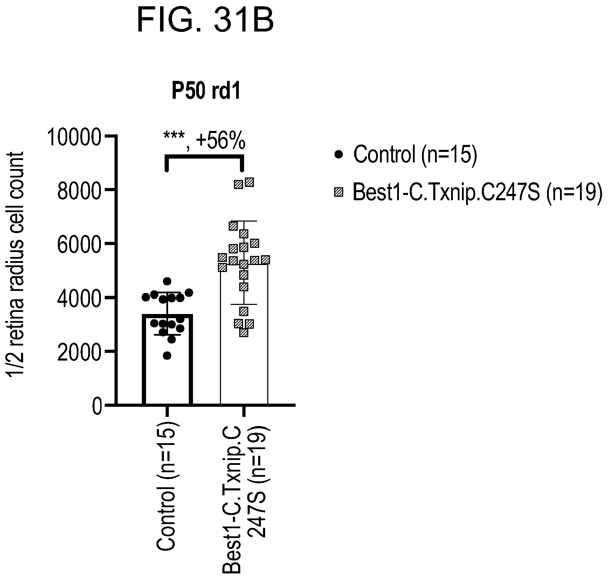

**FIG. 31B** is a graph depicting the quantification of H2BGFP-positive cones within the half radius of P50 rd1 retinas treated with control or AAV8-Best1-C, Txnip C247S.

### DETAILED DESCRIPTION OF THE INVENTION

The present invention is based, at least in part on the discovery of mutation-independent compositions and methods of treatment for subjects having RP.

More specifically, it has surprisingly been discovered that intraocular delivery of adeno-associated virus (AAV) comprising a thioredoxin interacting protein (TXNIP) variant that cannot bind thioredoxin (C247S) prolongs survival of cones in RP-mutant mice. Even more surprising, this C247S variant TXNIP-mediated effect was only observed when the TXNIP variant was specifically expressed in cones. In addition, it has been surprisingly discovered that a serine at amino acid residue 308 is required for this effect as replacing this residue with alanine abolishes the enhanced survival of cones resulting from the C247S variant. It has also surprisingly been discovered that overexpression of C247S variant TXNIP increases RP cone mitochondria size and function. Further, it has been supriringly discoved that overexpression of C247S variant TXNIP in combination with overexpression of nuclear factor erythroid 2-like 2 (Nrf2) prolongs survival of cones in RP-mutant mice further than overexpression of either C247S TXNIP or Nrf2 alone. It has also been surprising discovered that expression of a C247S.LL351&352AA variant of TXNIP driven by a photoreceptor-specific (PR- specific) promoter in either cones or retinal pigment epithelium (RPE) also improves cone survival. Moreover, expression of a dominant-negative allele of hypoxia inducible factor 1 subunit alpha (HIF1α) in cone cells was surprisingly found to also prolong cone surivival.

Accordingly, the present disclosure provides compositions, e.g., pharmaceutical compositions, which include a recombinant adeno-associated virus (AAV) vector, and methods of treating a subject having a degenerative ocular disorder, e.g., retinitis pigmentosa.

Various aspects of the invention are described in further detail in the following subsections:

### I. DEFINITIONS

As used herein, each of the following terms has the meaning associated with it in this section.

The articles "a" and "an" are used herein to refer to one or to more than one (*i.e.,* to at least one) of the grammatical object of the article. By way of example, "an element" means one element or more than one element, e.g., a plurality of elements.

The term "including" is used herein to mean, and is used interchangeably with, the phrase "including but not limited to".

The term "or" is used herein to mean, and is used interchangeably with, the term "and/or," unless context clearly indicates otherwise.

As used herein, the term "nucleic acid molecule" is intended to include DNA molecules (e.g., cDNA or genomic DNA) and RNA molecules (e.g., mRNA) and analogs of the DNA or RNA generated using nucleotide analogs. The nucleic acid molecule can be single-stranded or double-stranded, but preferably is double-stranded DNA. A nucleic acid molecule used in the methods of the present invention can be isolated using standard molecular biology techniques. Using all or portion of a nucleic acid sequence of interest as a hybridization probe, nucleic acid molecules can be isolated using standard hybridization and cloning techniques (e.g., as described in Sambrook, J., Fritsh, E. F., and Maniatis, T. Molecular Cloning. A Laboratory Manual. 2nd, ed., Cold Spring Harbor Laboratory, Cold Spring Harbor Laboratory Press, Cold Spring Harbor, N.Y., 1989).

An "isolated" nucleic acid molecule is one which is separated from other nucleic acid molecules which are present in the natural source of the nucleic acid. For example, with regards to genomic DNA, the term "isolated" includes nucleic acid molecules which are separated from the chromosome with which the genomic DNA is naturally associated. Preferably, an "isolated" nucleic acid molecule is free of sequences which naturally flank the nucleic acid molecule *(i.e.,* sequences located at the 5' and 3' ends of the nucleic acid molecule) in the genomic DNA of the organism from which the nucleic acid molecule is derived.

A nucleic acid molecule for use in the methods of the invention can also be isolated by the polymerase chain reaction (PCR) using synthetic oligonucleotide primers designed based upon the sequence of a nucleic acid molecule of interest. A nucleic acid molecule used in the methods of the invention can be amplified using cDNA, mRNA or, alternatively, genomic DNA as a template and appropriate oligonucleotide primers according to standard PCR amplification techniques. Furthermore, oligonucleotides corresponding to nucleotide sequences of interest can be prepared by standard synthetic techniques, e.g., using an automated DNA synthesizer.

The nucleic acids for use in the methods of the invention can also be prepared, e.g., by standard recombinant DNA techniques. A nucleic acid of the invention can also be chemically synthesized using standard techniques. Various methods of chemically synthesizing polydeoxynucleotides are known, including solid-phase synthesis which has been automated in commercially available DNA synthesizers (See *e.g.,* Itakura et al. U.S. Patent No. 4,598,049; Caruthers et al. U.S. Patent No. 4,458,066; and Itakura U.S. Patent Nos. 4,401,796 and 4,373,071).

As used herein, the term "vector" refers to a nucleic acid molecule capable of transporting another nucleic acid to which it has been linked. One type of vector is a "plasmid", which refers to a circular double stranded DNA loop into which additional DNA segments may be ligated. Another type of vector is a viral vector, wherein additional DNA segments may be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) are integrated into the genome of a host cell upon introduction into the host cell, and thereby are replicated along with the host genome. Moreover, certain vectors are capable of directing the expression of genes or nucleic acid molecules to which they are operatively linked and are referred to as "expression vectors" or "recombinant expression vectors.". Nucleic acid sequences necessary for expression in prokaryotes usually include a promoter, an operator (optional), and a ribosome binding site, often along with other sequences. Eukaryotic cells are known to utilize promoters, enhancers, and termination and polyadenylation signals. **In** some embodiments, "expression vectors" are used in order to permit pseudotyping of the viral envelope proteins.

Expression vectors are often in the form of plasmids. **In** the present specification, "plasmid" and "vector" may be used interchangeably as the plasmid is the most commonly used form of vector. However, the invention is intended to include such other forms of expression vectors, such as viral vectors (e.g., replication defective retroviruses, adenoviruses, adeno-associated viruses, lentiviruses), which serve equivalent functions.

The recombinant expression vectors of the invention comprise a nucleic acid of the invention in a form suitable for expression of the nucleic acid in a host cell, which means that the recombinant expression vectors include one or more regulatory sequences, selected on the basis of the host cells to be used for expression, which is operatively linked to the nucleic acid sequence to be expressed. Within a recombinant expression vector, "operably linked" is intended to mean that the nucleotide sequence of interest is linked to the regulatory sequence(s) in a manner which allows for expression of the nucleotide sequence *(e.g.,* in an *in vitro* transcription/translation system or in a host cell when the vector is introduced into the host cell). The term "regulatory sequence" is intended to include promoters, enhancers and other expression control elements (e.g., polyadenylation signals). Such regulatory sequences are described, for example, in Goeddel; Gene Expression Technology: Methods in Enzymology 185, Academic Press, San Diego, Calif. (1990). Regulatory sequences include those which direct constitutive expression of a nucleotide sequence in many types of host cells, those which are constitutively active, those which are inducible, and those which direct expression of the nucleotide sequence only in certain host cells (e.g., tissue-specific regulatory sequences). The expression vectors of the invention can be introduced into host cells to thereby produce proteins or portions thereof, including fusion proteins or portions thereof, encoded by nucleic acids as described herein.

The terms "transformation," "transfection," and "transduction" refer to introduction of a nucleic acid, *e.g.,* a viral vector, into a recipient cell.

As used herein, the term "subject" includes warm-blooded animals, preferably mammals, including humans. **In** a preferred embodiment, the subject is a primate. **In** an even more preferred embodiment, the primate is a human.

As used herein, the various forms of the term "modulate" are intended to include stimulation *(e.g.,* increasing or upregulating a particular response or activity) and inhibition *(e.g.,* decreasing or downregulating a particular response or activity).

As used herein, the term "contacting" *(i.e.,* contacting a cell with an agent) is intended to include incubating the agent and the cell together *in vitro (e.g.,* adding the agent to cells in culture) or administering the agent to a subject such that the agent and cells of the subject are contacted *in vivo.* The term "contacting" is not intended to include exposure of cells to an agent that may occur naturally in a subject (i.e., exposure that may occur as a result of a natural physiological process).

As used herein, the term "administering" to a subject includes dispensing, delivering or applying a composition of the invention to a subject by any suitable route for delivery of the composition to the desired location in the subject, including delivery by intraocular administration or intravenous administration. Alternatively or in combination, delivery is by the topical, parenteral or oral route, intracerebral injection, intramuscular injection, subcutaneous/intradermal injection, intravenous injection, buccal administration, transdermal delivery and administration by the rectal, colonic, vaginal, intranasal or respiratory tract route.

As used herein, the term "degenerative ocular disorder" refers generally to a disorder of the retina. In one embodiment, the degenerative ocular disorder is associated with death, of cone cells, and/ or rod cells. Moreover, in a particular embodiment, a degenerative ocular disorder is not associated with blood vessel leakage and/or growth, for example, as is the case with diabetic retinopathy, but, instead is characterized primarily by reduced viability of cone cells and/ or rod cells. In certain embodiments, the degenerative ocular disorder is a genetic or inherited disorder. In a particular embodiment, the degenerative ocular disorder is retinitis pigmentosa. In another embodiment, the degenerative ocular disorder is age-related macular degeneration. In another embodiment, the degenerative ocular disorder is cone-rod dystrophy. In another embodiment, the degenerative ocular disorder is rod-cone dystrophy. In other embodiments, the degenerative ocular disorder is not associated with blood vessel leakage and/or growth. In certain embodiments, the degenerative ocular disorder is not associated with diabetes and/or diabetic retinopathy. In further embodiments, the degenerative ocular disorder is not NARP (neuropathy, ataxia, and retinitis pigmentosa). In yet further embodiments, the degenerative ocular disorder is not a neurological disorder. In certain embodiments, thedegenerative ocular disorder is not a disorder that is associated with a compromised optic nerve and/or disorders of the brain. In the foregoing embodiments, the degenerative ocular disorder is associated with a compromised photoreceptor cell, and is not a neurological disorder.

As used herein, the term "retinitis pigmentosa" or "RP" is known in the art and encompasses a disparate group of genetic disorders of rods and cones. Retinitis pigmentosa generally refers to retinal degeneration often characterized by the following manifestations: night blindness, progressive loss of peripheral vision, eventually leading to total blindness; ophthalmoscopic changes consist in dark mosaic-like retinal pigmentation, attenuation of the retinal vessels, waxy pallor of the optic disc, and in the advanced forms, macular degeneration. In some cases there can be a lack of pigmentation. Retinitis pigmentosa can be associated to degenerative opacity of the vitreous body, and cataract. Family history is prominent in retinitis pigmentosa; the pattern of inheritance may be autosomal recessive, autosomal dominant, or X-linked; the autosomal recessive form is the most common and can occur sporadically.

As used herein, the terms "Cone-Rod Dystrophy" or "CRD" and "Rod-Cone Dystrophy" or "RCD" refer to art recognized inherited progressive diseases that cause deterioration of the cone and rod photoreceptor cells and often result in blindness. CRD is characterized by reduced viability or death of cone cells followed by reduced viability or death of rod cells. By contrast, RCD is characterized by reduced viability or death of rod cells followed by reduced viability or death of cone cells.

As used herein, the term "age-related macular degeneration" also referred to as "macular degeneration" or "AMD", refers to the art recognized pathological condition which causes blindness amongst elderly individuals. Age related macular degeneration includes both wet and dry forms of AMD. The dry form of AMD, which accounts for about 90 percent of all cases, is also known as atrophic, nonexudative, or drusenoid (age-related) macular degeneration. With the dry form of AMD, drusen typically accumulate in the retinal pigment epithelium (RPE) tissue beneath/within the Bruch's membrane. Vision loss can then occur when drusen interfere with the function of photoreceptors in the macula. The dry form of AMD results in the gradual loss of vision over many years. The dry form of AMD can lead to the wet form of AMD. The wet form of AMD, also known as exudative or neovascular (age-related) macular degeneration, can progress rapidly and cause severe damage to central vision. The macular dystrophies include Stargardt Disease, also known as Stargardt Macular Dystrophy or Fundus Flavimaculatus, which is the most frequently encountered juvenile onset form of macular dystrophy.

"Preventing" or "prevention" refers to a reduction in risk of acquiring a disease or disorder *(i.e.,* causing at least one of the clinical symptoms of the disease not to develop in a patient that may be exposed to or predisposed to the disease but does not yet experience or display symptoms of the disease).

As used herein, the terms "treating" or "treatment" refer to a beneficial or desired result including, but not limited to, alleviation or amelioration of one or more symptoms, diminishing the extent of infection, stabilized (i.e., not worsening) state of infection, amelioration or palliation of the infectious state, whether detectable or undetectable. "Treatment" can also mean prolonging survival as compared to expected survival in the absence of treatment.

Various additional aspects of the methods of the invention are described in further detail in the following subsections.

### II. COMPOSITIONS OF THE INVENTION

The present invention provides adeno-associated viral (AAV) expression cassettes, AAV expression cassetes present in AAV vectors, and AAV vectors comprising a recombinant viral genome which include an expression cassette.

Accordingly, in one aspect the present invention provides compositions comprising an adeno-associated virus (AAV) expression cassette, the expression cassette comprising a promoter and a nucleic acid molecule encoding a C247S variant thioredoxin-interacting protein (TXNIP).

The promoter is a cone-specific promoter. The cone-specific promoter is a human red opsin (RedO) promoter.

In some embodiments, the expression cassettes of the invention further comprise an intron, such as an intron between the promoter and the nucleic acid molecule encoding TXNIP.

In some embodiments of the invention, the expression cassettes of the invention further comprise expression control sequences including, but not limited to, appropriate transcription sequences *(i.e.* initiation, termination, and enhancer), efficient RNA processing signals *(e.g.* splicing and polyadenylation (polyA) signals), sequences that stabilize cytoplasmic mRNA, sequences that code for a transcriptional enhancer, sequences that code for a posttranscriptional enhancer, sequences that enhance translation efficiency *(i.e.* Kozak consensus sequence), sequences that enhance protein stability, and when desired, sequences that enhance secretion of the encoded product.

The terms "adeno-associated virus", "AAV virus", "AAV virion", "AAV viral particle", and "AAV particle", as used interchangeably herein, refer to a viral particle composed of at least one AAV capsid protein (preferably by all of the capsid proteins of a particular AAV serotype) and an encapsidated polynucleotide AAV genome. If the particle comprises a heterologous polynucleotide *(i.e.* a polynucleotide other than a wild-type AAV genome such as a transgene to be delivered to a mammalian cell) flanked by the AAV inverted terminal repeats (ITRs), it is typically referred to as an "AAV vector particle."

AAV viruses belonging to the genus *Dependovirus* of the *Parvoviridae* family and, as used herein, include any serotype of the over 100 serotypes of AAV viruses known. In general, serotypes of AAV viruses have genomic sequences with a significant homology at the level of amino acids and nucleic acids, provide an identical series of genetic functions, produce virions that are essentially equivalent in physical and functional terms, and replicate and assemble through practically identical mechanisms.

The AAV genome is approximately 4.7 kilobases long and is composed of single-stranded deoxyribonucleic acid (ssDNA) which may be either positive- or negative-sensed. The genome comprises inverted terminal repeats (ITRs) at both ends of the DNA strand, and two open reading frames (ORFs): rep and cap. The rep frame is made of four overlapping genes encoding Rep proteins required for the AAV life cycle. The cap frame contains overlapping nucleotide sequences of capsid proteins: VP 1, VP2 and VP3, which interact together to form a capsid of an icosahedral symmetry. See Carter B, Adeno-associated virus and adeno- associated virus vectors for gene delivery, Lassie D, et ah, Eds., "Gene Therapy: Therapeutic Mechanisms and Strategies" (Marcel Dekker, Inc., New York, NY, US, 2000) and Gao G, et al, J. Virol. 2004; 78(12):6381-6388.

The term "AAV vector" or "AAV construct" refers to a vector derived from an adeno-associated virus serotype, including without limitation, AAV-1, AAV-2, AAV-3, AAV-4, AAV-5, AAV6, AAV7, AAV8, and AAV9. "AAV vector" refers to a vector that includes AAV nucleotide sequences as well as heterologous nucleotide sequences. AAV vectors require only the 145 base terminal repeats in cis to generate virus. All other viral sequences are dispensable and may be supplied in trans (Muzyczka (1992) Curr. Topics Microbiol. Immunol. 158:97-129). Typically, the rAAV vector genome will only retain the inverted terminal repeat (ITR) sequences so as to maximize the size of the transgene that can be efficiently packaged by the vector. The ITRs need not be the wild-type nucleotide sequences, and may be altered, e.g., by the insertion, deletion or substitution of nucleotides, as long as the sequences provide for functional rescue, replication and packaging.

In particular embodiments, the AAV vector is an AAV2, AAV2.7m8, AAV2/5 or AAV2/8 vector. Suitable AAV vectors are described in, for example, U.S. Patent No. 7,056,502 and Yan et al. (2002) J. Virology 76(5):2043-2053.

Such AAV vectors can be replicated and packaged into infectious viral particles when present in a host cell that has been transfected with a vector encoding and expressing rep and cap gene products *(i.e.* AAV Rep and Cap proteins), and wherein the host cell has been transfected with a vector which encodes and expresses a protein from the adenovirus open reading frame E4orf6.

The term "cap gene" or "AAV cap gene", as used herein, refers to a gene that encodes a Cap protein. The term "Cap protein", as used herein, refers to a polypeptide having at least one functional activity of a native AAV Cap protein (e.g. VP1, VP2, VP3). Examples of functional activities of Cap proteins (e.g. VP1, VP2, VP3) include the ability to induce formation of a capsid, facilitate accumulation of single-stranded DNA, facilitate AAV DNA packaging into capsids *(i.e.* encapsidation), bind to cellular receptors, and facilitate entry of the virion into host.

The term "capsid", as used herein, refers to the structure in which the viral genome is packaged. A capsid consists of several oligomeric structural subunits made of proteins. For instance, AAV have an icosahedral capsid formed by the interaction of three capsid proteins: VP1, VP2 and VP3.

The term "genes providing helper functions", as used herein, refers to genes encoding polypeptides which perform functions upon which AAV is dependent for replication *(i.e.* "helper functions"). The helper functions include those functions required for AAV replication including, without limitation, those moieties involved in activation of AAV gene transcription, stage specific AAV mRNA splicing, AAV DNA replication, synthesis of cap expression products, and AAV capsid assembly. Viral- based accessory functions can be derived from any of the known helper viruses such as adenovirus, herpesvirus (other than herpes simplex virus type-1), and vaccinia virus. Helper functions include, without limitation, adenovirus E1, E2a, VA, and E4 or herpesvirus UL5, UL8, UL52, and UL29, and herpesvirus polymerase. In one embodiemtn, a helper function does not include adenovirus E1.

The term "rep gene" or "AAV rep gene", as used herein, refers to a gene that encodes a Rep protein. The term "Rep protein", as used herein, refers to a polypeptide having at least one functional activity of a native AAV Rep protein (e.g. Rep 40, 52, 68, 78). A "functional activity" of a Rep protein (e.g. Rep 40, 52, 68, 78) is any activity associated with the physiological function of the protein, including facilitating replication of DNA through recognition, binding and nicking of the AAV origin of DNA replication as well as DNA helicase activity. Additional functions include modulation of transcription from AAV (or other heterologous) promoters and site- specific integration of AAV DNA into a host chromosome.

The term "adeno-associated virus ITRs" or "AAV ITRs", as used herein, refers to the inverted terminal repeats present at both ends of the DNA strand of the genome of an adeno-associated virus. The ITR sequences are required for efficient multiplication of the AAV genome. Another property of these sequences is their ability to form a hairpin. This characteristic contributes to its self-priming which allows the primase- independent synthesis of the second DNA strand. The ITRs have also shown to be required for efficient encapsidation of the AAV DNA combined with generation of fully assembled, deoxyribonuclease- resistant AAV particles.

The term "expression cassette", as used herein, refers to a nucleic acid construct, generated recombinantly or synthetically, with a series of specified nucleic acid elements, which permit transcription of a particular nucleic acid in a target cell.

The expression cassettes of the invention include a promoter operably linked to a nucleic acid molecule encoding a C247S variant thioredoxin-interacting protein (TXNIP). Exemplary expression cassettes of the invention are depicted in FIGs. 20A, 21A, 26A, 27A, 28A, 29A and 30A.

The term "promoter" as used herein refers to a recognition site of a DNA strand to which the RNA polymerase binds. The promoter forms an initiation complex with RNA polymerase to initiate and drive transcriptional activity. The complex can be modified by activating sequences termed "enhancers" or inhibitory sequences termed "silencers".

Suitable promoters for use in the expression cassetees of the invention may be ubiquitous promoters, such as a CMV promoter or an SV40 promoter, but are preferably tissue-specific promoters, *i.e.,* promoters that direct expression of a nucleic acid molecule preferentially in a particular cell type.

The tissue-specific promoter for use in the present invention is a photoreceptor-specific (PR-specific) promoter, a promoter that drives expression which is substantially retricted to photoreceptor cells and/or retinal pigment epithelial cells . A PR-specific promoter may be a rod-specific promoter; a cone-specific promoter; or a rod- and cone-specific promoter. The tissue-specific promoter for use in the present invention is a cone-specific promoter.

Suitable PR-specific promoters are known in the art and include, for example, a human red opsin, a guanine nucleotide-binding protein G subunit alpha-2 (GNAT2) promoter, a human rhodopsin promoter, a human rhodopsin kinase (RK) promoter, a G protein-coupled receptor kinase 1 (GRK1) promoter.

The PR-specific promoter of the invention is a human red opsin (RedO) promoter.

As used interchangeably herein, the terms "human RO," "red opsin," "RedO," "RO," and "hRO" refer to Opsin 1, Long Wave Sensitive, also known as Red Cone Photoreceptor Pigment, Opsin 1 (Cone Pigments), Long-Wave-Sensitive, Cone Dystrophy 5 (X-Linked), Red-Sensitive Opsin, RCP, ROP, Long-Wave-Sensitive Opsin, Color Blindness, Protan, Red Cone Opsin, COD5, CBBm, and CBP. The nucleotide sequence of the genomic region containing the hRO gene (including the region upstream of the coding region of hRO which includes the hRO promoter region) is also known and may be found in, for example, GenBank Reference Sequence NG_009105.2 (SEQ ID NO: 8).

Suitable RedO promoters for use in the present invention include nucleic acid molecules which include nucleotides 452-2017 of SEQ ID NO:8 directly linked, *i.e.,* containing no intervening sequences, to nucleotides 4541-5032 of SEQ ID NO:8; or a nucleotide sequence having about 85%, 86%, 87%, 88%, 89%, 90%, 91%, 92%, 93%, 94%, 95%, 96%, 97%, 98%, or about 99% nucleotide sequence identity to the entire nucleotide sequence of nucleotides 452-2017 of SEQ ID NO:8 directly linked to nucleotides 4541-5032 of SEQ ID NO:8.

In one embodiment, the RedO promoter comprises the nucleotide sequence of SEQ ID NO:16, or a nucleotide sequence having about 85%, 86%, 87%, 88%, 89%, 90%, 91%, 92%, 93%, 94%, 95%, 96%, 97%, 98%, or about 99% nucleotide sequence identity to the entire nucleotide sequence of SEQ ID NO:16.

In one embodiment, the hRedO promoter comprises nucleotides 457-2514 of SEQ ID NO:26, or a nucleotide sequence having about 85%, 86%, 87%, 88%, 89%, 90%, 91%, 92%, 93%, 94%, 95%, 96%, 97%, 98%, or about 99% nucleotide sequence identity to the entire nucleotide sequence of nucleotides 457-2514 of SEQ ID NO:26.

In another embodiment, the hRedO promoter comprises nucleotides 457-2514 of SEQ ID NO: 49, or a nucleotide sequence having about 85%, 86%, 87%, 88%, 89%, 90%, 91%, 92%, 93%, 94%, 95%, 96%, 97%, 98%, or about 99% nucleotide sequence identity to the entire nucleotide sequence of nucleotides 457-2514 of SEQ ID NO: 49.

In another embodiment, the hRedO promoter comprises the nucleotide sequence of SEQ ID NO:119, or a nucleotide sequence having about 85%, 86%, 87%, 88%, 89%, 90%, 91%, 92%, 93%, 94%, 95%, 96%, 97%, 98%, or about 99% nucleotide sequence identity to the entire nucleotide sequence of SEQ ID NO:119.

As used interchangeably herein, the terms "GNAT2" and "guanine nucleotide-binding protein G subunit alpha-2 (GNAT2) promoter" also known as G Protein Subunit Alpha Transducin 2, also known as Guanine Nucleotide Binding Protein (G Protein), Alpha Transducing Activity Polypeptide 2, Guanine Nucleotide-Binding Protein G(T) Subunit Alpha-2, Transducin Alpha-2 Chain, GNATC ,Transducin, Cone-Specific, Alpha Polypeptide, Cone-Type Transducin Alpha Subunit, and ACHM4, refers to the well-known G protein that stimulates the coupling of rhodopsin and cGMP-phoshodiesterase during visual impulses. The nucleotide sequence of the genomic region containing the human GNAT2 gene (including the region upstream of the coding region of human GNAT2 gene which includes the GNAT2 promoter region) is also known and may be found in, for example, GenBank Reference Sequence NC_000001.11 (SEQ ID NO: 9).

A further tissue-specific promoter may be a retinal pigment epithelium (RPE)-specific promoter. In certain embodiments, a suitable RPE-specific promoter is a human bestrophin 1 (hBest1) promoter.

As used interchangeably herein, the terms "bestrophin 1," "hBest1," and "hBEST1" refer to bestrophin-1, also known as Bestrophin 1; Vitelliform Macular Dystrophy Protein 2; Best Disease; TU15B; VMD2; Vitelliform Macular Dystrophy 2; Best1V1Delta2; Bestrophin-1; BEST; RP50; ARB; and BMD refers to the gene that is highly and preferentially expressed in the RPE. There are four transcript variants of hBest, the nucleotide and amino acid sequences of which are known and may be found in, for example, GenBank Reference Sequences NM_001139443.1; NM_001300786.1; NM_001300787.1; and NM_004183.3. The nucleotide sequence of the genomic region containing the hBest1 gene (including the region upstream of the coding region of hBest1 which includes the hBest1 promoter region) is also known and may be found in, for example, GenBank Reference Sequence NG _009033.1 (SEQ ID NO:118).

Suitable hBest1 promoters for use in the present invention include nucleic acid molecules which include nucleotides -585 to +38 of the hBestlgene, *(i.e.,* nucleotides 4885-5507 of SEQ ID NO:118); nucleotides -154 to +38 of the hBest1 gene *(i.e.,* nucleotides 5316-5507 of SEQ ID NO:9); or nucleotides -104 to +38 bp of the hBest1 gene *(i.e.,* nucleotides 5366-5507 of SEQ ID NO:9), or or a nucleotide sequence having about 85%, 86%, 87%, 88%, 89%, 90%, 91%, 92%, 93%, 94%, 95%, 96%, 97%, 98%, or about 99% nucleotide sequence identity to the entire nucleotide sequence of nucleotides 4885-5507 of SEQ ID N0:118, nucleotides 5316-5507 of SEQ ID NO:118, or nucleotides 5366-5507 of SEQ ID NO:118. In one embodiement, an hBest1 promoter comprises nucleotides -585 to +38 of the hBestlgene, *(i.e.,* nucleotides 4885-5507 of SEQ ID NO:118), or a nucleotide sequence having about 85%, 86%, 87%, 88%, 89%, 90%, 91%, 92%, 93%, 94%, 95%, 96%, 97%, 98%, or about 99% nucleotide sequence identity to the entire nucleotide sequence of nucleotides 4885-5507 of SEQ ID NO:118.

Additional nucleotide sequences of hBest1 promoters are also described in PCT Publication No. WO 2020/132040.

As used herein, the term "TXNIP" refers to thioredoxin-interacting protein, a member of the alpha arrestin protein family. Thioredoxin is a thiol-oxidoreductase that is a major regulator of cellular redox signaling which protects cells from oxidative stress. TXNIP inhibits the antioxidative function of thioredoxin resulting in the accumulation of reactive oxygen species and cellular stress, and functions as a regulator of cellular metabolism and of endoplasmic reticulum (ER) stress. TXNIP is also known as Upregulated By 1,25-Dihydroxyvitamin D-3; Vitamin D3 Up-Regulated Protein 1; Thioredoxin Binding Protein 2; VDUP1; Thioredoxin-Binding Protein 2; EST01027; HHCPA78; ARRDC6; and THIF.

There are two wild-type transcript variants of human TXNIP and two wild-type transcript variants of mouse TXNIP, the nucleotide and amino acid sequences of which are known and may be found in, for example, GenBank Reference Sequences NM_006472.5 and NP_006463.3; NM_001313972.1 and NP_001300901.1; NM_001009935.2 and NP_001009935.1; and NM_023719.2 and NP_076208.2. The amino acid sequences of the foregoing GenBank entries (NP_ entries) are SEQ ID Nos: 1-4, respectively, and the corresponding nucleotide sequences (NM_ are SEQ ID NOs:101-104, respectively.

As used herein, the term "a C247S variant TXNIP protein" refers to a protein, or a portion *(e.g.,* the N-terminus or the C-terminus) of the protein in which the cysteine at amino acid residue position 247 of SEQ ID NO:1 is replaced with a serine. Based on the amino acid sequence similarities between SEQ ID Nos: 1-4, one of ordinary skill in the art will readily appreciate that, as used herein, a C247S variant of SEQ ID NO:1 is equivalent to a C192S variant of SEQ ID NO:2, or a C248S variant of SEQ ID NO:3 or a C247S variant of SEQ ID NO:4. Exemplary C247S variant TXNIP amino acid sequences are provided in SEQ ID Nos:105-108.

The codon for cysteine is TGT or TGC while the codon for serine is AGT or AGC.

In one embodiment, the nucleic acid molecule encoding the C247S variant TXNIP comprises nucleotides 366-1541 of SEQ ID NO:111; or a nucleotide sequence having about 85%, 86%, 87%, 88%, 89%, 90%, 91%, 92%, 93%, 94%, 95%, 96%, 97%, 98%, or about 99% nucleotide sequence identity to the entire nucleotide sequence of nucleotides 366-1541 of SEQ ID NO:111.

In another embodiment, the nucleic acid molecule encoding the C247S variant TXNIP comprises nucleotides 162-1172 of SEQ ID NO:112, or a nucleotide sequence having about 85%, 86%, 87%, 88%, 89%, 90%, 91%, 92%, 93%, 94%, 95%, 96%, 97%, 98%, or about 99% nucleotide sequence identity to the entire nucleotide sequence of nucleotides 162-1172 of SEQ ID NO:112.

In yet another embodiment, the nucleic acid molecule encoding the C247S variant TXNIP comprises nucleotides 280-1473 of SEQ ID NO:113; or a nucleotide sequence having about 85%, 86%, 87%, 88%, 89%, 90%, 91%, 92%, 93%, 94%, 95%, 96%, 97%, 98%, or about 99% nucleotide sequence identity to the entire nucleotide sequence of nucleotides 280-1473 of SEQ ID NO:113.

In one embodiment, the nucleic acid molecule encoding the C247S variant TXNIP comprises nucleotides 280-1470 of SEQ ID NO:114, or a nucleotide sequence having about 85%, 86%, 87%, 88%, 89%, 90%, 91%, 92%, 93%, 94%, 95%, 96%, 97%, 98%, or about 99% nucleotide sequence identity to the entire nucleotide sequence of nucleotides 280-1470 of SEQ ID NO:114.

In one embodiment, the nucleic acid molecule encoding the C247S variant TXNIP comprises SEQ ID NO: 120; or a nucleotide sequence having about 85%, 86%, 87%, 88%, 89%, 90%, 91%, 92%, 93%, 94%, 95%, 96%, 97%, 98%, or about 99% nucleotide sequence identity to the entire nucleotide sequence of SEQ ID NO:120.

The invention further encompasses nucleic acid molecules that differ, due to degeneracy of the genetic code, from the nucleotide sequence of nucleic acids encoding a C247S variant TXNIP polypeptide, and, thus, encode the same protein.

As used herein, the term "a C247S. LL351.352AA variant TXNIP protein" refers to protein, or a portion (e.g., the N-terminus or the C-terminus) of the protein in which the cysteine at amino acid residue position 247 of SEQ ID NO:1 is replaced with a serine, and the leucines at amino acid residue positions 351 and 352 are replaced by alanine residues.

In one embodiment, the nucleic acid molecule encoding the C247S. LL351.352AA variant TXNIP comprises SEQ ID NO:115, or a nucleotide sequence having about 85%, 86%, 87%, 88%, 89%, 90%, 91%, 92%, 93%, 94%, 95%, 96%, 97%, 98%, or about 99% nucleotide sequence identity to the entire nucleotide sequence of SEQ ID NO: 115.

In one embodiment, the nucleic acid molecule encoding the C247S. LL351.352AA variant TXNIP comprises SEQ ID NO: 121; or a nucleotide sequence having about 85%, 86%, 87%, 88%, 89%, 90%, 91%, 92%, 93%, 94%, 95%, 96%, 97%, 98%, or about 99% nucleotide sequence identity to the entire nucleotide sequence of SEQ ID NO:121.

The invention further encompasses nucleic acid molecules that differ, due to degeneracy of the genetic code, from the nucleotide sequence of nucleic acids encoding a C247S. LL351.352AA variant TXNIP polypeptide, and, thus, encode the same protein.

As used herein, the term "HIF1α" refers to hypoxia-inducible factor 1 subunit alpha, a heterodimeric basic helix-loop-helix transcription factor. HIF1α is also known as HIF-1-alpha; HIF-1A; HIF1; HIF1-ALPHA; MOP1; PASD8; and bHKHe78. The encoded transcription factor regulates hypoxia-inducible genes including the human erythropoietin (EPO) gene. There are three human transcript variants of HIF1α, the nucleotide and amino acid sequences of which are known and maybe found in, for example, GenBank Reference Squences NM_001530.4, NM_181054.3 and NM_001243084.1. There are three mouse transcript variants of HIF1α, the nucleotide and amino acid sequences of which are known and maybe found in, for example, GenBank Reference Squences NM_001313919.1, NM_010431.2 and NM_001313920.1. Examplary nucleotide sequence encoding mouse HIF1α may be found in SEQ ID NO: 116.

As used herein, the term "dominant-negative HIF1α" or "dominant-negative variant of hypoxia inducible factor 1 subunit alpha" refers to a dominant-negative mutant of HIF1α that lacks both the basic DNA binding domain and carboxyl-terminal transactivation domain. Dominant-negative HIF1α is also known as HIF1αΔNBAAB. The expression of dominant-negative HIF1α competes with wild-type HIF1α in dimerization with HIF1β, leading to, *e.g.,* loss of DNA binding activity and and blocking transactiviation of reporter genes containing *EPO* enhancer in hypoxic cells. In one instance, a nucleic acid molecule encoding dominant-negative variant of H1F1α comprises SEQ ID NO: 117, or a nucleotide sequence having about 85%, 86%, 87%, 88%, 89%, 90%, 91%, 92%, 93%, 94%, 95%, 96%, 97%, 98%, or about 99% nucleotide sequence identity to the entire nucleotide sequence of SEQ ID NO: 117

As used herein, the term "Nrf2" or "NRF2" refers to nuclear factor (erythroid-derived 2)-like 2 (nrf2), a transcription factor which is a member of a small family of basic leucine zipper (bZIP) proteins. Nrf2 is also known as Nuclear Factor, Erythroid 2 Like 2; NF-E2-Related Factor 2; HEBP1, Nuclear Factor Erythroid 2-Related Factor 2; Nuclear Factor, Erythroid Derived 2, Like 2; Nuclear Factor (Erythroid-Derived 2)-Like 2; Nuclear Factor Erythroid-Derived 2-Like 2; Nuclear Factor, Erythroid 2-Like 2; NFE2-Related Factor 2; and IMDDHH. The encoded transcription factor regulates genes which contain antioxidant response elements (ARE) in their promoters. There are eight transcript variants of Nrf2, the nucleotide and amino acid sequences of which are known and may be found in, for example, GenBank Reference Sequences NM_006164.4; NM_001145412.3; NM_001145413.3; NM_001313900.1; NM_001313901.1; NM_001313902.1; NM_001313903.1; and NM_001313904.1. In one embodiment, a nucleic acid molecule encoding Nrf2 comprises the nucleotide sequence selected from the group consisting of the Nrf2 transcript variant 1, the Nrf2 transcript variant 2, the Nrf2 transcript variant 3, the Nrf2 transcript variant 4, the Nrf2 transcript variant 5, the Nrf2 transcript variant 6, the Nrf2 transcript variant 7, and the Nrf2 transcript variant 8, or a nucleotide sequence having about 85%, 86%, 87%, 88%, 89%, 90%, 91%, 92%, 93%, 94%, 95%, 96%, 97%, 98%, or about 99% nucleotide sequence identity to the entire nucleotide sequence of any one of the Nrf2 transcript variant 1, the Nrf2 transcript variant 2, the Nrf2 transcript variant 3, the Nrf2 transcript variant 4, the Nrf2 transcript variant 5, the Nrf2 transcript variant 6, the Nrf2 transcript variant 7, or the Nrf2 transcript variant 8. Exemplary nucleotide sequences encoding human Nrf2 are also described in PCT Publication No. WO 2020/132040.

The invention further encompasses nucleic acid molecules that differ, due to degeneracy of the genetic code, from the nucleotide sequence of nucleic acids encoding a Nrf2 polypeptide, and thus encode the same protein.

As used herein, the term "Tgfb1" or "TGFB1" refers to transforming growth factor beta 1, a member of the transforming growth factor beta superfamily of cytokines. It is a secreted protein that performs many cellular functions, including the control of cell growth, cell proliferation, cell differentiation and apoptosis. The nucleotide and amino acid sequences of human Tgfb1 may be found in, for example, GenBank Reference Sequences NM_000660.7. In one embodiment, a nucleic acid molecule encoding Tgfb1 comprises the nucleotide sequence of human Tgfb1, or a nucleotide sequence having about 85%, 86%, 87%, 88%, 89%, 90%, 91%, 92%, 93%, 94%, 95%, 96%, 97%, 98%, or about 99% nucleotide sequence identity to the entire nucleotide sequence of human Tgfb1.

The invention further encompasses nucleic acid molecules that differ, due to degeneracy of the genetic code, from the nucleotide sequence of nucleic acids encoding a TGFB1 polypeptide, and thus encode the same protein.

To determine the percent identity of two amino acid sequences or of two nucleic acids, the sequences are aligned for optimal comparison purposes (e.g., gaps can be introduced in the sequence of a first amino acid or nucleic acid sequence for optimal alignment with a second amino or nucleic acid sequence). The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position. The percent identity between the two sequences is a function of the number of identical positions shared by the sequences *(i.e.,* % identity = # of identical positions total # of positions (e.g., overlapping positions) x100).

The determination of percent identity between two sequences may be accomplished using a mathematical algorithm. A non-limiting example of a mathematical algorithm utilized for the comparison of two sequences is the algorithm of Karlin and Altschul (1990) Proc. Natl. Acad. Sol. USA 87:2264-2268, modified as in Karlin and Altschul (1993) Proc. Nati. Accid Sci. USA 90:5873-5877. Such an algorithm is incorporated into the BLASTN and BLASTX programs of Altschul, *et al.*

(1990) J. Mol. Biol. 215:403-410. BLAST nucleotide searches can be performed with the BLASTN program, score = 100, wordlength = 12 to obtain nucleotide sequences homologous to a nucleic acid molecules of the invention. BLAST protein searches can be performed with the BLASTP program, score - 50, wordlength = 3 to obtain amino acid sequences homologous to a protein molecules of the invention. To obtain gapped alignments for comparison purposes, a newer version of the BLAST algorithm called Gapped BLAST can be utilized as described in Altschul et al. (1997) Nucleic Acids Res 25:3389-3402, which is able to perform gapped local alignments for the programs BLASTN, BLASTP and BLASTX.

In some embodiments, the expression cassettes of the invention further comprise an intron between the promoter and the nucleic acid molecule encoding the C247S variant TXNIP and/or between the promoter and the nucleic acid molecule encoding Nrf2.

In some embodiments, the expression cassettes of the invention further comprise an intron between the promoter and the nucleic acid molecule encoding the C247S variant TXNIP and/or between the promoter and the nucleic acid molecule encoding Tgfb1.

In some embodiments, the expression cassettes of the invention further comprise an intron between the promoter and the nucleic acid molecule encoding the C247S.LL.351.352AA variant TXNIP and/or between the promoter and the nucleic acid molecule encoding Nrf2.

In some embodiments, the expression cassettes of the invention further comprise an intron between the promoter and the nucleic acid molecule encoding the C247S.LL.351.352AA and/or between the promoter and the nucleic acid molecule encoding Tgfb1.

As used herein, "an intron" refers to a non-coding nucleic acid molecule which is removed by RNA splicing during maturation of a final RNA product.

In one embodiment, the intron is an SV40 intron, e.g., the intron comprises the nucleotide sequence of SEQ ID NO:20, or a nucleotide sequence having about 85%, 86%, 87%, 88%, 89%, 90%, 91%, 92%, 93%, 94%, 95%, 96%, 97%, 98%, or about 99% nucleotide sequence identity to the entire nucleotide sequence of the nucleotide sequence of SEQ ID NO: 20.

In yet another embodiment, the intron is a human beta-globin intron, e.g., the intron comprises the nucleotide sequence of SEQ ID NO: 12, or a nucleotide sequence having about 85%, 86%, 87%, 88%, 89%, 90%, 91%, 92%, 93%, 94%, 95%, 96%, 97%, 98%, or about 99% nucleotide sequence identity to the entire nucleotide sequence of the nucleotide sequence of SEQ ID NO: 12.

In another embodiment, the intron is a chimeric intron.

A "chimeric intron" is an artificial (or non-naturally occurring intron that enhances mRNA processing and increases expression levels of a downstream open reading frame.

In some embodiments of the invention, for example, when the expression cassette comprises a PR- specific promoter operably linked to a nucleic acid molecule encoding a C247S variant TXNIP and a nucleic acid molecule encoding Nrf2, i.e., variant TXNIP and Nrf2 are co-expressed by the PR-specific promoter, the expression cassette further comprises a linker between the nucleic acid molecule encoding TXNIP and the nucleic acid molecule encoding Nrf2. Suitable linkers for co-expression of genes from a single promoter are known in the art.

In one embodiment, a suitable linker comprises a nucleotide sequence encoding a 2A peptide. As used herein, a "2A peptide" refers to the art-known peptides also referred to as "self-cleaving 2A peptides" first discovered in picornaviruses. 2A peptides are short (about 20 amino acids) and produce equimolar levels of mulitple genes from the same mRNA. Exemplary nucleotide sequnces of suitable 2A peptides are provided in SEQ ID NOs:21-24.

In some embodiments, the expression cassettes of the invention further comprise a post-transcriptional regulatory region.

The term "post-transcriptional regulatory region", as used herein, refers to any polynucleotide that facilitates the expression, stabilization, or localization of the sequences contained in the cassette or the resulting gene product.

In one embodiment, a post-transcriptional regulatory region suitable for use in the expression cassettes of the invention includes a Woodchuck hepatitis virus post-transcriptional regulatory element.

As used herein, the term "Woodchuck hepatitis virus posttranscriptional regulatory element" or "WPRE," refers to a DNA sequence that, when transcribed, creates a tertiary structure capable of enhancing the expression of a gene. See Lee Y, et al, Exp. Physiol. 2005; 90(l):33-37 and Donello J, et al, J. Virol. 1998; 72(6):5085-5092.

In one embodiment, a WPRE includes the nucleotide sequence of SEQ ID NO: 10 (See, *e.g.,* J Virol. 1998 Jun; 72(6): 5085-5092), or a nucleotide sequence having about 85%, 86%, 87%, 88%, 89%, 90%, 91%, 92%, 93%, 94%, 95%, 96%, 97%, 98%, or about 99% nucleotide sequence identity to the entire nucleotide sequence of the nucleotide sequence of SEQ ID NO: 10.

In another embodiment, a WPRE includes the nucleotide sequence of SEQ ID NO: 11, or a nucleotide sequence having about 85%, 86%, 87%, 88%, 89%, 90%, 91%, 92%, 93%, 94%, 95%, 96%, 97%, 98%, or about 99% nucleotide sequence identity to the entire nucleotide sequence of the nucleotide sequence of SEQ ID NO: 11.

In another embodiment, a WPRE includes nucleotides 1868-2025 of SEQ ID NO: 39, or a nucleotide sequence having about 85%, 86%, 87%, 88%, 89%, 90%, 91%, 92%, 93%, 94%, 95%, 96%, 97%, 98%, or about 99% nucleotide sequence identity to the entire nucleotide sequence of nucleotides 1868-2025 of SEQ ID NO: 39.

In another embodiment, a WPRE includes nucleotides 3529-4070 of SEQ ID NO: 49, or a nucleotide sequence having about 85%, 86%, 87%, 88%, 89%, 90%, 91%, 92%, 93%, 94%, 95%, 96%, 97%, 98%, or about 99% nucleotide sequence identity to the entire nucleotide sequence of nucleotides 3529-4070 of SEQ ID NO: 49.

In some embodiments, the expression cassettes of the invention further comprises a polyadenylation signal.

As used herein, a "polyadenylation signal" or "polyA signal," as used herein refers to a nucleotide sequence that terminates transcription. Suitable polyadenylation signals for use in the AAV vectors of the invention are known in the art and include, for example, a bovine growth hormone polyA signal (BGH pA) or an SV40 polyadenylation signal (SV40 polyA).

In one embodiment, a SV40 pA includes the nucleotide sequence of SEQ ID NO: 13, or a nucleotide sequence having about 85%, 86%, 87%, 88%, 89%, 90%, 91%, 92%, 93%, 94%, 95%, 96%, 97%, 98%, or about 99% nucleotide sequence identity to the entire nucleotide sequence of the nucleotide sequence of SEQ ID NO: 13.

In one embodiment, a BGH pA includes the nucleotide sequence of SEQ ID NO: 25, or a nucleotide sequence having about 85%, 86%, 87%, 88%, 89%, 90%, 91%, 92%, 93%, 94%, 95%, 96%, 97%, 98%, or about 99% nucleotide sequence identity to the entire nucleotide sequence of the nucleotide sequence of SEQ ID NO: 25.

In one embodiment, a BGH pA includes the nucleotide sequence of nucleotides 4270-4484 of SEQ ID NO:26, or a nucleotide sequence having about 85%, 86%, 87%, 88%, 89%, 90%, 91%, 92%, 93%, 94%, 95%, 96%, 97%, 98%, or about 99% nucleotide sequence identity to the entire nucleotide sequence of nucleotides 4270-4484 of SEQ ID NO:26.

In one embodiment, a SV40 pA includes the nucleotide sequence of nucleotides 2026-2228 of SEQ ID NO: 39, or a nucleotide sequence having about 85%, 86%, 87%, 88%, 89%, 90%, 91%, 92%, 93%, 94%, 95%, 96%, 97%, 98%, or about 99% nucleotide sequence identity to the entire nucleotide sequence of nucleotides 2026-2228 of SEQ ID NO: 39.

In one embodiment, a BGH pA includes the nucleotide sequence of nucleotides 4077-4291 of SEQ ID NO: 49, or a nucleotide sequence having about 85%, 86%, 87%, 88%, 89%, 90%, 91%, 92%, 93%, 94%, 95%, 96%, 97%, 98%, or about 99% nucleotide sequence identity to the entire nucleotide sequence of nucleotides 4077-4291 of SEQ ID NO: 49.

In some embodiments, the expression cassettes of the invention further comprise an enhancer.

The term "enhancer", as used herein, refers to a DNA sequence element to which transcription factors bind to increase gene transcription.

The AAV vectors of the invention may also include cis- acting 5' and 3' inverted terminal repeat (ITR) sequences. In some embodiments, the ITR sequences are about 145 bp in length. In some embodiments, substantially the entire sequences encoding the ITRs are used in the molecule. In other embodiments, the ITRs include modifications. Procedures for modifying these ITR sequences are known in the art. See Brown T, "Gene Cloning" (Chapman & Hall, London, GB, 1995), Watson R, et al, "Recombinant DNA", 2nd Ed. (Scientific American Books, New York, NY, US, 1992), Alberts B, et al, "Molecular Biology of the Cell" (Garland Publishing Inc., New York, NY, US, 2008), Innis M, et al, Eds., "PCR Protocols. A Guide to Methods and Applications" (Academic Press Inc., San Diego, CA, US, 1990), Erlich H, Ed., "PCR Technology. Principles and Applications for DNA Amplification" (Stockton Press, New York, NY, US, 1989), Sambrook J, et al, "Molecular Cloning. A Laboratory Manual" (Cold Spring Harbor Laboratory Press, Cold Spring Harbor, NY, US, 1989), Bishop T, et al, "Nucleic Acid and Protein Sequence. A Practical Approach" (IRL Press, Oxford, GB, 1987), Reznikoff W, Ed., "Maximizing Gene Expression" (Butterworths Publishers, Stoneham, MA, US, 1987), Davis L, et al, "Basic Methods in Molecular Biology" (Elsevier Science Publishing Co., New York, NY, US, 1986), and Schleef M, Ed., "Plasmid for Therapy and Vaccination" (Wiley- VCH Verlag GmbH, Weinheim, DE, 2001).

The AAV vectors of the invention may include ITR nucleotide sequences derived from any one of the AAV serotypes. In a preferred embodiment, the AAV vector comprises 5' and 3' AAV ITRs. In one embodiment, the 5' and 3' AAV ITRs derive from AAV2. AAV ITRs for use in the AAV vectors of the invention need not have a wild- type nucleotide sequence (See Kotin, Hum. Gene Ther., 1994, 5:793-801). As long as ITR sequences function as intended for the rescue, replication and packaging of the AAV virion, the ITRs may be altered by the insertion, deletion or substitution of nucleotides or the ITRs may be derived from any of several AAV serotypes or its mutations.

In one embodiment, a 5' ITR includes nucleotides 248-377 of SEQ ID NO:26; nucleotides 1-141 of SEQ ID NO: 39; or nucleotides 248-377 of SEQ ID NO: 49, or a nucleotide sequence having about 85%, 86%, 87%, 88%, 89%, 90%, 91%, 92%, 93%, 94%, 95%, 96%, 97%, 98%, or about 99% nucleotide sequence identity to the entire nucleotide sequence of nucleotides 248-377 of SEQ ID NO:26; nucleotides 1-141 of SEQ ID NO: 39; or nucleotides 248-377 of SEQ ID NO: 49.

In one embodiment, a 3' ITR includes nucleotides 4571-4201 of SEQ ID NO:26; nucleotides 2301-2441 of SEQ ID NO: 39; or nucleotides 4378-4508 of SEQ ID NO: 49, or a nucleotide sequence having about 85%, 86%, 87%, 88%, 89%, 90%, 91%, 92%, 93%, 94%, 95%, 96%, 97%, 98%, or about 99% nucleotide sequence identity to the entire nucleotide sequence of nucleotides 4571-4201 of SEQ ID NO:26; nucleotides 2301-2441 of SEQ ID NO: 39; or nucleotides 4378-4508 of SEQ ID NO: 49.

In addition, an AAV vector can contain one or more selectable or screenable marker genes for initially isolating, identifying, or tracking host cells that contain DNA encoding the ithe AAV vector (and/or rep, cap and/helper genes), e.g., antibiotic resistance, as described herein.

As indicated above, the AAV vectors of the invention may be packaged into AAV viral particles for use in the methods, e.g., gene therapy methods, of the invention (discussed below) to produce AAV vector particles using methods known in the art.

Such methods generally include packaging the AAV vectors of the invention into infectious AAV viral particles in a host cell that has been transfected with a vector encoding and expressing rep and cap gene products *(i.e.* AAV Rep and Cap proteins), and with a vector which encodes and expresses a protein from the adenovirus open reading frame E4orf6.

Suitable AAV Caps may be derived from any serotype. In one embodiment, the capsid is derived from the AAV of the group consisting on AAV1, AAV2, AAV3, AAV4, AAV5, AAV6, AAV7, AAV8 and AAV9. In another embodiment, the AAV of the invention comprises a capsid derived from the AAV7m8, AAV5 or AAV8 serotypes.

In some embodiments, an AAV Cap for use in the method of the invention can be generated by mutagenesis *(i.e.* by insertions, deletions, or substitutions) of one of the aforementioned AAV Caps or its encoding nucleic acid. In some embodiments, the AAV Cap is at least 70%, 75%, 80%, 85%, 90%, 95%, 98%, or 99% or more similar to one or more of the aforementioned AAV Caps.

In some embodiments, the AAV Cap is chimeric, comprising domains from two, three, four, or more of the aforementioned AAV Caps. In some embodiments, the AAV Cap is a mosaic of VP1, VP2, and VP3 monomers originating from two or three different AAV or a recombinant AAV. In some embodiments, a rAAV composition comprises more than one of the aforementioned Caps.

Suitable rep may be derived from any AAV serotype. In one embodiment, the rep is derived from any of the serotypes selected from the group consisting of AAV1, AAV2, AAV3, AAV4, AAV5, AAV6, AAV7, AAV8, or AAV9. In another embodiment, the AAV rep is derived from the serotype AAV2.

Suitable helper genes may be derived from any AAV serotype and include adenovirus E4, E2a and VA.

The AAV rep, AAV cap and genes providing helper functions can be introduced into the cell by incorporating the genes into a vector such as, for example, a plasmid, and introducing the vector into a cell. The genes can be incorporated into the same plasmid or into different plasmids. In one, the AAV rep and cap genes are incorporated into one plasmid and the genes providing helper functions are incorporated into another plasmid.

The AAV vectors of the invention and the polynucleotides comprising AAV rep and cap genes and genes providing helper functions may be introduced into a host cell using any suitable method well known in the art. See Ausubel F, et al, Eds., "Short Protocols in Molecular Biology", 4th Ed. (John Wiley and Sons, Inc., New York, NY, US, 1997), Brown (1995), Watson (1992), Alberts (2008), Innis (1990), Erlich (1989), Sambrook (1989), Bishop (1987), Reznikoff (1987), Davis (1986), and Schleef (2001), *supra.* Examples of transfection methods include, but are not limited to, co-precipitation with calcium phosphate, DEAE-dextran, polybrene, electroporation, microinjection, liposome-mediated fusion, lipofection, retrovirus infection and biolistic transfection. When the cell lacks the expression of any of the AAV rep and cap genes and genes providing adenoviral helper functions, said genes can be introduced into the cell simultaneously with the AAV vector. Alternatively, the genes can be introduced in the cell before or after the introduction of the AAV vector of the invention.

Methods of culturing packaging cells and exemplary conditions which promote the release of AAV vector particles, such as the producing of a cell lysate, are known in the art. Producer cells are grown for a suitable period of time in order to promote release of viral vectors into the media. Generally, cells may be grown for about 24 hours, about 36 hours, about 48 hours, about 72 hours, about 4 days, about 5 days, about 6 days, about 7 days, about 8 days, about 9 days, up to about 10 days. After about 10 days (or sooner, depending on the culture conditions and the particular producer cell used), the level of production generally decreases significantly. Generally, time of culture is measured from the point of viral production. For example, in the case of AAV, viral production generally begins upon supplying helper virus function in an appropriate producer cell as described herein. Generally, cells are harvested about 48 to about 100, preferably about 48 to about 96, preferably about 72 to about 96, preferably about 68 to about 72 hours after helper virus infection (or after viral production begins).

The AAV vector particles of the invention can be obtained from both: i) the cells transfected with theforegoing and ii) the culture medium of the cells after a period of time post-transfection, preferably 72 hours. Any method for the purification of the AAV vector particles from the cells or the culture medium can be used for obtaining the AAV vector particles of the invention. In a particular embodiment, the AAV vector particles of the invention are purified following an optimized method based on a polyethylene glycol precipitation step and two consecutive cesium chloride (CsCl) or iodixanol density gradient ultracentrifugation. See Ayuso et al., 2014, Zolotukhin S, et al., Gene Ther. 1999; 6; 973-985. Purified AAV vector particles of the invention can be dialyzed against an appropriate formulation buffer such as PBS, filtered and stored at -80°C. Titers of viral genomes can be determined by quantitative PCR following the protocol described for the AAV2 reference standard material using linearized plasmid DNA as standard curve. See Aurnhammer C, et al., Hum Gene Ther Methods, 2012, 23, 18-28, D'Costa S, et al., Mol Ther Methods Clin Dev. 2016, 5, 16019.

In some embodiments, the methods further comprise purification steps, such as treatment of the cell lysate with benzonase, purification of the cell lysate with the use of affinity chromatography and/or ion-exchange chromotography. See Halbert C, et al, Methods Mol. Biol. 2004; 246:201-212, Nass S, et al., Mol Ther Methods Clin Dev. 2018 Jun 15; 9: 33-46.

AAV Rep and Cap proteins and their sequences, as well as methods for isolating or generating, propagating, and purifying such AAV, and in particular, their capsids, suitable for use in producing AAV are known in the art. See Gao, 2004, *supra,* Russell D, et al, US 6,156,303, Hildinger M, et al, US 7,056,502, Gao G, et al, US 7,198,951, Zolotukhin S, US 7,220,577, Gao G, et al, US 7,235,393, Gao G, et al, US 7,282,199, Wilson J, et al, US 7,319,002, Gao G, et al, US 7,790,449, Gao G, et al, US 20030138772, Gao G, et al, US 20080075740, Hildinger M, et al, WO 2001/083692, Wilson J, et al, WO 2003/014367, Gao G, et al, WO 2003/042397, Gao G, et al, WO 2003/052052, Wilson J, et al, WO 2005/033321, Vandenberghe L, et al, WO 2006/110689, Vandenberghe L, et al, WO 2007/127264, and Vandenberghe L, et al, WO 2008/027084.

### III. PHARMACEUTICAL COMPOSITIONS OF THE INVENTION

In one aspect of the invention, an AAV viral particle of the invention will be in the form of a pharmaceutical composition containing a pharmaceutically acceptable carrier. As used herein "pharmaceutically acceptable carrier" refers to any substantially non-toxic carrier conventionally useable for administration of pharmaceuticals in which the isolated polypeptide of the present invention will remain stable and bioavailable. The pharmaceutically acceptable carrier must be of sufficiently high purity and of sufficiently low toxicity to render it suitable for administration to the mammal being treated. It further should maintain the stability and bioavailability of an active agent. The pharmaceutically acceptable carrier can be liquid or solid and is selected, with the planned manner of administration in mind, to provide for the desired bulk, consistency, *etc.,* when combined with an active agent and other components of a given composition. Suitable pharmaceutically acceptable carriers include any and all solvents, dispersion media, coatings, antibacterial and antifungal agents, isotonic and absorption delaying agents, and the like that are physiologically compatible. Pharmaceutically acceptable carriers also include sterile aqueous solutions or dispersions and sterile powders for the extemporaneous preparation of sterile injectable solutions or dispersion. The use of such media and agents for pharmaceutically active substances is well known in the art. Except insofar as any conventional media or agent is incompatible with the gene therapy vector, use thereof in the pharmaceutical compositions of the invention is contemplated. Supplementary active compounds can also be incorporated into the compositions.

Pharmaceutical compositions of the invention may be formulated for delivery to animals for veterinary purposes (e.g. livestock (cattle, pigs, dogs, mice, rats), and other non-human mammalian subjects, as well as to human subjects.

In a particular embodiment, the pharmaceutical compositions of the present invention are in the form of injectable compositions. The compositions can be prepared as an injectable, either as liquid solutions or suspensions. The preparation may also be emulsified. Suitable excipients are, for example, water, saline, dextrose, glycerol, ethanol, phosphate buffered saline or the like and combinations thereof. In addition, if desired, the preparation may contain minor amounts of auxiliary substances such as wetting or emulsifying agents, pH-buffering agents, adjuvants, surfactant or immunopotentiators.

In a particular embodiment, the AAV particles of the invention are incorporated in a composition suitable for intraocular administration. For example, the compositions may be designed for intravitreal, subretinal, subconjuctival, sub-tenon, periocular, retrobulbar, suprachoroidal, and/or intrascleral administration, for example, by injection, to effectively treat the retinal disorder. Additionally, a sutured or refillable dome can be placed over the administration site to prevent or to reduce "wash out", leaching and/or diffusion of the active agent in a non-preferred direction.

Relatively high viscosity compositions, as described herein, may be used to provide effective, and preferably substantially long-lasting delivery of the nucleic acid molecules and/or vectors, for example, by injection to the posterior segment of the eye. A viscosity inducing agent can serve to maintain the nucleic acid molecules and/or vectors in a desirable suspension form, thereby preventing deposition of the composition in the bottom surface of the eye. Such compositions can be prepared as described in U.S. Patent No. 5,292,724.

Sterile injectable solutions can be prepared by incorporating the compositions of the invention in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation include vacuum drying and freeze-drying which yields a powder of the active ingredient plus any additional desired ingredient from a previously sterile-filtered solution thereof.

Toxicity and therapeutic efficacy of nucleic acid molecules described herein can be determined by standard pharmaceutical procedures in cell cultures or experimental animals, *e.g.,* for determining the ED₅₀ (the dose therapeutically effective in 50% of the population). Data obtained from cell culture assays and/or animal studies can be used in formulating a range of dosage for use in humans. The dosage typically will lie within a range of concentrations that include the ED₅₀ with little or no toxicity. The dosage may vary within this range depending upon the dosage form employed and the route of administration utilized. For any compound used in the method of the invention, the therapeutically effective dose can be estimated initially from cell culture assays.

### IV. METHODS OF THE INVENTION

The present invention also provides compositions of the invention for use in methods, which generally include contacting an ocular cell with an AAV viral particle or pharmaceutical composition comprising an AAV particle of the invention.

Accordingly, in one aspect, the present invention provides compositions for use in methods for prolonging the viability of a photoreceptor cell, *e.g.,* a photoreceptor cell, compromised by degenerative ocular disorder, e.g., retinitis pigmentosa, age related macular degeneration, cone rod dystrophy, and rod cone dystrophy. The methods generally include contacting the cell with an AAV viral particle or pharmaceutical composition comprising an AAV particle of the invention.

The present invention further provides compositions for use in methods for treating a degenerative ocular disorder in a subject having a degenerative ocular disorder, e.g., retinitis pigmentosa, age related macular degeneration, cone rod dystrophy, and rod cone dystrophy. The methods include administering to the subject a therapeutically effective amount of an AAV viral particle or pharmaceutical composition comprising an AAV particle of the invention.

The present invention also provides compositions for use in methods for preventing a degenerative ocular disorder in a subject having a degenerative ocular disorder, e.g., retinitis pigmentosa, age related macular degeneration, cone rod dystrophy, and rod cone dystrophy. The methods include administering to the subject a prohylatically effective amount of an AAV viral particle or pharmaceutical composition comprising an AAV particle of the invention.

In another aspect, the present invention provides compositions for use in methods of treating a subject having retinitis pigmentosa. The methods include administering to the subject a therapeutically effective amount of an AAV viral particle or pharmaceutical composition comprising an AAV particle of the invention.

In another aspect, the present invention provides compositions for use in methods of treating a subject having age-related macular degeneration. The methods include administering to the subject a therapeutically effective amount of an AAV viral particle or pharmaceutical composition comprising an AAV particle of the invention.

In one embodiment, the methods comprise administering to the subject a therapeutically effective amount of a composition comprising an adeno-associated virus (AAV) expression cassette, the expression cassette comprising a human bestrophin 1 (hBest1) promoter, a chimeric intron, and a nucleic acid molecule encoding nuclear factor erythroid 2-like 2 (Nrf2), or a pharmaceutical comprising an adeno-associated virus (AAV) expression cassette, the expression cassette comprising a human bestrophin 1 (hBest1) promoter, a chimeric intron, and a nucleic acid molecule encoding nuclear factor erythroid 2-like 2 (Nrf2). In such methods, the AAV comprising the C247S variant TXNIP and the AAV comprsing Nrf 2 may be formulated in the same composition or different compositions and/or may administered to the subject in the same composition or in separate compositions. The AAV comprising the C247S variant TXNIP and the AAV comprsing Nrf2 may be administered to a subject at the same dose or different doses and at the same time or at different times.

Generally, methods are known in the art for viral infection of the cells of interest. The virus can be placed in contact with the cell of interest or alternatively, can be injected into a subject suffering from a disorder associated with photoreceptor cell oxidative stress.

Guidance in the introduction of the compositions of the invention into subjects for therapeutic purposes are known in the art and may be obtained in the above-referenced publications, as well as in U.S. Patent Nos. 5,631,236, 5,688,773, 5,691,177, 5,670,488, 5,529,774, 5,601,818, and PCT Publication No. WO 95/06486.

The compositions of the invention may be delivered to a subject by, for example, intravenous injection, local administration (see, *e.g.,* U.S. Patent No. 5,328,470), stereotactic injection (see, *e.g.,* Chen et al. (1994) Proc. Natl. Acad. Sci. U.S.A. 91:3054-3057), or by *in vivo* electroporation (see, *e.g.,* Matsuda and Cepko (2007) Proc. Natl. Acad. Sci. U.S.A. 104:1027-1032). Preferably, the compositions of the invention are administered to the subject locally. Local administration of the compositions described herein can be by any suitable method in the art including, for example, injection (e.g., intravitreal or subretinal, subvitreal, subconjuctival, sub-tenon, periocular, retrobulbar, suprachoroidal, and/or intrascleral injection), gene gun, by topical application of thecomposition in a gel, oil, or cream, by electroporation, using lipid-based transfection reagents, transcleral delivery, by implantation of scleral plugs or a drug delivery device, or by any other suitable transfection method.

Application of the methods of the invention for the treatment and/or prevention of a disorder can result in curing the disorder, decreasing at least one symptom associated with the disorder, either in the long term or short term or simply a transient beneficial effect to the subject.

Accordingly, as used herein, the terms "treat," "treatment" and "treating" include the application or administration of compositions, as described herein, to a subject who is suffering from a degenerative ocular disease or disorder, or who is susceptible to such conditions with the purpose of curing, healing, alleviating, relieving, altering, remedying, ameliorating, improving or affecting such conditions or at least one symptom of such conditions. As used herein, the condition is also "treated" if recurrence of the condition is reduced, slowed, delayed or prevented.

The term "prophylactic" or "therapeutic" treatment refers to administration to the subject of one or more of the subject compositions. If it is administered prior to clinical manifestation of the unwanted condition *(e.g.,* disease or other unwanted state of the host animal) then the treatment is prophylactic, *i.e.,* it protects the host against developing the unwanted condition, whereas if administered after manifestation of the unwanted condition, the treatment is therapeutic *(i.e.,* it is intended to diminish, ameliorate or maintain the existing unwanted condition or side effects therefrom).

"Therapeutically effective amount," as used herein, is intended to include the amount of a composition of the invention that, when administered to a patient for treating a degenerative ocular disease, is sufficient to effect treatment of the disease (e.g., by diminishing, ameliorating or maintaining the existing disease or one or more symptoms of disease). The "therapeutically effective amount" may vary depending on the composition, how the composition is administered, the disease and its severity and the history, age, weight, family history, genetic makeup, stage of pathological processes mediated by the disease expression, the types of preceding or concomitant treatments, if any, and other individual characteristics of the patient to be treated.

"Prophylactically effective amount," as used herein, is intended to include the amount of a composition that, when administered to a subject who does not yet experience or display symptoms of *e.g.,* a degenerative ocular disorder, but who may be predisposed to the disease, is sufficient to prevent or ameliorate the disease or one or more symptoms of the disease. Ameliorating the disease includes slowing the course of the disease or reducing the severity of later-developing disease. The "prophylactically effective amount" may vary depending on the composition, how the composition is administered, the degree of risk of disease, and the history, age, weight, family history, genetic makeup, the types of preceding or concomitant treatments, if any, and other individual characteristics of the patient to be treated.

A "therapeutically-effective amount" or "prophylacticaly effective amount" also includes an amount of a composition that produces some desired local or systemic effect at a reasonable benefit/risk ratio applicable to any treatment. A composition employed in the methods of the present invention may be administered in a sufficient amount to produce a reasonable benefit/risk ratio applicable to such treatment.

Subjects suitable for treatment using the regimens of the present invention should have or are susceptible to developing a degenerative ocular disease or disorder. For example, subjects may be genetically predisposed to development of the disorders. Alternatively, abnormal progression of the following factors including, but not limited to visual acuity, the rate of death of cone and/ or rod cells, night vision, peripheral vision, attenuation of the retinal vessels, and other ophthalmoscopic factors associated with degenerative ocular disorders such as retinitis pigmentosa may indicate the existence of or a predisposition to a retinal disorder.

In one embodiment, the disorder includes, but not limited to, retinitis pigmentosa, age related macular degeneration, cone rod dystrophy, and rod cone dystrophy. In other embodiments, the disorder is not associated with blood vessel leakage and/or growth. In certain embodiments, the disorder is not associated with diabetes. In another embodiment, the disorder is not diabetic retinopathy. In further embodiments, the disorder is not NARP (neuropathy, ataxia and retinitis pigmentosa). In one embodiment, the disorder is a disorder associated with decreased viability of cone and/or rod cells. In yet another embodiment, the disorder is a genetic disorder.

The compositions, as described herein, may be administered as necessary to achieve the desired effect and depend on a variety of factors including, but not limited to, the severity of the condition, age and history of the subject and the nature of the composition, for example, the identity of the genes or the affected biochemical pathway.

The pharmaceutical compositions of the invention may be administered in a single dose or, in particular embodiments of the invention, multiples doses *(e.g.* two, three, four, or more administrations) may be employed to achieve a therapeutic effect.

The therapeutic or preventative regimens may cover a period of at least about 2, 3, 4, 5, 6, 7, 8, 9, 10, 11, 12, 13, 14, 15, 16, 17, 18, 19, 20, 21, 22, 23 or 24 weeks, or be chronically administered to the subject.

In one embodiment, the viability or survival of photoreceptor cells, such as cones cells, is, e.g., about 1 week, about 2 weeks, about 3 weeks, about 4 weeks, about 5 weeks, about 6 weeks, about 7 weeks, about 8 weeks, about 3 years, about 4 years, about 5 years, about 10 years, about 15, years, about 20 years, about 25 years, about 30 years, about 40 years, about 50 years, about 60 years, about 70 years, and about 80 years.

In general, the nucleic acid molecules and/or the vectors of the invention are provided in a therapeutically effective amount to elicit the desired effect, e.g., increase C247S variant TXNIP expression. The quantity of the viral particle to be administered, both according to number of treatments and amount, will also depend on factors such as the clinical status, age, previous treatments, the general health and/or age of the subject, other diseases present, and the severity of the disorder. Precise amounts of active ingredient required to be administered depend on the judgment of the gene therapist and will be particular to each individual patient. Moreover, treatment of a subject with a therapeutically effective amount of the nucleic acid molecules and/or the vectors of the invention can include a single treatment or, preferably, can include a series of treatments. It will also be appreciated that the effective dosage used for treatment may increase or decrease over the course of a particular treatment. Changes in dosage may result from the results of diagnostic assays as described herein. The pharmaceutical compositions can be included in a container, pack, or dispenser together with instructions for administration.

In some embodiments, a therapeutically effective amount or a prophylactically effective amount of a viral particle of the invention (or pharmaceutical composition of the invention) is in titers ranging from about 1x10⁵ , about 1.5x10⁵, about 2x10⁵, about 2.5x10⁵, about 3x10⁵, about 3.5x10^{5,} about 4x10°, about 4.5x10⁵, about 5x10⁵, about 5.5x10⁵, about 6x10°, about 6.5x10⁵, about 7x10⁵, about 7.5x10⁵, about 8x10⁵, about 8.5x10⁵, about 9x10⁵, about 9.5x10⁵, about 1x10⁶, about 1.5x10⁶, about 2x10⁶, about 2.5x10⁶, about 3x10⁶, about 3.5x10⁶, about 4x10⁶, about 4.5x10⁶, about 5x10°, about 5.5x10⁶, about 6x10⁶, about 6.5x10⁶, about 7x10⁶, about 7.5x10⁶, about 8x10⁶, about 8.5x10, about 9x10⁶, about 9.5x10⁶, about 1x10⁷, about 1.5x10⁷, about 2x10⁷, about 2.5x10⁷, about 3x10⁷, about 3.5x10⁷, about 4x10⁷, about 4.5x10⁷, about 5x10⁷, about 5.5x10⁷, about 6x10⁷, about 6.5x10⁷, about 7x10⁷, about 7.5x10⁷, about 8x10⁷, about 8.5x10⁷, about 9x10⁷, about 9.5x10⁷, about 1x10⁸, about 1.5x10⁸, about 2x10⁸, about 2.5x10⁸, about 3x10⁸, about 3.5x10⁸, about 4x10⁸, about 4.5x10⁸, about 5x10⁸, about 5.5x10⁸, about 6x10⁸, about 6.5x10⁸, about 7x10⁸, about 7.5x10⁸, about 8x10⁸, about 8.5x10⁸, about 9x10⁸, about 9.5x10⁸, about 1x10⁹, about 1.5x10⁹, about 2x10⁹, about 2.5x109⁸, about 3x10⁹, about 3.5x10⁹, about 4x10⁹, about 4.5x10⁹, about 5x10⁹, about 5.5x10⁹, about 6x10⁹, about 6.5x10⁹, about 7x10⁹, about 7.5x10⁹, about 8x10⁹, about 8.5x10⁹, about 9x10⁹, about 9.5x10⁹, about 1x10'°, about 1.5x10¹⁰, about 2x10¹⁰, about 2.5x10¹⁰, about 3x10¹⁰, about 3.5x10¹⁰, about 4x10¹⁰, about 4.5x10¹⁰, about 5x10¹⁰, about 5.5x10¹⁰, about 6x10¹⁰, about 6.5x10¹⁰, about 7x10¹⁰, about 7.5x10¹⁰, about 8x10¹⁰, about 8.5x10¹⁰, about 9x10¹⁰, about 9.5x10¹⁰, about 1x10¹¹, about 1.5x10¹¹, about 2x10¹¹, about 2.5x10¹¹, about 3x10¹¹, about 3.5x10¹¹, about 4x10¹¹, about 4.5x10¹¹, about 5x10¹¹, about 5.5x10¹¹, about 6x10¹¹, about 6.5x10¹¹, about 7x10¹¹, about 7.5x10¹¹, about 8x10¹¹, about 8.5x10¹¹, about 9x10¹¹, about 9.5x10¹¹, about 1x10¹² viral particles (vp).

In some embodiments, a therapeutically effective amount or a prophylactically effective amount of a viral particle of the invention (or pharmaceutical composition of the invention) is in genome copies ("GC"), also referred to as "viral genomes" ("vg") ranging from about 1x10⁵ , about 1.5x10⁵, about 2x10⁵, about 2.5x10⁵, about 3x10⁵, about 3.5x10⁵, about 4x10⁵, about 4.5x10⁵, about 5x10⁵, about 5.5x10⁵, about 6x10⁵, about 6.5x10⁵, about 7x10⁵, about 7.5x10⁵, about 8x10⁵, about 8.5x10⁵, about 9x10⁵, about 9.5x10⁵, about 1x10⁶, about 1.5x10⁶, about 2x10⁶, about 2.5x10⁶, about 3x10⁶, about 3.5x10⁶, about 4x10⁶, about 4.5x10⁶, about 5x10⁶, about 5.5x10⁶, about 6x10⁶, about 6.5x10⁶, about 7x10⁶, about 7.5x10⁶, about 8x10⁶, about 8.5x10, about 9x10⁶, about 9.5x10⁶, about 1x10⁷, about 1.5x10⁷, about 2x10⁷, about 2.5x10⁷, about 3x10⁷, about 3.5x10⁷, about 4x10⁷, about 4.5x10⁷, about 5x10⁷, about 5.5x10⁷, about 6x10⁷, about 6.5x10⁷, about 7x10⁷, about 7.5x10⁷, about 8x10⁷, about 8.5x10⁷, about 9x10⁷, about 9.5x10⁷, about 1x10⁸, about 1.5x10⁸, about 2x10⁸, about 2.5x10⁸, about 3x10⁸, about 3.5x10⁸, about 4x10⁸, about 4.5x10⁸, about 5x10⁸, about 5.5x10⁸, about 6x10⁸, about 6.5x10⁸, about 7x10⁸, about 7.5x10⁸, about 8x10⁸, about 8.5x10⁸, about 9x10⁸, about 9.5x10⁸, about 1x10⁹, about 1.5x10⁹, about 2x10⁹, about 2.5x109⁸, about 3x10⁹, about 3.5x10⁹, about 4x10⁹, about 4.5x10⁹, about 5x10⁹, about 5.5x10⁹, about 6x10⁹, about 6.5x10⁹, about 7x10⁹, about 7.5x10⁹, about 8x10⁹, about 8.5x10⁹, about 9x10⁹, about 9.5x10⁹, about 1x10¹⁰, about 1.5x10¹⁰, about 2x10¹⁰, about 2.5x10¹⁰, about 3x10¹⁰, about 3.5x10¹⁰, about 4x10¹⁰, about 4.5x10¹⁰, about 5x10¹⁰, about 5.5x10¹⁰, about 6x10¹⁰, about 6.5x10¹⁰, about 7x10¹⁰, about 7.5x10¹⁰, about 8x10¹⁰, about 8.5x10¹⁰, about 9x10¹⁰, about 9.5x10¹⁰, about 1x10¹¹, about 1.5x10¹¹, about 2x10¹¹, about 2.5x10¹¹, about 3x10¹¹, about 3.5x10¹¹, about 4x10¹¹, about 4.5x10¹¹, about 5x10¹¹, about 5.5x10¹¹, about 6x10¹¹, about 6.5x10¹¹, about 7x10¹¹, about 7.5x10¹¹, about 8x10¹¹, about 8.5x10¹¹, about 9x10¹¹, about 9.5x10¹¹, about 1x10¹² vg.

Any method known in the art can be used to determine the genome copy (GC) number of the viral compositions of the invention. One method for performing AAV GC number titration is as follows: purified AAV viral particle samples are first treated with DNase to eliminate un-encapsidated AAV genome DNA or contaminating plasmid DNA from the production process. The DNase resistant particles are then subjected to heat treatment to release the genome from the capsid. The released genomes are then quantitated by real-time PCR using primer/probe sets targeting specific region of the viral genome.

In various embodiments, the methods of the present invention further comprise monitoring the effectiveness of treatment. For example, visual acuity, the rate of death of cone and/ or rod cells, night vision, peripheral vision, attenuation of the retinal vessels, and other ophthalmoscopic changes associated with retinal disorders such as retinitis pigmentosa may be monitored to assess the effectiveness of treatment. Additionally, the rate of death of cells associated with the particular disorder that is the subject of treatment and/or prevention, may be monitored. Alternatively, the viability of such cells may be monitored, for example, as measured by phospholipid production. The assays described in the Examples section below may also be used to monitor the effectiveness of treatment (e.g., electroretinography - ERG).

In certain embodiments of the invention, a composition of the invention is administered in combination with an additional therapeutic agent or treatment. The compositions and an additional therapeutic agent can be administered in combination in the same composition or the additional therapeutic agent can be administered as part of a separate composition or by another method described herein.

Examples of additional therapeutic agents suitable for use in the methods of the invention include those agents known to treat retinal disorders, such as retinitis pigmentosa and age-related macular degeneration and include, for example, fat soluble vitamins (e.g., vitamin A, vitamin E, and ascorbic acid), calcium channel blockers *(e.g.,* diltiazem) carbonic anhydrase inhibitors *(e.g.,* acetazolamide and methazolamide), anti-angiogenics (*e.g.*,antiVEGF antibodies), growth factors (e.g., rod-derived cone viability factor (RdCVF), BDNF, CNTF, bFGF, and PEDF), antioxidants, other gene therapy agents *(e.g.,* optogenetic gene threrapy, *e.g.,* channelrhodopsin, melanopsin, and halorhodopsin), and compounds that drive photoreceptor regeneration by, e.g., reprogramming Müller cells into photoreceptor progenitors (e.g., alpha-aminoadipate). Exemplary treatments for use in combination with the treatment methods of the present invention include, for example, retinal and/or retinal pigmented epithelium transplantation, stem cell therapies, retinal prostheses, laser photocoagulation, photodynamic therapy, low vision aid implantation, submacular surgery, and retinal translocation.

This invention is further illustrated by the following examples which should not be construed as limiting.

### EXAMPLES

The following Materials and Methods were used in the Examples below.

### Animals

*rd1* mice were the albino FVB strain, which carries the *Pde6b^{rd1}* allele (MGI: 1856373). BALB/c, CD1, and FVB mice were purchased from Charles River Laboratories and from Taconic. C57BL/6J, *rd10,* and *parp*^{*-*/*-*} mice were purchased from The Jackson Laboratories and bred in house. *parp*^{*-*/*-*} mice were crossed with FVB mice to generate homozygous *parpl*^{*-*/}*⁻ rd1* and *parp1*^{*+*/}*⁺ rd1* mice. Genotyping of these mice was done by Transnetyx (Cordova, TN). The *rho*^{*-*/*-*} mice were provided from by Lem (Tufts University, MA) (Lem *et al.,* 1999).

### AAV vector design, authentication, and preparation

Detailed information of all AAVs used in this study is listed in **Table 1,** along with the authentication information. cDNAs of mouse *txnip, hif1a, hk2, ldha, ldhb, slc2a1, bsg1, cpt1a, oxct1, mpcl* and *mpc2,* and human *nrf2,* were purchased from GeneCopeia (Rockville, MD). Mouse *vegf164* cDNA (Robinson and Stringer, 2001) was synthesized by Integrated DNA Technologies (Coralville, Iowa). The following plasmids were gifts from various depositors through Addgene (Watertown, MA): *hk1, pfkm* and *pkm2* (William Hahn & David Root; #23730, #23728, #23757), *pkm1* (Lewis Cantley & Matthew Vander Heiden; #44241), H2B-GFP (Geoff Wahl; #11680), mitoRFP *(i.e.* DsRed2-mito, Michael Davidson; #55838), GFP-Txnip (Clark Distelhorst; #18758), W3SL (Bong-Kiun Kaang; #61463), 3xFLAG (Thorsten Mascher, #55180), PercevalHR and pHRed (Gary Yellen; #490820, #31473). The cDNA of mouse RdCVF was a gift from Leah Byrne and John Flannery (UC Berkeley, CA). iGlucoSnFR was provided under a Material Transfer Agreement by Jacob Keller and Loren Looger (Janelia Research Campus, VA). RedO promoter was provided as a gift, and SynPVI and SynP136 promoters were provided under a Material Transfer Agreement, from Botond Roska (IOB, Switzerland). The Best1 promoter was synthesized by lab member, Wenjun Xiong, using Integrated DNA Technologies based on literature (Esumi *et al.,* 2009). Mutated Txnip, dominant-negative HIF1α (Jiang *et al.,* 1996) and RO1.7 promoter (Ye *et al.,* 2016) were created from the corresponding wildtype plasmids in house using Gibson assembly.

**Table 1: AAV vectors**

| **Inserts** | **Insert species** | **Promoter** | **Intron** | **WPRE** | **poly(A)** | **ITR-to-ITR size** | **Digestion (XmaI, ITR)** | **Partial seq (ligation site)** | **Complete plasmid seq** | **AAV genome seq** |
|---|---|---|---|---|---|---|---|---|---|---|
| **H2BGFP** | N/A | RedO (default) | N/A | WPRE | Bovine GH | 4.4 kb | Correct | Correct | - | - |
| **H2BGFP** | N/A | SynP136 | N/A | WPRE3 | SV40-Late | 3.9 kb | Correct | Correct | - | - |
| **mitoRFP** | N/A | SynP136 | N/A | WPRE3 | SV40-Late | 3.5 kb | Correct | Correct | Correct | - |
| **Txnip** | Mouse | RedO (default) | N/A | WPRE | Bovine GH | 4.5kb | Correct | Correct | Correct | - |
| **Txnip** | Mouse | SynPVI | N/A | WPRE3 | SV40-Late | 2.4kb | Correct | Correct | - | - |
| **Txnip** | Mouse | Best1 | SV40 | WPRE | Bovine GH | 3.1kb | Correct | Correct | - | - |
| **Txnip.C247S** | Mouse | RedO | N/A | WPRE | Bovine GH | 4.5kb | Correct | Correct | Correct | Correct |
| **Txnip.S308A** | Mouse | RedO | N/A | WPRE | Bovine GH | 4.5kb | Correct | Correct | Correct | Correct |
| **Txnip.C247S.S308A** | Mouse | RedO | N/A | WPRE | Bovine GH | 4.5kb | Correct | Correct | Correct | - |
| **Txnip.C247S.LL351&352AA** | Mouse | RedO | N/A | WPRE | Bovine GH | 4.5kb | Correct | Correct | Correct | - |
| **Txnip.C247S.LL351&352AA** | Mouse | Best1 | SV40 | WPRE | Bovine GH | 3.1kb | Correct | Correct | Correct | - |
| **GFP-Txnip** | Mouse | RO1.7 | N/A | WPRE3 | SV40-Late | 4.4 kb | Correct | Correct | Correct | - |
| **Nrf2** | Human | CMV | human β-globin | N/A | SV40 | 3.7 kb | - | - | - | - |
| **Nrf2** | Human | SvnPVI | N/A | WPRE3 | SV40-Late | 3.1kb | Correct | Correct | Correct | - |
| **Nrf2** | Human | Best1 | SV40 | WPRE | Bovine GH | 3.8 kb | Correct | Correct | - | - |
| **Cx3cl1** | Mouse | Best1 | SV40 | WPRE | Bovine GH | 3.0 kb | - | Correct | Correct | - |
| **Cx3cl1** | Mouse | RedO | N/A | WPRE | Bovine GH | 4.3 kb | - | Correct | - | - |
| **Tgfb1** | Mouse | RedO | N/A | WPRE | Bovine GH | 4.4 kb | - | Correct | - | - |
| **dnHIF1α** | Mouse | RO1.7 | N/A | WPRE3 | SV40-Late | 3.6 kb | Correct | Correct | Correct | - |
| **dnHIF1α** | Mouse | Best1 | SV40 | WPRE | Bovine GH | 3.1kb | Correct | Correct | Correct | - |
| **Hif1a** | Mouse | SynPVI | N/A | WPRE3 | SV40-Late | 3.8 kb | Correct | Correct | Correct | - |
| **Hk1** | Human | SvnPVI | N/A | WPRE3 | SV40-Late | 4.0 kb | Correct | Correct | - | - |
| **Hk2** | Mouse | SvnPVI | N/A | WPRE3 | SV40-Late | 4.0 kb | Correct | Correct | - | - |
| **Pfkm** | Human | SvnPVI | N/A | WPRE3 | SV40-Late | 3.6 kb | Correct | Correct | - | - |
| **Pkm1** | Human | SynPVI | N/A | WPRE3 | SV40-Late | 2.8 kb | Correct | Correct | - | - |
| **Pkm2** | Human | SynPVI | N/A | WPRE3 | SV40-Late | 2.8 kb | Correct | Correct | - | - |
| **Ldha** | Mouse | RO1.7 | N/A | WPRE3 | SV40-Late | 3.6 kb | Correct | Correct | Correct | - |
| **Ldhb** | Mouse | RedO | N/A | WPRE | Bovine GH | 4.3 kb | Correct | Correct | Correct | - |
| **Ldhb-3xFLAG** | Mouse | RO1.7 | N/A | WPRE3 | SV40-Late | 3.6 kb | Correct | Correct | Correct | - |
| **Slc2a1** | Mouse | RO1.7 | N/A | WPRE3 | SV40-Late | 4.0 kb | Correct | Correct | Correct | - |
| **Bsg1** | Mouse | RedO | N/A | WPRE | Bovine GH | 4.4 kb | Correct | Correct | - | - |
| **RdCVF** | Mouse | RedO | N/A | WPRE | Bovine GH | 3.6 kb | Correct | Correct | - | - |
| **RdCVF** | Mouse | Best1 | SV40 | WPRE | Bovine GH | 2.3 kb | Correct | Correct | - | - |
| **Cpt1a** | Mouse | RedO | N/A | WPRE | Bovine GH | 5.6 kb | Correct | Correct | Correct | - |
| **Oxct1** | Mouse | RedO | N/A | WPRE | Bovine GH | 4.8 kb | Correct | Correct | Correct | - |
| **Mpc1** | Mouse | RO1.7 | N/A | WPRE3 | SV40-Late | 3.0 kb | Correct | Correct | - | - |
| **Mpc2** | Mouse | RO1.7 | N/A | WPRE3 | SV40-Late | 2.9kb | Correct | Correct | - | - |
| **Vegf164** | Mouse | RO1.7 | N/A | WPRE3 | SV40-Late | 3.1kb | Correct | Correct | Correct | - |
| | | | | | | | | | | |
| **PercevalHR** | N/A | RO1.7 | N/A | WPRE3 | SV40-Late | 4.2 kb | Correct | Correct | Correct | - |
| **iGlucoSnFR** | N/A | SynPVI | N/A | WPRE3 | SV40-Late | 4.0 kb | Correct | Correct | Correct | - |
| **pHRed** | N/A | SynP136 | N/A | WPRE3 | SV40-Late | 3.5 kb | Correct | Correct | Correct | - |
| | | | | | | | | | | |
| **siNC** | N/A | RedO | N/A | WPRE | Bovine GH | 3.3 kb | Correct | Correct | Correct | - |
| **siLdhb^{(#2)} (default)** | Mouse | RedO | N/A | WPRE | Bovine GH | 3.3 kb | Correct | Correct | Correct | - |
| **siLdhb** ^{(#1)} | Mouse | RedO | N/A | WPRE | Bovine GH | 3.3 kb | Correct | Correct | Correct | - |
| **siLdhb^{(#3)}** | Mouse | RedO | N/A | WPRE | Bovine GH | 3.3 kb | Correct | Correct | Correct | - |
| **siOxct1** | Mouse | RedO | N/A | WPRE | Bovine GH | 3.3 kb | Correct | Correct | Correct | - |
| **siCpt1a** | Mouse | RedO | N/A | WPRE | Bovine GH | 3.3 kb | Correct | Correct | Correct | - |
| **siSlc2a1** | Mouse | RedO | N/A | WPRE | Bovine GH | 3.3 kb | Correct | Correct | Correct | - |

All of the new constructs in this study were cloned using Gibson assembly. For example, AAV-RedO-Txnip was cloned by replacing the EGFP sequence of AAV-RedO-EGFP at the NotI/HindIII sites, with the Txnip sequence, which was PCR-amplified from the cDNA vector adding two 20-bp overlapping sequences at the 5'- and 3'-ends. All of the AAV plasmids were amplified using Stbl3 *E. coli* (Thermo Fisher Scientific). The sequences of all AAV plasmids were verified with directed sequencing and restriction enzyme digestion. The key plasmids were triple-verified with Next-Generation complete plasmid sequencing (MGH CCIB DNA Core), which is able to capture the full sequence of the ITR regions. The genome sequence of critical AAVs *(i.e.* AAV8-RedO-Txnip.C247S and AAV8-RedO-Txnip.S308A) were quadruple-verified with PCR and directed sequencing.

All of the vectors were packaged in recombinant AAV8 capsids using HEK293T cells and purified with iodixanol gradient as previously described (Grieger *et al.,* 2006; Xiong *et al.,* 2015). The titer of each AAV batch was determined using protein gels, comparing viral band intensities with a previously established AAV standard. The concentration of our AAV production usually ranged from 2 x 10¹² to 3 x 10¹³ gc/mL. Multiple batches of key AAV vectors *(e.g.* 4 batches of AAV8-RedO-Txnip, and 3 batches of AAV-RedO-siLdhb^{(#2)}) were made and tested *in vivo* to avoid any unknown batch effects.

### shRNA

The shRNA plasmids of Ldhb, Slc2a1, Oxct1 and Cpt1a were purchased from GeneCopeia, and they were provided as three or four distinct sequences for each gene, driven by the H1 or U6 promoter. The knockdown efficiency of these candidate shRNA sequences was tested by co-transfecting with CAG-TargetGene-IRES-d2GFP vector in HEK293T cells as previously described (Matsuda and Cepko, 2007; Wang *et al.,* 2014). The GFP fluorescence intensity served as a fast and direct read out of the knockdown efficiency of these shRNAs. Using this method, we selected the following sense strand sequences to knockdown the targeted genes **(****FIG. 16-11****):** siLdhb^{(#2)} 5'-CCATCATCGTGGTTTCCAACC-3' (SEQ ID NO: 125); siLdhb^{(#1)} 5'-GCAGAGAAATGTCAACGTGTT-3' (SEQ ID NO: 126); siLdhb^{(#3)} 5'-GCCGATAAAGATTACTCTGTG-3' (SEQ ID NO: 127); siS1c2a1^{(#a)} 5'-GGTTATTGAGGAGTTCTACAA-3' (SEQ ID NO: 128); siOxct1^{(#c)} 5'-GGAAACAGTTACTGTTCTCCC-3' (SEQ ID NO: 129); siCpt1a^{(#c)} 5'-GCATAAACGCAGAGCATTCCT-3' (SEQ ID NO: 130); and siNC (non-targeting scrambled control sequence) 5'-GCTTCGCGCCGTAGTCTTA-3' (SEQ ID NO: 131). The entire hairpin sequence (including a 6-bp 5'-end lead sequence 5'-gatccg-3', a 7-bp loop sequence 5'-TCAAGAG-3' was cloned between sense and antisense strands, and a > 7-bp 3'end sequence 5'-ttttttg-3') and packaged them into AAV8-RedO-shRNA using Gibson assembly as described above. To maximize the knockdown efficacy using a Pol II promoter in AAV (Giering *et al.,* 2008), no extra base pair was kept between the RedO promoter and the 5'-end lead sequence of shRNAs. The *in vivo* Ldhb knockdown efficiency of all three AAV8-RedO-siLdhb vectors was confirmed by co-injection with an AAV8-Ldhb-3xFLAG vector into wildtype mouse eyes and detection for FLAG immunofluorescence as described in the Histology section below **(****FIG. 11a****).**

### Subretinal injection

On the day of birth (P0), ≈1 x 10⁹ vg/eye of AAV was injected into the eyes of pups as previously described(Matsuda and Cepko, 2007; Xiong *et al.,* 2015). For all experiments in which cones were quantified, and to provide a means to trace infection *(e.g.* for immunohistochemistry), 2.5 x 10⁸ vg/eye of AAV8-RedO-H2BGFP was co-injected with other AAVs, or alone as a control. For all other experiments, such as FACS sorting and *ex vivo* live-imaging, 1 x 10⁹ vg/eye of AAV8-SynP136-H2BGFP was co-injected.

### Photopic visual acuity measured for optomotor response

The photopic optomotor response of mice were measured using the OptoMotry System (CerebralMechanics) at a background light of ≈70 cd/m² as previously described (Xiong *et al.,* 2019). The contrast of the grates was set to be 100%, and temporal frequency was 1.5 Hz. The threshold of mouse visual acuity *(i.e.* maximal spatial frequency) was tested by an examiner without knowledge of the injected or the treatment group. During each test, the direction of movement of the grates *(i.e.* clockwise or counterclockwise) was randomized, and the spatial frequency of each testing episode was determined by the software. Without knowing the spatial frequency of the moving grates, the examiner reported either "yes" or "no" to the system until the threshold of acuity was determined by the software.

### Histology

Mice were euthanized with CO₂ and cervical dislocation, and the eye was enucleated. For flat-mounts, retinas were separated from the rest of the eye using a dissecting microscope and were fixed in 4% paraformaldehyde solution for 30 minutes. The retinas were then flat mounted on a glass slide and coverslip. For H2BGFP labeled cone imaging, we used a Keyence microscope with a 10x objective (Plan Apo Lamda 10x/0.45 Air DIC N1) and GFP filter box (OP66836).

For cone opsin antibody staining in whole-mount retinas, after fixation, retinas were blocked for 1 hour in PBS with 5% normal donkey serum and 0.3% Triton X-100 at room temperature. After blocking, retinas were incubated with a mixture of 1:200 anti-s-opsin antibody (AB5407, EMD Millipore) and 1:600 anti-m-opsin antibody (AB5405, EMD Millipore) in the same blocking solution overnight at 4 °C, followed by secondary donkey-anti-rabbit antibody staining (1:1000, Alexa Fluor 594) at room temperature for 2 hours, then flat-mounted on a glass slide and coverslip.

For frozen sections, whole eyes were fixed in 4% paraformaldehyde solution for 2 hours at room temperature, followed by removing the cornea, lens and iris. Then the eye cups went through 15% and 30% sucrose gradient to dehydrate at room temperature, followed by overnight incubation in 1:1 30% sucrose and Tissue-Tek^{®} O.C.T. solution at 4 °C. Eye cups were embedded in a plastic mold, frozen in a -80 °C freezer, and cut into 20 µm or 12 µm thin radial cross-sections which were placed on glass slides. Antibody staining was done similarly to whole-mounts as described above and previously (Wang *et al.,* 2014). PBS with 0.1% Triton X-100, 5% normal donkey serum and 1% bovine serum albumin (BSA) was used as the blocking solution, except for FLAG detection (10% donkey serum and 3% BSA). Glut1 (encoded by *slc2a1* gene) antibody (GT11-A, Alpha Diagnostics) was used at 1:300 dilution, Parp1 antibody (ab227244, Abcam) was used at 1:300 dilution, GFP antibody (ab13970, Abcam) was used at 1:1000 dilution to detect GFP-Txnip, and FLAG antibody (ab1257, Abcam) was used at 1:2000 based on a previous study(Ferrando *et al.,* 2015). If applicable, 1:1000 PNA (CY5 or Rhodamine labeled) for cone extracellular matrix labeling, and 1:1000 DAPI were used to co-stain with secondary antibodies. Stained sections were imaged with a confocal microscope (LSM710, Zeiss) using 20x or 63x objectives (Plan Apo 20x/0.8 Air DIC II, or Plan Apo 63X/1.4 Oil DIC III).

### Automated RP cone counting

The cone-H2BGFP images of whole flat-mounted retinas were first analyzed in ImageJ to acquire the diameter and the center parameters of the sample. We used a custom MATLAB script to automatically count the number of H2BGFP-positive cones in the central half of the retina, since RP cones degenerate faster in the central than the peripheral retina. The algorithm was based on a Gaussian model to identify the centers of labeled cells, and published recently (Wu *et al.,* n.d.). The threshold of peak intensity and the variance of distribution were initially determined using visual inspection, and a comparison to the number of manually counted cones from 6 retinas. The threshold of intensity and variance thus determined were then set at fixed values for all the experiments that used cone quantification. The background intensity did not interfere with the accurate counting on the raw images by this MATLAB script, despite the representative images at low-magnification might look differently.

### Live-imaging of cones on ex vivo retinal explants

For JC-1 mitochondrial dye staining, the retina was quickly dissected in a solution of 50% Ham's F-12 Nutrient Mix (11765054, Thermo Fisher Scientific) and 50% Dulbecco's Modified Eagle Medium (DMEM; 11995065, Thermo Fisher Scientific) at room temperature. They were then incubated in a culture solution containing 50% Fluorobrite DMEM (A1896701, Thermo Fisher Scientific), 25% heat inactivated horse serum (26050088, Thermo Fisher Scientific) and 25% Hanks' Balanced Salt Solution (HBSS; 14065056, Thermo Fisher Scientific) with 2 µM JC-1 dye (M34152, Thermo Fisher Scientific) at 37 °C in a 5% CO₂ incubator for 20 minutes. The retinas were washed in 37 °C culture medium without JC-1 for three times, transferred in a glass-bottom culture dish (MatTek P50G-1.5-30-F) with culture medium, and imaged using a confocal microscope (LSM710 Zeiss), which was equipped with a chamber pre-heated to 37 °C with pre-filled 5% CO₂. Right before imaging, a cover slip (VWR 89015-725) was gently applied to flatten the retina. Regions of interest (with H2BGFP as an indicator of successful AAV infection and to set the correct focal plane on the cone layer) were selected under the eyepiece with a 63x objective (Plan Apo 63X/1.4 Oil DIC III). Fluorescent images from the same region of interest were obtained with the excitation-wavelength in the order of 561 nm (for J-aggregates), 514 nm (for JC-1 monomer), and 488 nm (for H2BGFP). Four different regions of interest from the central part of the same retina were imaged before moving to the next retina.

For RH421 (Na⁺/K⁺ ATPase dye) staining, similar steps were taken as for JC-1 staining, with the following modifications: 1) 0.83 µM RH421 dye (61017, Biotium) was added to the glass-bottom culture dishes just before imaging, but not during incubation in the incubator, due to the fast action of RH421. 2) 5 regions of interest were imaged per retina from the central area. 3) The dissection and culture medium were lactate-only medium (see below). 4) Excitation wavelengths: 561 nm (RH421), and 488 nm (H2BGFP).

For imaging genetically-encoded metabolic sensors (PercevalHR, iGlucoSnFR and pHRed), retinas were placed in the incubator for 12 minutes and then taken to confocal imaging without any staining. For the high-glucose condition, the culture medium described above contains ≈15 mM glucose without lactate or pyruvate. For the lactate-only condition, the culture and dissection media were both glucose-pyruvate-free DMEM (A144300, Thermo Fisher Scientific) and were supplemented with 20mM sodium L-lactate (71718, Sigma-Aldrich). For the pyruvate-only condition, the culture and dissection media, were both glucose-pyruvate-free DMEM plus 10 or 20 mM sodium pyruvate (P2256, Sigma-Aldrich). No AAV-H2BGFP was co-injected with these sensors, since the sensors themselves could be used to trace the area of infection. The excitation wavelengths for sensors were 488 nm and 405 nm (PercevalHR, ratiometric high and low ATP:ADP), 488 nm and 561 nm (iGlucoSnFR, glucose-sensing GFP and normalization mRuby), and 561 nm and 458 nm (pHRed, ratiometric low and high pH).

The fluorescent intensity of all acquired images was measured by ImageJ. The ratio of sensors/dye was normalized to averaged control results taken at the same condition.

### Flow cytometry and cell sorting

All flow cytometry and cell sorting were performed on MoFlo Astrios EQ equipment. Retinas were freshly dissected and dissociated using cysteine-activated papain followed by gentle pipetting (Shekhar *et al.,* 2016). Before sorting, all samples were passed through a 35-µm filter with buffer containing Fluorobrite DMEM (A1896701, Thermo Fisher Scientific) and 0.4% BSA. Cones labeled with AAV8-SynP136-H2BGFP (highly cone-specific) were sorted into the appropriate buffer for either ddPCR or RNA-sequencing.

### RNA sequencing

RNA sequencing was done as previously described (Wang *et al.,* 2019). 1,000 H2BGFP-positive cones per retina were sorted into 10 µL of Buffer TCL (Qiagen) containing 1% β-mercaptoethanol and immediately frozen in -80 °C. On the day of sample submission, the frozen cone lysates were thawed on ice and loaded into a 96-well plate for cDNA library synthesis and sequencing. A modified Smart-Seq2 protocol was performed on samples by the Broad Institute Genomics Platform with ~6 million reads per sample (Picelli *et al.,* 2013). The reads were mapped to the GRCm38.p6 reference genome after quality control measures. Reads assigned to each gene were quantified using featureCounts (Liao *et al.,* 2014). Count data were analyzed using DESeq2 to identify differentially expressed genes, with an adjusted p value less than 0.05 considered significant(Anders and Huber, 2010). The raw results have been deposited to Gene Expression Omnibus (accession number GSE161622).

### ddPCR

RNA was isolated from 20,000 sorted cones per retina using RNeasy Micro Kit (Qiagen) as previously described (Wang *et al.,* 2020), and converted to cDNA using the SuperScript III First-Strand Synthesis System (Invitrogen). cDNA from each sample was packaged in droplets for Droplet Digital^{™} PCR (ddPCR) using QX200 EvaGreen Supermix (#1864034). The reads of expression were normalized to the housekeeping gene *Hprt.* Sequences for RT-PCR primers were designed using the IDT online RealTime qPCR primer design tool. The following primers were selected for the genes of interest: *Txnip* (forward 5'-ACATTATCTCAGGGACTTGCG-3' (SEQ ID NO: 132); reverse 5'-AAGGATGACTTTCTTGGAGCC-3' (SEQ ID NO: 133)), *Hprt* (forward 5'-TCAGTCAACGGGGGACATAAA-3' (SEQ ID NO: 134); reverse 5'-GGGGCTGTACTGCTTAACCAG-3' (SEQ ID NO: 135)), *mt-Nd4* (forward 5'-AGCTCAATCTGCTTACGCCA-3' (SEQ ID NO: 136); reverse 5'-TGTGAGGCCATGTGCGATTA-3' (SEQ ID NO: 137)), *mt-Cytb* (forward 5'-ATTCTACGCTCAATCCCCAAT-3' (SEQ ID NO: 138); reverse 5'-TATGAGATGGAGGCTAGTTGGC-3' (SEQ ID NO: 139)), *mt-Co1* (forward 5'-TCTGTTCTGATTCTTTGGGCACC-3' (SEQ ID NO: 140); reverse 5'-CTACTGTGAATATGTGGTGGGCT-3' (SEQ ID NO: 141)), *Acsl3* (forward 5'-AACCACGTATCTTCAACACCATC-3' (SEQ ID NO: 142); reverse 5'-AGTCCGGTTTGGAACTGACAG-3' (SEQ ID NO: 143)), and *Ftl1* (forward 5'-CCATCTGACCAACCTCCGC-3' (SEQ ID NO: 144); reverse 5'- CGCTCAAAGAGATACTCGCC-3'(SEQ ID NO: 145))).

### Electron microscopy

Intracardial perfusion (4% PFA+1% glutaraldehyde) was performed on ketamine/xylazine (100/10 mg/kg) anesthetized mice before the removal of eyes. The cornea was sliced open and the eye was fixed with a fixative buffer (1.25% formaldehyde+ 2.5 % glutaraldehyde + 0.03% picric acid in 0.1 M sodium cacodylate buffer, pH 7.4) overnight at 4 °C. The cornea, lens and retina were removed before resin embedding, ultrathin sectioning and negative staining at Harvard Medical School Electron Microscopy Core. The detailed methods can be found on the core's website (https://electron-microscopy.hms.harvard.edu/methods). The stained thin sections were imaged on a conventional transmission electron microscope (JEOL 1200EX) with an AMT 2k CCD camera.

### Statistics

For the comparison of two sample groups, two-tailed unpaired Student's t test was used to test for the significance of difference, except for P140 *rho*^{*-*/*-*} optomotor assay (paired two-tail t-test). For comparison of more than two sample groups, ANOVA and Dunnett's multiple comparison test was performed in Prism 8 software to determine the significance. A p value of less than 0.05 was considered statistically significant. All error bars are presented as mean ± standard deviation, except for the *rd10* optomotor assays (mean ± SEM).

### Example 1: Txnip prolongs RP cone survival and visual acuity

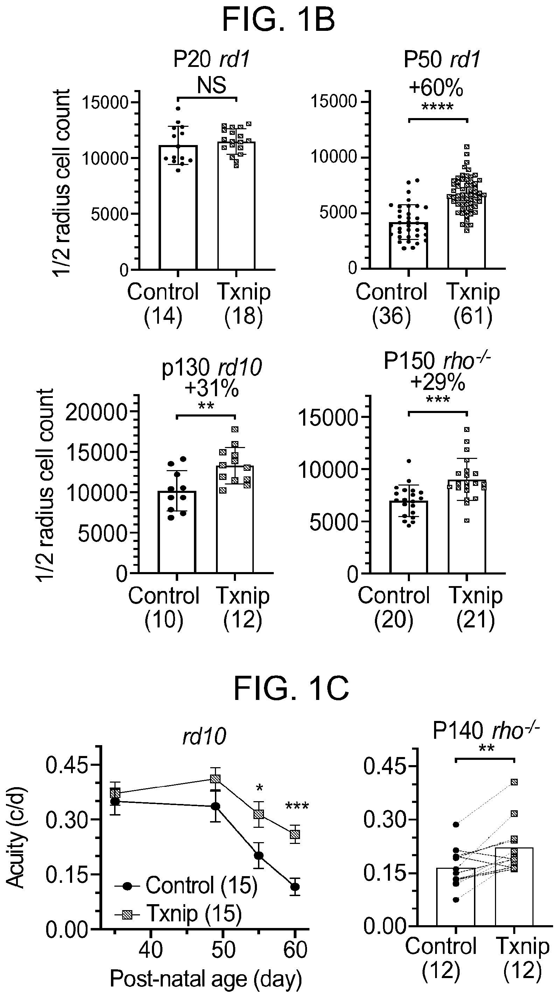

Twelve AAV vectors were constructed (FIG. 9E and Table 1) and tested singly or in combinations. Most of these vectors carried genes to augment the utilization of glucose, such as hexokinases (Hk1 and Hk2), phosphofructokinase (Pfkm) and pyruvate kinase (Pkm1 and Pkm2). Each AAV vector used a cone-specific promoter, which was previously found to be non-toxic at the doses used in this study. An initial screen was carried out in *rd1* mice, which harbor a null allele in the rod-specific gene, *Pde6b.* This strain has a rapid loss of rods, followed by cone death. The vectors were subretinally injected into the eyes of neonatal *rd1* mice, in combination with a vector using the human red opsin (RedO) promoter to express a histone 2B-GFP fusion protein (AAV-RedO-H2BGFP). The H2BGFP provides a very bright cone-specific nuclear labelling, enabling automated quantification. As a control, eyes were injected with AAV-RedO-H2BGFP alone. *Rd1* cones begin to die at ≈postnatal day 20 (P20), when almost all rods have died (FIG. 9A). The number of *rd1* cones was quantified by counting the H2BGFP+ cells using a custom-made MATLAB program (FIG. 1A and FIG. 9C). Only cones within the central region of the retina were counted, since RP cones in the periphery die much later (Hartong *et al.,* 2006; Punzo *et al.,* 2009). Among the twelve tested vectors, and six of their combinations, it was found that only Txnip led to an increase in P50 *rd1* cones. The effects were likely on cone survival, as it did not change the number of cones at P20 prior to their death (FIGS. 1A, 1B and FIGS. 9C, 9E). The level of Txnip rescue in P50 *rd1* cones was comparable to using AAV with a CMV promoter to express a transcription factor, Nrf2, that regulates antioxidation pathways and reduces inflammation as we found previously (Xiong *et al.,* 2015) (FIG. 9E). One combination led to a reduction in cone survival, that of Hk1 plus Pfkm (FIG. 9E).

To evaluate a different cone-specific promoter, Txnip also was tested using a newly described cone-specific promoter, SynPVI, which is a guanine nucleotide-binding protein G subunit alpha-2 (GNAT2) promoter. This promoter also led to prolonged cone survival **(****FIG. 9E****).** To explore whether Txnip gene therapy is effective beyond *rd1,* it was tested in *rd10* mice, which carry a missense *Pde6b* mutation, and in *rho*^{*-*/*-*} mice, which carry a null allele in a rod-specific protein, rhodopsin. Prolonged cone survival was observed in both strains **(****FIGS. 1A****, 1B).** To determine if Txnip-treated mice sustained greater visual acuity than control RP mice, an optomotor assay was used (Prusky *et al.,* 2004). Under conditions that simulated daylight, Txnip treated eyes showed enhanced visual acuity compared to the control contralateral eyes in *rd10* and *rho*^{*-*/*-*} mice **(****FIG. 1C****).** Txnip also was evaluated for effects on cones in wildtype (wt) mice, using PNA staining, which stains the cone-specific extracellular matrix and reflects cone health. The approximate number and morphology of Txnip-treated cones appeared normal by this assay **(****FIG. 9D****).**

### Example 2: Evaluation of txnip alleles for cone survival.

Previous studies of Txnip provided a number of alleles that could potentially lead to a more effective cone rescue by Txnip, and/or provide some insight into which of the Txnip functions are required for enhancing cone survival. A C247S mutation has been shown to block Txnip's inhibitory interaction with thioredoxin (Patwari *et al.,* 2009), which is an important component of a cell's ability to fight oxidative damage via thiol groups (Junn *et al.,* 2000; Nishinaka *et al.,* 2001; Nishiyama *et al.,* 1999). If cone rescue by Txnip required this function, the C247S allele should be less potent for cone rescue. Alternatively, if loss of thioredoxin binding freed Txnip for its other functions, and made more thioredoxin available for oxidative damage control, this allele might more effectively promote cone survival. The C247S clearly provided more robust cone rescue than wildtype (wt) Txnip in all three RP mouse strains **(****FIGS. 2A, 2B** and **FIGS. 10A****, 10B).** These results indicate that the therapeutic effect of Txnip is not based on the inhibitory interaction with thioredoxins. This finding is in keeping with previous work which showed that anti-oxidation strategies promoted cone survival in RP mice (Komeima *et al.,* 2006; Wu *et al.,* n.d.; Xiong *et al.,* 2015). An additional mutant, S308A, which loses an AMPK/Akt-phosphorylation site on Txnip (Waldhart *et al.,* 2017; Wu *et al.,* 2013), was tested in the context of wt Txnip and in the context of the C247S allele. The S308A allele did not benefit cone survival in either context **(****FIGS. 2A, 2B).** In addition, the S308A allele was assayed for negative effects on cones by an assessment of *rd1* cone number prior to P20, *i.e.* before the onset of cone death **(****FIG. 10C****).** It did not reduce the cone number at this early timepoint, indicating that Txnip.S308A was not toxic to cones. This finding suggests that the S308 residue is critical for the therapeutic function of Txnip, through an unclear mechanism. One additional allele, LL351&352AA, was tested in the context of C247S. This allele eliminates a clathrin-binding site, and thus hampers Txnip's ability to remove Glut1 from cell surface through clathrin-coated pits (Wu *et al.,* 2013). Txnip.C247S.LL351&352AA could still delay RP cone death compared to the control **(****FIG. 2B****),** suggesting that the therapeutic effect of Txnip was unlikely to be only through the removal of Glut1 from the cell surface. To further explore the role of Glut1, an shRNA to *slc2a1,* which encodes Glut1, was tested. It did not prolong RP cone survival **(****FIG. 10D**). The slight decrease of Txnip.C247S.LL351&352AA in cone rescue compared to Txnip.C247S might be due to other, currently unknown effects of LL351&352, or a less specific effect, *e.g.* a protein conformational change.

### Example 3: Txnip requires lactate dehydrogenase b (Ldhb) to prolong cone survival.

Humans carrying a Txnip null mutant present with lactic acidosis (Katsu-Jiménez *et al.,* 2019), suggesting Txnip deficiency compromises lactate catabolism. A recent metabolomic study of muscle using a targeted knock-out of Txnip suggested that Txnip increases the catabolism of non-glucose fuels, such as lactate, ketone bodies and lipids (DeBalsi *et al.,* 2014). This switch in fuel preference was proposed to benefit the mitochondrial tricarboxylic acid cycle (TCA) cycle, leading to a greater production of ATP. As presented earlier, a problem for cones in the RP environment might be a shortage of glucose (Aït-Ali *et al.,* 2015; Punzo *et al.,* 2009; Wang *et al.,* 2016). A benefit of Txnip might then be to enable and/or force cells to switch from a preference for glucose to one or more alternative fuels. To test this hypothesis, AAV-Txnip with shRNAs targeting the rate-limiting genes for the catalysis of lactate, ketones or lipids were co-injected. Ldhb, encoded by *ldhb* gene, is the enzyme that converts lactate to pyruvate to potentially fuel the TCA cycle, and lactate dehydrogenase a (Ldha, encoded by *ldha* gene), converts pyruvate to lactate (Eventoff *et al.,* 1977). It was found that Txnip rescue was significantly decreased by any one of three Ldhb shRNAs (siLdhb) or by overexpression of Ldha **(****FIGS. 3A, 3B** and **FIGS. 11A-11E****).** The rescue effect of Txnip plus an shRNA against Oxct1 (siOxct1), a critical enzyme for ketolysis (Zhang and Xie, 2017), or against Cpt1a (siCptla), a component for lipid transporter that is rate limiting for β-oxidation (Shriver and Manchester, 2011) was also tested. These shRNAs, tested singly or in combination, did not reduce the effectiveness of Txnip rescue **(****FIG. 3C****).** Taken together, these data support the use of lactate, but not ketones or lipids, as a critical alternative fuel for cones when Txnip is overexpressed.

### Example 4: Txnip improves the ATP:ADP ratio in RP cones in the presence of lactate.

If the improved survival of cones following Txnip overexpression is due to improved utilization of non-glucose fuels, cones might show improved mitochondrial metabolism. To begin to examine the metabolism of cones, metabolomics of cones with and without Txnip were performed. However, so few cones are present in these retinas that meaningful results could not be achieved. An alternative assay was conducted to measure the ratio of ATP to ADP using a genetically-encoded fluorescent sensor (GEFS). AAV was used to deliver PercevalHR, an ATP:ADP GEFS (Tantama *et al.,* 2013), to *rd1* cones with and without AAV-Txnip. The infected P20 *rd1* retinas were explanted and imaged in three different types of media to measure the cone cytosolic ratio of ATP:ADP. Txnip increased the ATP:ADP ratio (*i.e.* higher F_{PercevalHR}^{488:405}) of *rd1* cones in lactate-only or pyruvate-only media. Consistent with the role of Txnip in removing Glut1 from the plasma membrane, Txnip treated cones had a lower ATP:ADP ratio (*i.e.* lower F_{percevaIHR}^{488:405}) in high glucose medium **(****FIGS. 4A****, 4B).** To further probe whether intracellular glucose was reduced after overexpression of Txnip (Wu *et al.,* 2013), a glucose sensor iGlucoSnFR was used (Keller *et al.,* 2019). This sensor showed reduced intracellular glucose in Txnip-treated cones **(****FIGS. 12A****, 12B).** Because the fluorescence of GEFS may be also subject to environmental pH, we used a pH sensor, pHRed (Tantama *et al.,* 2011), to determine if the changes of PercevalHR and iGlucoseSnFR were due to a change in pH, and it showed no significant pH change **(****FIGS. 12C, 12D**). It was also found that lactate, but not pyruvate, utilization by Txnip-treated cones was critically dependent upon Ldhb for ATP production, as introduction of siLdhb abrogated the increase in ATP:ADP in Txnip-treated cones **(****FIG. 12C****).** Furthermore, in correlation with improved cone survival by Txnip.C247S compared to wt Txnip **(****FIG. 2B****),** cones had a higher ATP:ADP ratio in lactate medium when Txnip.C247S was used relative to wt Txnip **(****FIG. 4E****).** Similarly, in correlation with no survival benefit when treated with Txnip.S308A **(****FIG. 2B****),** there was no difference in the ATP:ADP ratio when Txnip.S308A was used, relative to control, in lactate medium **(****FIG. 4E****).**

### Example 5: Txnip improved RP cone mitochondrial gene expression, size, and function.

To further probe the mechanism(s) of Txnip rescue, it was determined if all of the benefits of Txnip were due to Txnip's effects on Ldhb. Ldhb was thus overexpressed alone or with Txnip. Ldhb alone did not prolong cone survival, nor did it increase the Txnip rescue **(****FIG. 14E****).** An additional experiment was carried out to investigate if there might be a shortage of the mitochondrial pyruvate carrier, which could limit the uptake of pyruvate into the mitochondria of photoreceptors for ATP synthesis (Grenell *et al.,* 2019). The pyruvate carrier, which is a dimer encoded by *mpc1* and *mpc2* genes, thus was overexpressed, but did not prolong *rd1* cone survival **(****FIG. 14C****).** To take a less biased approach, the transcriptomic differences between Txnip-treated and control RP cones were characterized. H2BGFP labeled RP cones were isolated by FACS-sorting at an age when cones were beginning to die, and RNA-sequencing was performed **(****FIG. 13A**). Data were obtained from two RP strains, *rd1* and *rho*^{*-*/}*⁻.* By comparing the differentially expressed genes in common between the two strains, relative to control, seven genes were seen to be upregulated and 17 were downregulated **(Table 2).** Three of the seven upregulated genes were mitochondrial electron transport chain (ETC) genes. The upregulation of these three ETC genes in Txnip-treated *rd1* cones was confirmed by ddPCR **(****FIG. 13B****).**

**Table 2: RNA-sequencing genes commonly regulated in rd1 and Rho^{-/-} by Txnip**

| | **P90 *rho*^{*-*/*-*}** | | | | **P21 *rd1*** | | | |
|---|---|---|---|---|---|---|---|---|
| **MGI symbol** | **Base Mean** | **log2Fold Change** | **log2Fold SE** | **Adjusted p-value** | **Base Mean** | **log2Fold Change** | **log2Fold SE** | **Adjusted p-value** |
| **Txnip** | 1324.3 | 10.211 | 0.516 | 4.78E-83 | 582.4 | 9.325 | 0.775 | 2.17E-29 |
| **mt-Cytb** | 13643.7 | 1.248 | 0.257 | 0.00079349 | 5651.4 | 0.384 | 0.082 | 0.00014272 |
| **mt-Nd4** | 3118.0 | 1.195 | 0.212 | 3.52E-05 | 1504.6 | 0.392 | 0.076 | 1.76E-05 |
| **Vax2os** | 67.2 | 1.028 | 0.323 | 0.04091883 | 33.6 | 0.932 | 0.321 | 0.04383248 |
| **mt-Co1** | 9876.4 | 0.808 | 0.189 | 0.00393386 | 5235.1 | 0.388 | 0.075 | 1.7625E-05 |
| **Rom1** | 5040.6 | 0.748 | 0.190 | 0.00789643 | 4432.7 | 0.164 | 0.056 | 0.04435279 |
| **Cd63** | 488.9 | 0.717 | 0.233 | 0.04957176 | 249.8 | 0.370 | 0.110 | 0.01303008 |
| **Ftl1** | 799.9 | 0.657 | 0.212 | 0.04701416 | 934.6 | 0.252 | 0.081 | 0.02671713 |
| **Utp14b** | 51.1 | -2.486 | 0.586 | 0.00406139 | 60.1 | -1.679 | 0.246 | 2.9705E-09 |
| **Slc9a7** | 52.9 | -1.993 | 0.494 | 0.00646284 | 45.3 | -1.047 | 0.278 | 0.00408081 |
| **Megf9** | 369.0 | -1.752 | 0.529 | 0.03204053 | 417.7 | -0.666 | 0.173 | 0.00313404 |
| **Mgat2** | 33.4 | -1.572 | 0.434 | 0.01699743 | 36.7 | -1.343 | 0.343 | 0.00251377 |
| **Rnf168** | 40.0 | -1.508 | 0.476 | 0.04171633 | 45.7 | -0.970 | 0.275 | 0.00857761 |
| **Mid1** | 60.9 | -1.478 | 0.395 | 0.0126336 | 213.7 | -0.995 | 0.198 | 3.5144E-05 |
| **Ptprn2** | 333.4 | -1.461 | 0.358 | 0.00598384 | 45.8 | -0.994 | 0.309 | 0.01998408 |
| **Ankle2** | 115.9 | -1.429 | 0.350 | 0.00598384 | 99.1 | -0.605 | 0.207 | 0.04246472 |
| **Ccny** | 71.1 | -1.274 | 0.379 | 0.02959143 | 74.7 | -1.034 | 0.258 | 0.00181454 |
| **Galnt13** | 361.3 | -1.244 | 0.341 | 0.0159734 | 504.9 | -0.371 | 0.113 | 0.01655375 |
| **Ablim1** | 135.7 | -1.172 | 0.296 | 0.00760665 | 158.7 | -0.798 | 0.151 | 1.1301E-05 |
| **Acsl3** | 460.5 | -1.075 | 0.309 | 0.0236877 | 703.9 | -1.467 | 0.153 | 3.0866E-18 |
| **Ube3a** | 161.2 | -1.027 | 0.303 | 0.02803579 | 209.5 | -0.688 | 0.187 | 0.00513769 |
| **Socs5** | 358.6 | -0.820 | 0.256 | 0.03984866 | 337.8 | -0.811 | 0.126 | 2.8137E-08 |
| **Heg1** | 1328.6 | -0.795 | 0.209 | 0.01057592 | 1062.7 | -0.378 | 0.067 | 1.6244E-06 |
| **Cand1** | 323.4 | -0.744 | 0.231 | 0.03864972 | 283.1 | -0.597 | 0.187 | 0.02106014 |
| **Gprasp1** | 509.6 | -0.534 | 0.163 | 0.03426564 | 356.1 | -0.416 | 0.145 | 0.04690377 |

The finding of upregulated ETC genes in Txnip-treated cones suggested effects on mitochondria, and thus the morphology of Txnip-treated mitochondria in RP cones was examined by electron microscopy (EM). There was an increase in mitochondrial size by Txnip treatment, with a greater increase in size following treatment with Txnip.C247S **(****FIGS. 5A****, 5B).** Mitochondrial membrane potential (ΔΨm) activity, a reflection of mitochondrial ETC function, was also examined using JC-1 dye staining of freshly explanted Txnip-treated P20 *rd1* retinas (Reers *et al.,* 1995). Both Txnip and Txnip.C247S increased the ratio of J-aggregates:JC1-monomers (Fig. 5c,d), indicating an increased ΔΨm and/or a greater number/size of mitochondria with a high ΔΨm following Txnip overexpression. This finding was further investigated *in vivo* using infection by an AAV encoding mitoRFP, which only accumulates in mitochondria with a high ΔΨm (Brodier *et al.,* 2020; Hood *et al.,* 2003). Compared to the control cones without Txnip treatment, the intensity of mitoRFP was higher in P20 *rd1* cones treated with Txnip **(****FIGS. 13C****, 13D).**

A previous study identified 15 proteins that interact with Txnip.C247S (Forred *et al.,* 2016). Among these interactors was Parp1, which can negatively affect mitochondria through deleterious effects on the mitochondrial genome (Hocsak *et al.,* 2017; Szczesny *et al.,* 2014), as well as have effects on inflammation and other cellular pathways (Fehr *et al.,* 2020). Due to the similarities between the effects of Txnip addition and of Parp1 inhibition on mitochondria, Parp1 was tested for a potential role in Txnip-mediated rescue. Parp1 expression was first examined by immunohistochemistry and found to be enriched in cone inner segments, which are packed with mitochondria (Hoang *et al.,* 2002), and in cone nuclei **(****FIG. 13G****).** Interestingly, these are the same locations where a GFP-Txnip fusion protein was found **(****FIG. 9B****).** To test for a role of Parp1, *parp1*^{*-*/*-*} mice were bred to *rd1* mice, and their cone mitochondria were examined by EM and mitoRFP. *Parp1*^{*-*/}*⁻ rd1* cones possessed larger mitochondria **(****FIGS. 13H, 13I**) and higher mitoRFP signals than cones from *parp1*^{*+*/}*⁺ rd1* controls. Addition of Txnip.C247S to *parp1*^{*-*/}*⁻ rd1* cones did not alter the mitoRFP signals (Fig. 5e,f). However, when Txnip.C247S was added to *parp1*^{*-*/}*⁻ rd1* retinas, cone survival was similar to that of Txnip.C247S-treatred *parp1*^{*+*/}*⁺ rd1* retinas, showing that Txnip-mediated survival does not require Parp1 **(****FIGS. 5G****, 5H).**

The discordance between improved mitochondria and cone survival in these experiments suggested that mitochondrial improvement alone is not sufficient to prolong cone survival. This is consistent with the observations from treatment with Txnip.S308A, as well as Txnip + siLdhb, both of which failed to prolong *rd1* cone survival despite improvements in mitochondria **(****FIGS. 2A, 2B****, 5A-5D** and **13C-13F).** To test if improved cone survival requires both mitochondrial improvement and enhanced lactate catabolism, we delivered Ldhb to *parp1*^{*-*/}*⁻ rd1* cones. A small but significant improvement in cone survival was observed **(****FIGS. 5I, 5J**).

### Example 6: Txnip enhances Na+/K+ pump function and cone opsin expression.

The results above suggest that Txnip may prolong RP cone survival by enhancing lactate catabolism via Ldhb, which may lead to greater ATP production by the oxidative phosphorylation (OXPHOS) pathway. Cone photoreceptors are known to require high levels of ATP to maintain their membrane potential, relying primarily upon the Na⁺/K⁺ ATPase pump (Ingram *et al.,* 2020). To investigate whether Txnip affects the function of the Na⁺/K⁺ pump in RP cones, freshly explanted P20 *rd1* retinas were treated with RH421, a fluorescent small-molecule probe for Na+/K+ pump function (Fedosova *et al.,* 1995). Addition of Txnip improved Na⁺/K⁺ pump function of these cones in lactate medium as reflected by an increase in RH421 fluorescence **(****FIGS. 6A****, 6B),** consistent with Txnip enabling greater utilization of lactate. In RP cones, it is also known that protein expression of cone opsin is down-regulated, postulated to be due to insufficient energy supply (Punzo *et al.,* 2009). Compared to control, greater anti-opsin staining was observed in Txnip-treated *rd1* cones at P50 **(****FIG. 6C****),** further supporting the idea that Txnip improves the energy supply to RP cones.

### Example 7: Dominant-negative HIF1α improves RP cone survival.

If improved lactate catabolism and OXPHOS are at least part of the mechanism of Txnip rescue, RP cone survival might be promoted by other molecules serving similar functions. HIF1α can upregulate the transcription of glycolytic genes (Majmundar *et al.,* 2010). Increased glycolytic enzyme levels might push RP cones to rely on glucose, rather than lactate, to their detriment if glucose is limited. To investigate whether HIF1α might play a role in cone survival, a wt and a dominant-negative HIF1α (dnHIF1α) allele (Jiang *et al.,* 1996) were delivered to *rd1* retinas using AAV. A target gene of HIF1α, *vegf,* which might improve blood flow and thus nutrient delivery, also was tested. The dnHIF1α increased *rd1* cone survival, while wt HIF1α and Vegf each decreased cone survival **(****FIGS. 7A, 7B****,** **14D 14E****).**

### Example 8: Txnip effects on Glut1 levels in the RPE and cone survival.

Several lines of evidence support the hypothesis that RP cones do not have sufficient glucose to satisfy their needs via glycolysis (Chinchore *et al.,* 2019; Kanow *et al.,* 2017; Punzo *et al.,* 2012, 2009; Wang *et al.,* 2016). To determine if retention of glucose by the RPE might underlie a glucose shortage for cones (Kanow *et al.,* 2017; Wang *et al.,* 2016), we attempted to reprogram RPE metabolism to a more "OXPHOS" and less "glycolytic" status by overexpressing Txnip or dnHIF1α with an RPE-specific promoter, the Best1 promoter (Esumi *et al.,* 2009). The goal was to increase lactate consumption in the RPE, thus freeing up more glucose for delivery to cones. However, no RP cone rescue was observed **(****FIG. 14B****),** possibly due to a clearance of Glut1 from the surface of cells, which would create a glucose shortage for both the RPE and the cones (Swarup *et al.,* 2019) **(****FIG. 14A****).** To examine the level of Glut1 in the RPE following introduction of wt Txnip, or Txnip.C247S.LL351&352AA, which should prevent efficient removal of Glut1, immunohistochemistry for Glut1 was carried out. This assay showed that AAV-Best1-Txnip.LL351&352AA did result in less clearance of Glut1 from the surface of the RPE **(****FIG. 14A****)** relative to wt Txnip. Txnip.C247S.LL351&352AA was then tested for *rd1* cone rescue, where it was found to improve cone survival **(****FIGS. 7A****, 7B),** in keeping with the model that the RPE retains glucose to the detriment of cones in RP.

### Example 9: Combination of Txnip.C247S with other rescue genes provides an additive effect.

Finally, as the goal is to provide effective, generic gene therapy for RP, and potentially other diseases that affect photoreceptor survival, combinations of AAVs that encode genes that we have previously shown prolong RP cone survival and vision were used. The combination of Txnip.C247S expression in cones, with expression of Nrf2, a gene with anti-oxidative damage and anti-inflammatory activity, in the RPE, provided an additive effect on cone survival relative to either gene alone **(****FIGS. 8A****, 8B).** This combination also preserved the RP cone outer segments, which is the structure packed with opsin for photon detection, and reduced the mislocalization of opsin to the plasma membrane **(****FIGS. 8C****).** An interesting phenotype that is especially prominent in the FVB *rd1* strain is that of "craters" in the photoreceptor layer. These are areas of circumscribed cone death that are obvious when the retina is viewed as a flat-mount. AAV-Best1-Nrf2 alone suppressed the formation of these craters (Wu *et al.,* n.d.), while AAV-RedO-Txnip did not, despite the fact that AAV-RedO-Txnip.C247S provides the most robust RP cone rescue that we have seen **(****FIGS. 2A****, 6F, 6H).** An additional combination that was tested was AAV-RedO-Txnip.C247S with AAV-RedO-Tgfbl, an anti-inflammatory gene (Wang *et al.,* 2020). This combination did not improve cone survival beyond that of Txnip alone, but almost completely eliminated the craters (Fig. 8d,e). In addition, we tried other genes in combination with wt Txnip, but did not observe any obvious improvement over Txnip alone **(****FIG. 14E**).

### Example 10: Additional Txnip.C247S vectors

Three additional AAV vectors were constructed **(****FIGS. 28A-28E****, 29A-29E and 30A-30E)** and tested in *rd1* mice, as described above. The vectors comprise the C terminal domain of Txnip, which was about 60% of the full Txnip sequence, and comprise either the C247S variant or the C247S.LL351.352AA varaint. The vectors were subretinally injected into the eyes of neonatal *rd1* mice, in combination with the AAV-RedO-H2BGFP vector for automated quantification. As a control, eyes were injected with AAV-RedO-H2BGFP alone. As shown in **FIGS. 31A** **and** **31B****,** treatment of the vector comprising the C terminus of Txnip with the C247S mutation under the control of a Best1 promoter (AAV-Best1-C.Txnip.C247S) led to a significant increase in P50 *rd1* cones when compared to the control, suggesting that this vector was capable of cone rescue in RP mice.

### Discussion

Retinitis pigmentosa (RP) is one of the most prevalent types of inherited retinal diseases, affecting approximately one in 3,000 people (Hartong *et al.,* 2006). In RP, the rod photoreceptors, which initiate night vision, are primarily affected by the disease genes, and degenerate first. The degeneration of cones, the photoreceptors that initiate daylight, color and high acuity vision, then follows, which greatly impacts the quality of life. Currently, one therapy that holds great promise for RP is gene therapy using AAV (Maguire *et al.,* 2019). This approach has proven successful for a small number of genes affecting a few disease families (Cehajic-Kapetanovic *et al.,* 2020). However, due to the number and functional heterogeneity of RP disease genes (≈100 genes that primarily affect rods (https://sph.uth.edu/retnet/), gene therapy for each RP gene will be logistically and financially difficult. In addition, a considerable number of RP patients do not have an identified disease gene. A disease gene-agnostic treatment aimed at prolonging cone function/survival in the majority of RP patients could thus benefit many more patients. Given that the disease gene is typically not expressed in cones, answers to the question of why cones die are crucial for this strategy to be successful. To date, the suggested mechanisms include oxidative damage (Komeima *et al.,* 2006; Wellard *et al.,* 2005; Xiong *et al.,* 2015), inflammation (Wang *et al.,* 2020, 2019; Zhao *et al.,* 2015), and a shortage of nutrients (Aït-Ali *et al.,* 2015; Kanow *et al.,* 2017; Punzo *et al.,* 2012, 2009; Wang *et al.,* 2016).

In 2009, gene expression changes that occurred during retinal degeneration in four mouse models of RP were surveyed (Punzo *et al.,* 2009). Those data led us to suggest a model wherein cones starve and die due to a shortage of glucose, which is typically used for energy and anabolic needs in photoreceptors *via* glycolysis. Evidence for this "glucose shortage hypothesis" was subsequently provided by orthogonal approaches from other groups (Aït-Ali *et al.,* 2015; Wang *et al.,* 2016). In order to determine whether genes that affect the uptake and/or utilization of glucose by cones can be used to treat RP regardless of the mutation, the effect of >20 genes that might affect the uptake and/or utilization of glucose by cones *in vivo* in three mouse models of RP was analyzed. Only one gene, *txnip,* had a beneficial effect, prolonging cone survival and visual acuity in these models. *Txnip* encodes an α-arrestin family member protein with multiple functions, including binding to thioredoxin (Junn *et al.,* 2000; Nishiyama *et al.,* 1999), facilitating removal of the glucose transporter 1 (Glut1), from the cell membrane (Wu *et al.,* 2013), and promoting the use of non-glucose fuels (DeBalsi *et al.,* 2014). A number of *txnip* alleles were tested and it was found that one allele, C247S, which blocks the association of Txnip with thioredoxin (Patwari *et al.,* 2009), provided the greatest benefit. Investigation of the mechanism of Txnip rescue revealed that it required lactate dehydrogenase b (Ldhb), which catalyzes the conversion of lactate to pyruvate. Imaging of metabolic reporters demonstrated an enhanced cytosolic ATP:ADP ratio when the retina was placed in lactate medium. Moreover, mitochondria appeared to be healthier as a result of Txnip addition, but this improvement was not sufficient for cone rescue.

The above observations led to a model wherein Txnip shifts cones from their normal reliance on glucose to enhanced utilization of lactate, as well as marked improvement in mitochondrial structure and function. Analysis of the rescue activity of several additional genes predicted to affect glycolysis, provided support for this model. Finally, as the goal is to rescue cones that suffer not only from metabolic challenges, but also from inflammation and oxidative damage, Txnip in combination with anti-inflammatory and anti-oxidative damage genes were tested, and additive benefits for cones were found. These treatments will benefit cones not only in RP, but also in other ocular diseases where similar environmental stresses are present, such as in age related macular degeneration (AMD).

Photoreceptors have been characterized as being highly glycolytic, even under aerobic conditions, as originally described by Otto Warburg (Warburg, 1925). Glucose appears to be supplied primarily from the circulation, *via* the RPE, which has a high level of Glut1 (Gospe *et al.,* 2010). Photoreceptors, at least rods, carry out glycolysis to support anabolism, to replace their outer segments (Chinchore *et al.,* 2017), and contribute ATP, to run their ion pumps (Okawa *et al.,* 2008). If glucose becomes limited, as has been proposed to occur in RP, cones may have insufficient fuel for their needs. To explore whether a therapy to address some of these metabolic shortcomings in RP could be developed, many different types of genes that might alter metabolic programming were delivered. From these, Txnip had the strongest benefit on cone survival and vision **(****FIGS. 1A-1C** and **FIGS. 9A-9E****).** This was surprising as Txnip has been shown to inhibit glucose uptake, by binding to and aiding in the removal of Glut1 from plasma membrane, and it inhibits the anti-oxidation proteins, the thioredoxins, again by direct binding. The results with Txnip in its wild-type (wt) form, and from the study of several mutant alleles, provide some insight into how it might benefit cones. The Txnip.C247S allele prevents binding to thioredoxins, and gave enhanced cone survival relative to wt Txnip **(****FIGS. 2A, 2B** and **FIGS. 10A-10D****).** Thus, by being free of this interaction, the C247S mutant protein may be more available for other Txnip-mediated activities. In addition, thioredoxin may be made more available for its role in fighting oxidative damage.

The mechanisms by which Txnip might benefit cones are not fully known, but a study of Txnip's function in skeletal muscle suggested that it plays a role in fuel selection (DeBalsi *et al.,* 2014). If glucose is limited in RP, then cones may need to switch from a reliance on glucose and glycolysis to an alternative fuel(s), such as ketones, fatty acids, amino acids, or lactate. Cones express *oxct1* mRNA (Shekhar *et al.,* 2016), which encodes a critical enzyme for ketone catabolism, suggesting cones are capable of ketolysis. In addition, a previous study showed that lipids might be an alternative energy source for cones by β-oxidation (Joyal *et al.,* 2016). It is likely that cones can use these alternative fuels to meet their intense energy demands (Ingram *et al.,* 2020) **(****FIGS. 6A-6C****, 7A-7B).** However, the Txnip rescue did not depend on ketolysis or β-oxidation **(****FIGS. 3A-3C****).** Due to the diversity of amino acid catabolic pathways, these pathways were not studied to fdetermine if they were required for Txnip's rescue effect. However, it was discovered that Ldhb, which converts lactate to pyruvate, was required. This is an interesting switch, as photoreceptors normally have high levels of Ldha, and produce lactate (Chinchore *et al.,* 2017). An important factor in the reliance on Ldhb could be the availability of lactate, which is highly available from serum (Hui *et al.,* 2017). Lactate could be transported *via* the RPE and/or Müller glia, and/or the internal retinal vasculature which comes in closer proximity to cones after rod death. Ketones are usually only available during fasting, and lipids are hydrophobic molecules which are slow to be transported across the plasma membranes. Moreover, lipids are required to rebuild the membrane-rich outer segments, and thus might be somewhat limited. Ldhb is not sufficient, however, to delay RP cone degeneration, as its overexpression did not promote RP cone survival.

Txnip-treated RP cones also had larger mitochondria with a greater membrane potential, and likely were able to use the pyruvate produced by Ldhb for greater ATP production *via* OXPHOS. Indeed, Txnip-treated cones had an enhanced ATP:ADP ratio **(****FIGS. 4A-4E****).** However, healthier mitochondria were not sufficient to prolong RP cone survival. Txnip.S308A led to larger mitochondria than control mitochondria, brighter JC-1 staining and mitoRFP signals, which are indicators of better mitochondrial health, but this allele did not induce greater cone survival **(****FIGS. 5A-5J** and **FIGS. 13A-13I**). Moreover, as Txnip has been shown to interact with Parp1, which can negatively affect mitochondria, we investigated if Parp1 knock-out mice might have cones that survive longer in RP. Indeed, the Parp1 knock-out mitochondria appeared to be healthier, but Parp1 knock-out retinas did not have better RP cone survival than Parpl-wt *rd1* retinas. In addition, cone rescue by Txnip was not changed in the Parp1 knock-out retinas.

The well-described effects of Txnip on the removal of Glut1 from the cell membrane might seem at odds with the promotion of cone survival. It could be that removal of Glut1 from the plasma membrane of cones forces the cones to choose an alternative fuel, such as lactate, and perhaps others too. Interestingly, as Glut1 knock-down was not sufficient for cone survival, Txnip must not only lead to a reduction in membrane localized Glut1, but also potentiate a fuel switch, *via* an unknown mechanism(s) that at least involves an increase of Ldhb activity. A reduction in glycolysis might also lead to a fuel switch. Introduction of dnHIF1α, which should reduce expression of glycolytic enzymes, also benefitted cones, while introduction of wt HIF1α did not **(****FIGS. 7A-7B****).** HIF1α has many target genes, and may alter pathways in addition to that of glycolysis, thus also potentiating a fuel switch once glycolysis is down regulated. An additional finding supporting the notion that the level of glycolysis is important for cone survival was the observation that AAV-Pfkm plus AAV-Hk1 led to a reduction in cone survival **(****FIG. 9E****).** Phosphorylation of glucose by Hk1 followed by phosphorylation of fructose-6-phosphate by the Pfkm complex commits glucose to glycolysis at the cost of ATP. These AAVs may have promoted the flux of glucose through glycolysis, which may have inhibited a fuel switch, and/or depleted the ATP pool, *e.g.* if downstream glycolytic intermediates were used for anabolic needs so that ATP production by glycolysis did not occur.

The observations described above suggest that at least two different pathways are required for the promotion of cone survival by Txnip **(****FIGS. 16A-16B****).** One pathway requires lactate utilization *via* Ldhb, but as Ldhb was not sufficient, another pathway is also required. As greater mitochondrial health was observed following Txnip treatment, a second pathway may include the effects on mitochondria. This notion is supported by the observation that the addition of Ldhb to *parp1*^{*-*/}*⁻ rd1* cones, which have healthier mitochondria, led to improved cone survival **(****FIGS. 5A-5J****).** Txnip alone may be able to promote cone health by impacting both lactate catabolism and mitochondrial health. There may be additional pathways required as well.

The effects of Txnip alleles expressed only in the RPE provide some support for the hypothesis that the RPE transports glucose to cones for their use, while primarily using lactate for its own needs (Kanow *et al.,* 2017; Swarup *et al.,* 2019). Lactate is normally produced at high levels by photoreceptors in the healthy retina. When rods, which are 97% of the photoreceptors, die, lactate production goes down dramatically. The RPE might then need to retain glucose for its own needs. Introduction of an allele of Txnip, C247S.LL351&352AA, to the RPE provided a rescue effect for cones, while introduction of the wt allele of Txnip to the RPE did not. The LL351&352AA mutations lead to a loss of efficiency of the removal of Glut1 from the plasma membrane, while the C247S mutation might create an even less glycolytic RPE. The combination of these mutations might then allow more glucose to flow to cones. The untreated RP cones seem to be able to use glucose at a high concentration for ATP production, at least in freshly explanted retinas **(****FIG. 4A****).** These findings are also consistent with the reported mechanism for cone survival promoted by RdCVF, a factor that is proposed to improve glucose uptake by RP cones, which might be important if glucose is present in low concentration due to retention by the RPE (Aït-Ali *et al.,* 2015; Byrne *et al.,* 2015).

As cones face multiple challenges in the degenerating RP retina,Txnip in combination with genes that have been found to promote cone survival *via* other mechanisms were tested. The combination of Txnip with vectors fighting oxidative stress (AAV-Best1-Nrf2) or inflammation (AAV-RedO-Tgfbl) supported greater cone survival than any of these treatments alone. These combinations utilize cell type-specific promoters, reducing the chances of side effects from global expression of these genes. Of note, the Nrf2 expression was limited to the RPE, yet was additive for cone survival. This finding is in keeping with the interdependence of photoreceptors and the RPE, which is undoubtably important not only in a healthy retina, but in disease as well.

### References:

Aït-Ali N, Fridlich R, Millet-Puel G, Clérin E, Delalande F, Jaillard C, Blond F, Perrocheau L, Reichman S, Byrne LCC, Olivier-Bandini A, Bellalou J, Moyse E, Bouillaud F, Nicol X, Dalkara D, van Dorsselaer A, Sahel J-A, Léveillard T, van Dorsselaer A, Sahel J-A, Léveillard T. 2015. Rod-Derived Cone Viability Factor Promotes Cone Survival by Stimulating Aerobic Glycolysis. Cell 161:817-832. doi:10.1016/j.cell.2015.03.023
Anders S, Huber W. 2010. Differential expression analysis for sequence count data. Genome Biol 11:R106. doi:10.1186/gb-2010-11-10-r106
Brodier L, Rodrigues T, Matter-Sadzinski L, Matter J-M. 2020. A transient decrease in mitochondrial activity is required to establish the ganglion cell fate in retina adapted for high acuity vision. bioRxiv 2020.03.23.002998. doi:10.1101/2020.03.23.002998
Byrne LC, Dalkara D, Luna G, Fisher SK, Clérin E, Sahel J-A, Léveillard T, Flannery JG. 2015. Viral-mediated RdCVF and RdCVFL expression protects cone and rod photoreceptors in retinal degeneration. J Clin Invest 125:105-16. doi:10.1172/JCI65654
Cehajic-Kapetanovic J, Xue K, Martinez-Fernandez de la Camara C, Nanda A, Davies A, Wood LJ, Salvetti AP, Fischer MD, Aylward JW, Barnard AR, Jolly JK, Luo E, Lujan BJ, Ong T, Girach A, Black GCM, Gregori NZ, Davis JL, Rosa PR, Lotery AJ, Lam BL, Stanga PE, MacLaren RE. 2020. Initial results from a first-in-human gene therapy trial on X-linked retinitis pigmentosa caused by mutations in RPGR. Nat Med. doi:10.1038/s41591-020-0763-1
Chang B, Hawes NL, Pardue MT, German AM, Hurd RE, Davisson MT, Nusinowitz S, Rengarajan K, Boyd AP, Sidney SS, Phillips MJ, Stewart RE, Chaudhury R, Nickerson JM, Heckenlively JR, Boatright JH. 2007. Two mouse retinal degenerations caused by missense mutations in the β-subunit of rod cGMP phosphodiesterase gene. Vision Res 47:624-633. doi: 10. 1016/j.visres.2006. 11.020
Chinchore Y, Begaj T, Guillermeir C, Steinhauser M, Punzo C, Cepko C. 2019. Transduction of gluconeogenic enzymes prolongs cone photoreceptor survival and function in models of retinitis pigmentosa. bioRxiv 569665. doi:10.1101/569665
Chinchore Y, Begaj T, Wu D, Drokhlyansky E, Cepko CL. 2017. Glycolytic reliance promotes anabolism in photoreceptors. Elife 6. doi:10.7554/eLife.25946
DeBalsi KL, Wong KE, Koves TR, Slentz DH, Seiler SE, Wittmann AH, Ilkayeva OR, Stevens RD, Perry CGR, Lark DS, Hui ST, Szweda L, Neufer PD, Muoio DM. 2014. Targeted Metabolomics Connects Thioredoxin-interacting Protein (TXNIP) to Mitochondrial Fuel Selection and Regulation of Specific Oxidoreductase Enzymes in Skeletal Muscle. J Biol Chem 289:8106-8120. doi:10.1074/jbc.M113.511535
Esumi N, Kachi S, Hackler L, Masuda T, Yang Z, Campochiaro PA, Zack DJ. 2009. BEST1 expression in the retinal pigment epithelium is modulated by OTX family members. Hum Mol Genet 18:128-141. doi:10.1093/hmg/ddn323
Eventoff W, Rossmann MG, Taylor SS, Torff HJ, Meyer H, Keil W, Kiltz HH. 1977. Structural adaptations of lactate dehydrogenase isozymes. Proc Natl Acad Sci U S A 74:2677-2681. doi:10.1073/pnas.74.7.2677
Fedosova NU, Cornelius F, Klodos I. 1995. Fluorescent Styryl Dyes as Probes for Na,K-ATPase Reaction Mechanism: Significance of the Charge of the Hydrophilic Moiety of RH Dyes. Biochemistry 34:16806-16814. doi:10.1021/bi00051a031
Fehr AR, Singh SA, Kerr CM, Mukai S, Higashi H, Aikawa M. 2020. The impact of PARPs and ADP-ribosylation on inflammation and host-pathogen interactions. Genes Dev. doi:10.1101/gad.334425.119
Ferrando RE, Newton K, Chu F, Webster JD, French DM. 2015. Immunohistochemical Detection of FLAG-Tagged Endogenous Proteins in Knock-In Mice. J Histochem Cytochem 63:244-255. doi:10.1369/0022155414568101
Forred BJ, Neuharth S, Kim DI, Amolins MW, Motamedchaboki K, Roux KJ, Vitiello PF. 2016. Identification of Redox and Glucose-Dependent Txnip Protein Interactions. Oxid Med Cell Longev 2016. doi:10.1155/2016/5829063
Giering JC, Grimm D, Storm TA, Kay MA. 2008. Expression of shRNA from a tissue-specific pol II promoter is an effective and safe RNAi therapeutic. Mol Ther 16:1630-1636. doi:10.1038/mt.2008.144
Gospe SM, Baker SA, Arshavsky VY. 2010. Facilitative glucose transporter Glut1 is actively excluded from rod outer segments. J Cell Sci 123:3639-3644. doi:10.1242/jcs.072389
Grenell A, Wang Y, Yam M, Swarup A, Dilan TL, Hauer A, Linton JD, Philp NJ, Gregor E, Zhu S, Shi Q, Murphy J, Guan T, Lohner D, Kolandaivelu S, Ramamurthy V, Goldberg AFX, Hurley JB, Du J. 2019. Loss of MPC1 reprograms retinal metabolism to impair visual function. Proc Natl Acad Sci U S A 116:3530-3535. doi:10.1073/pnas.1812941116
Grieger JC, Choi VW, Samulski RJ. 2006. Production and characterization of adeno-associated viral vectors. Nat Protoc. doi:10.1038/nprot.2006.207
Hartong DT, Berson EL, Dryja TP. 2006. Retinitis pigmentosa. Lancet 368:1795-809. doi: 10.10 16/S0 140-6736(06)69740-7
Hoang Q, Linsenmeier R, Chung C, Curcio C. 2002. Photoreceptor inner segments in monkey and human retina: mitochondrial density, optics, and regional variation. Vis Neurosci 19:395-407. doi:10.1017/s0952523802194028
Hocsak E, Szabo V, Kalman N, Antus C, Cseh A, Sumegi K, Eros K, Hegedus Z, Gallyas F, Sumegi B, Racz B. 2017. PARP inhibition protects mitochondria and reduces ROS production via PARP-1-ATF4-MKP-1-MAPK retrograde pathway. Free Radic Biol Med 108:770-784. doi:10.1016/j.freeradbiomed.2017.04.018
Hood DA, Adhihetty PJ, Colavecchia M, Gordon JW, Irrcher I, Joseph AM, Lowe ST, Rungi AA. 2003. Mitochondrial biogenesis and the role of the protein import pathway. Med Sci Sports Exerc 35:86-94. doi:10.1097/00005768-200301000-00015
Hui S, Ghergurovich JM, Morscher RJ, Jang C, Teng X, Lu W, Esparza LA, Reya T, Zhan L, Yanxiang Guo J, White E, Rabinowitz JD. 2017. Glucose feeds the TCA cycle via circulating lactate. Nature 551:115-118. doi:10.1038/nature24057
Ingram NT, Fain GL, Sampath AP. 2020. Elevated energy requirement of cone photoreceptors. Proc Natl Acad Sci U S A 117:19599-19603. doi:10.1073/pnas.2001776117
Jiang BH, Rue E, Wang GL, Roe R, Semenza GL. 1996. Dimerization, DNA binding, and transactivation properties of hypoxia- inducible factor 1. J Biol Chem 271:17771-17778. doi:10.1074/jbc.271.30.17771
Joyal J-S, Sun Y, Gantner ML, Shao Z, Evans LP, Saba N, Fredrick T, Burnim S, Kim JS, Patel G, Juan AM, Hurst CG, Hatton CJ, Cui Z, Pierce KA, Bherer P, Aguilar E, Powner MB, Vevis K, Boisvert M, Fu Z, Levy E, Fruttiger M, Packard A, Rezende FA, Maranda B, Sapieha P, Chen J, Friedlander M, Clish CB, Smith LEH. 2016. Retinal lipid and glucose metabolism dictates angiogenesis through the lipid sensor Ffarl. Nat Med 22:439-445. doi:10.1038/nm.4059
Junn E, Han SH, Im JY, Yang Y, Cho EW, Um HD, Kim DK, Lee KW, Han PL, Rhee SG, Choi I. 2000. Vitamin D3 up-regulated protein 1 mediates oxidative stress via suppressing the thioredoxin function. J Immunol 164:6287-95.
Jüttner J, Szabo A, Gross-Scherf B, Morikawa RK, Rompani SB, Hantz P, Szikra T, Esposti F, Cowan CS, Bharioke A, Patino-Alvarez CP, Keles Ö, Kusnyerik A, Azoulay T, Hartl D, Krebs AR, Schübeler D, Hajdu RI, Lukats A, Nemeth J, Nagy ZZ, Wu KC, Wu RH, Xiang L, Fang XL, Jin ZB, Goldblum D, Hasler PW, Scholl HPN, Krol J, Roska B. 2019. Targeting neuronal and glial cell types with synthetic promoter AAVs in mice, non-human primates and humans. Nat Neurosci 22:1345-1356. doi:10.1038/s41593-019-0431-2
Kanow MA, Giarmarco MM, Jankowski CS, Tsantilas K, Engel AL, Du J, Linton JD, Farnsworth CC, Sloat SR, Rountree A, Sweet IR, Lindsay KJ, Parker ED, Brockerhoff SE, Sadilek M, Chao JR, Hurley JB. 2017. Biochemical adaptations of the retina and retinal pigment epithelium support a metabolic ecosystem in the vertebrate eye. Elife 6. doi:10.7554/eLife.28899
Katsu-Jiménez Y, Vázquez-Calvo C, Maffezzini C, Halldin M, Peng X, Freyer C, Wredenberg A, Giménez-Cassina A, Wedell A, Arnér ESJ. 2019. Absence of TXNIP in humans leads to lactic acidosis and low serum methionine linked to deficient respiration on pyruvate. Diabetes 68:709-723. doi:10.2337/db18-0557
Keller J, Marvin J, Lacin H, Lemon W, Shea J, Kim S, Lee R, Koyama M, Keller P, Looger L. 2019. In Vivo Glucose Imaging in Multiple Model Organisms with an Engineered Single-Wavelength Sensor. SSRN Electron J 571422. doi:10.1101/571422
Komeima K, Rogers BS, Lu L, Campochiaro PA. 2006. Antioxidants reduce cone cell death in a model of retinitis pigmentosa. Proc Natl Acad Sci U S A 103:11300-11305. doi:10.1073/pnas.0604056103
Lem J, Krasnoperova N V., Calvert PD, Kosaras B, Cameron DA, Nicolò M, Makino CL, Sidman RL. 1999. Morphological, physiological, and biochemical changes in rhodopsin knockout mice. Proc Natl Acad Sci U S A 96:736-741. doi:10.1073/pnas.96.2.736
Liao Y, Smyth GK, Shi W. 2014. FeatureCounts: An efficient general purpose program for assigning sequence reads to genomic features. Bioinformatics 30:923-930. doi: 10.1 093/bioinformatics/btt656
Maguire AM, Russell S, Wellman JA, Chung DC, Yu ZF, Tillman A, Wittes J, Pappas J, Elci O, Marshall KA, McCague S, Reichert H, Davis M, Simonelli F, Leroy BP, Wright JF, High KA, Bennett J. 2019. Efficacy, Safety, and Durability of Voretigene Neparvovec-rzyl in RPE65 Mutation-Associated Inherited Retinal Dystrophy: Results of Phase 1 and 3 Trials. Ophthalmology 126:1273-1285. doi:10.1016/j.ophtha.2019.06.017
Majmundar AJ, Wong WJ, Simon MC. 2010. Hypoxia-inducible factors and the response to hypoxic stress. Mol Cell 40:294-309. doi:10.1016/j.molcel.2010.09.022
Matsuda T, Cepko CL. 2007. Controlled expression of transgenes introduced by in vivo electroporation. Proc Natl Acad Sci 104:1027-1032. doi:10.1073/pnas.0610155104
Nishinaka Y, Masutani H, Nakamura H, Yodoi J. 2001. Regulatory roles of thioredoxin in oxidative stress-induced cellular responses. Redox Rep. doi:10.1179/135100001101536427
Nishiyama A, Matsui M, Iwata S, Hirota K, Masutani H, Nakamura H, Takagi Y, Sono H, Gon Y, Yodoi J. 1999. Identification of thioredoxin-binding protein-2/vitamin D(3) up-regulated protein 1 as a negative regulator of thioredoxin function and expression. J Biol Chem 274:21645-50.
Okawa H, Sampath AP, Laughlin SB, Fain GL. 2008. ATP consumption by mammalian rod photoreceptors in darkness and in light. Curr Biol 18:1917-21. doi:10.1016/j.cub.2008.10.029
Patwari P, Chutkow WA, Cummings K, Verstraeten VLRM, Lammerding J, Schreiter ER, Lee RT. 2009. Thioredoxin-independent regulation of metabolism by the α-arrestin proteins. J Biol Chem 284:24996-25003. doi:10.1074/jbc.M109.018093
Picelli S, Björklund ÅK, Faridani OR, Sagasser S, Winberg G, Sandberg R. 2013. Smart-seq2 for sensitive full-length transcriptome profiling in single cells. Nat Methods 10:1096-1098. doi:10.1038/nmeth.2639
Prusky GT, Alam NM, Beekman S, Douglas RM. 2004. Rapid quantification of adult and developing mouse spatial vision using a virtual optomotor system. Investig Ophthalmol Vis Sci 45:4611-4616. doi:10.1167/iovs.04-0541
Punzo C, Kornacker K, Cepko CL. 2009. Stimulation of the insulin/mTOR pathway delays cone death in a mouse model of retinitis pigmentosa. Nat Neurosci 12:44-52. doi:10.1038/nn.2234
Punzo C, Xiong W, Cepko CL. 2012. Loss of daylight vision in retinal degeneration: are oxidative stress and metabolic dysregulation to blame? J Biol Chem 287:1642-8. doi: 10.107 4/jbc.R111.304428
Reers M, Smiley ST, Mottola-Hartshorn C, Chen A, Lin M, Chen LB. 1995. Mitochondrial membrane potential monitored by JC-1 dye. Methods Enzymol 260:406-17.
Robinson C, Stringer S. 2001. The splice variants of vascular endothelial growth factor (VEGF) and their receptors - PubMed. J Cell Sci 114:853-65.
Shekhar K, Lapan SW, Whitney IE, Tran NM, Macosko EZ, Kowalczyk M, Adiconis X, Levin JZ, Nemesh J, Goldman M, McCarroll SA, Cepko CL, Regev A, Sanes JR. 2016. Comprehensive Classification of Retinal Bipolar Neurons by Single-Cell Transcriptomics. Cell 166:1308-1323.e30. doi:10.1016/j.cell.2016.07.054
Shriver LP, Manchester M. 2011. Inhibition of fatty acid metabolism ameliorates disease activity in an animal model of multiple sclerosis. Sci Rep 1:79. doi:10.1038/srep00079
Swarup A, Samuels IS, Bell BA, Han JYS, Du J, Massenzio E, Abel ED, Boesze-Battaglia K, Peachey NS, Philp NJ. 2019. Modulating GLUT1 expression in retinal pigment epithelium decreases glucose levels in the retina: Impact on photoreceptors and müller glial cells. Am J Physiol - Cell Physiol 316:C121-C133. doi:10.1152/ajpcell.00410.2018
Szczesny B, Brunyanszki A, Olah G, Mitra S, Szabo C. 2014. Opposing roles of mitochondrial and nuclear PARP1 in the regulation of mitochondrial and nuclear DNA integrity: Implications for the regulation of mitochondrial function. Nucleic Acids Res 42:13161-13173. doi:10.1093/nar/gku1089
Tantama M, Hung YP, Yellen G. 2011. Imaging intracellular pH in live cells with a genetically encoded red fluorescent protein sensor. J Am Chem Soc 133:10034-7. doi:10.1021/ja202902d
Tantama M, Martínez-Fran 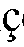 ois JR, Mongeon R, Yellen G. 2013. Imaging energy status in live cells with a fluorescent biosensor of the intracellular ATP-to-ADP ratio. *Nat Commun* **4**:2550. doi:10.1038/ncomms3550
Waldhart AN, Dykstra H, Peck AS, Boguslawski EA, Madaj ZB, Wen J, Veldkamp K, Hollowell M, Zheng B, Cantley LC, McGraw TE, Wu N. 2017. Phosphorylation of TXNIP by AKT Mediates Acute Influx of Glucose in Response to Insulin. Cell Rep 19:2005-2013. doi:10.1016/j.celrep.2017.05.041
Wang S, Sengel C, Emerson MM, Cepko CL. 2014. A Gene Regulatory Network Controls the Binary Fate Decision of Rod and Bipolar Cells in the Vertebrate Retina. Dev Cell 30:513-527. doi:10.1016/j.devcel.2014.07.018
Wang SK, Xue Y, Cepko CL. 2020. Microglia modulation by TGF-β1 protects cones in mouse models of retinal degeneration. J Clin Invest 140:4360-4369. doi:10.1172/JCI136160
Wang SK, Xue Y, Rana P, Hong CM, Cepko CL. 2019. Soluble CX3CL1 gene therapy improves cone survival and function in mouse models of retinitis pigmentosa. Proc Natl Acad Sci U S A 116:10140-10149. doi:10.1073/pnas.1901787116
Wang W, Lee SJ, Scott PA, Lu X, Emery D, Liu Y, Ezashi T, Roberts MR, Ross JW, Kaplan HJ, Dean DC. 2016. Two-Step Reactivation of Dormant Cones in Retinitis Pigmentosa. Cell Rep 15:372-385. doi:10.1016/j.celrep.2016.03.022
Warburg O. 1925. The metabolism of carcinoma cells. J Cancer Res 9:148-163. doi:10.1158/jcr.1925.148
Wellard J, Lee D, Valter K, Stone J. 2005. Photoreceptors in the rat retina are specifically vulnerable to both hypoxia and hyperoxia. Vis Neurosci 22:501-507. doi:10.1017/S0952523805224112
Wu D, Ji X, Ivanchenko M, Chung M, Piper M, Wang S, Xue Y, Xu H, West E, Zhao S, Xiong W, Cepko C. n.d. Nrf2 overexpression rescues the RPE in mouse models of retinitis pigmentosa. JCI Insight.
Wu N, Zheng B, Shaywitz A, Dagon Y, Tower C, Bellinger G, Shen C-H, Wen J, Asara J, McGraw TE, Kahn BB, Cantley LC. 2013. AMPK-dependent degradation of TXNIP upon energy stress leads to enhanced glucose uptake via GLUT1. Mol Cell 49:1167-75. doi:10.1016/j.molcel.2013.01.035
Xiong W, MacColl Garfinkel AE, Li Y, Benowitz LI, Cepko CL. 2015. NRF2 promotes neuronal survival in neurodegeneration and acute nerve damage. J Clin Invest 125:1433-45. doi:10.1172/1CI79735
Xiong W, Wu DM, Xue Y, Wang SK, Chung MJ, Ji X, Rana P, Zhao SR, Mai S, Cepko CL. 2019. AAV cis-regulatory sequences are correlated with ocular toxicity. Proc Natl Acad Sci U S A 116:5785-5794. doi:10.1073/pnas.1821000116
Ye GJ, Budzynski E, Sonnentag P, Nork TM, Sheibani N, Gurel Z, Boye SL, Peterson JJ, Boye SE, Hauswirth WW, Chulay JD. 2016. Cone-Specific Promoters for Gene Therapy of Achromatopsia and Other Retinal Diseases. Hum Gene Ther 27:72-82. doi:10.1089/hum.2015.130
Zhang S, Xie C. 2017. The role of OXCT1 in the pathogenesis of cancer as a rate-limiting enzyme of ketone body metabolism. Life Sci 183:110-115. doi:10.1016/j.lfs.2017.07.003
Zhao L, Zabel MK, Wang X, Ma W, Shah P, Fariss RN, Qian H, Parkhurst CN, Gan W, Wong WT. 2015. Microglial phagocytosis of living photoreceptors contributes to inherited retinal degeneration. EMBO Mol Med 7:1179-1197. doi:10.15252/emmm.201505298

### Partial List of Sequences Disclosed in the Application

SEQ ID NO:1
   >NP_006463.3 thioredoxin-interacting protein isoform 1 [Homo sapiens]
SEQ ID NO:2
   >NP_001300901.1 thioredoxin-interacting protein isoform 2 [Homo sapiens]
SEQ ID NO:3
   >NP_001009935.1 thioredoxin-interacting protein isoform 1 [Mus musculus]
SEQ ID NO:4
   >NP_076208.2 thioredoxin-interacting protein isoform 2 [Mus musculus]
SEQ ID NO::8
   >NG_009105.2 Homo sapiens opsin 1, long wave sensitive (OPN1LW), RefSeqGene on chromosome X
SEQ ID NO:9
   >NC_000001.11:c109619929-109602906 Homo sapiens chromosome 1, GRCh38.p12 Primary Assembly
SEQ ID NO:10
   WPRE
SEQ ID NO:11
   WPRE
SEQ ID NO:12
   Human beta-globin intron
SEQ ID NO:13
   SV40 poly-adenylation (polyA)
SEQ ID NO:14
   5' ITR
SEQ ID NO:15
   3' ITR
SEQ ID NO:16
   >KT886395.1 Homo sapiens clone PR1.7 red cone opsin gene, promoter region and partial cds
SEQ ID NO:17
   >hg38_knownGene_ENST00000351050.7 range=chr1:109613058-109615057 5'pad=0 3'pad=0 strand=- repeatMasking=none
SEQ ID NO:18
   SynPVI:
SEQ ID NO:19
   SynP136:
SEQ ID NO:20
   SV40 Intron
SEQ ID NO:21
   2A
SEQ ID NO:22
   P2A
SEQ ID NO:23
   T2a
SEQ ID NO:24
   E2a
SEQ ID NO:25
   Bovine Growth Hormone Polyadenylation Signal (BGH pA)
SEQ ID NO:101
   >NM_006472.6 Homo sapiens thioredoxin interacting protein (TXNIP), transcript variant 1, mRNA
SEQ ID NO:102
   >NM_001313972.2 Homo sapiens thioredoxin interacting protein (TXNIP), transcript variant 2, mRNA
SEQ ID NO:103
   >NM_001009935.2 Mus musculus thioredoxin interacting protein (Txnip), transcript variant 1, mRNA
SEQ ID NO:104
   >NM_023719.2 Mus musculus thioredoxin interacting protein (Txnip), transcript variant 2, mRNA
SEQ ID NO:105
   >NP_006463.3 thioredoxin-interacting protein isoform 1 [Homo sapiens]
SEQ ID NO:106
   >NP_001300901.1 thioredoxin-interacting protein isoform 2 [Homo sapiens]
SEQ ID NO:107
   >NP_001009935.1 thioredoxin-interacting protein isoform 1 [Mus musculus]
SEQ ID NO:108
   >NP_076208.2 thioredoxin-interacting protein isoform 2 [Mus musculus]
SEQ ID NO:115
   >TXNIP.C247S.LL351&352AA
SEQ ID NO:116
   >Mouse HIF1a
SEQ ID NO:117
   >Dominant-negative HIF1alpha
SEQ ID NO:118
   Homo sapiens bestrophin 1 (BEST1), RefSeqGene on chromosome 11
   NCBI Reference Sequence: NG_009033.1
   >NG_009033.1 Homo sapiens bestrophin 1 (BEST1), RefSeqGene on chromosome 11
SEQ ID NO:119
   >RO1.7 sequence (modified hRedO promoter)
SEQ ID NO:120
   >-C.Txnip.C247S sequence
SEQ ID NO: 121
   >-C.Txnip.C247S.LL351&352AA sequence
SEQ ID NO: 122 AAV-Best1-**C.Txnip.C247S**
SEQ ID NO: 123 AAV-Best1-**C.Txnip.C247S.LL351&352AA**
SEQ ID NO: 124 AAV-RedO-**C.Txnip.C247S**

## Claims

1. A composition, comprising an adeno-associated virus (AAV) expression cassette, the expression cassette comprising a photoreceptor-specific (PR- specific) promoter and a nucleic acid molecule encoding a C247S variant thioredoxin-interacting 5 protein (TXNIP), wherein the PR-specific promoter is a human red opsin (hRedO) promoter.

2. The composition of claim 1, wherein:
(a) the hRedO promoter comprises nucleotides 452-2017 of SEQ ID NO:8 directly linked to nucleotides 4541-5032 of SEQ ID NO:8; or a nucleotide sequence having about 85%, 86%, 87%, 88%, 89%, 90%, 91%, 92%, 93%, 94%, 95%, 96%, 97%, 98%, or about 99% nucleotide sequence identity to the entire nucleotide sequence of nucleotides 452-2017 of SEQ ID NO:8 directly linked to nucleotides 4541-5032 of SEQ ID NO:8, or
(b) the hRedO promoter comprises the nucleotide sequence of SEQ ID NO:16, or a nucleotide sequence having about 85%, 86%, 87%, 88%, 89%, 90%, 91%, 92%, 93%, 94%, 95%, 96%, 97%, 98%, or about 99% nucleotide sequence identity to the entire nucleotide sequence of SEQ ID NO:16.

3. The composition of any one of claims 1-2, wherein the nucleic acid molecule encoding TXNIP comprises:
(a) nucleotides 366-1541 of SEQ ID NO:111; or a nucleotide sequence having about 85%, 86%, 87%, 88%, 89%, 90%, 91%, 92%, 93%, 94%, 95%, 96%, 97%, 98%, or about 99% nucleotide sequence identity to the entire nucleotide sequence of nucleotides 366-1541 of SEQ ID NO:111; or
(b) nucleotides 162-1172 of SEQ ID NO:112, or a nucleotide sequence having about 85%, 86%, 87%, 88%, 89%, 90%, 91%, 92%, 93%, 94%, 95%, 96%, 97%, 98%, or about 99% nucleotide sequence identity to the entire nucleotide sequence of nucleotides 162-1172 of SEQ ID NO:112; or
(c) nucleotides 280-1473 of SEQ ID NO:113; or a nucleotide sequence having about 85%, 86%, 87%, 88%, 89%, 90%, 91%, 92%, 93%, 94%, 95%, 96%, 97%, 98%, or about 99% nucleotide sequence identity to the entire nucleotide sequence of nucleotides 280-1473 of SEQ ID NO:113; or
(d) nucleotides 280-1470 of SEQ ID NO:114, or a nucleotide sequence having about 85%, 86%, 87%, 88%, 89%, 90%, 91%, 92%, 93%, 94%, 95%, 96%, 97%, 98%, or about 99% nucleotide sequence identity to the entire nucleotide sequence of nucleotides 280-1470 of SEQ ID NO:114; or
(e) the nucleotide sequence of SEQ ID NO: 120; or a nucleotide sequence having about 85%, 86%, 87%, 88%, 89%, 90%, 91%, 92%, 93%, 94%, 95%, 96%, 97%, 98%, or about 99% nucleotide sequence identity to the entire nucleotide sequence of SEQ ID NO:120.

4. The composition of claim 1 or 2, wherein the nucleic acid molecule encodes a C247S .LL351.352AA variant thioredoxin-interacting 5 protein (TXNIP).

5. The composition of claim 4, wherein:
(i) the nucleic acid molecule encoding TXNIP comprises SEQ ID NO: 115; or a nucleotide sequence having about 85%, 86%, 87%, 88%, 89%, 90%, 91%, 92%, 93%, 94%, 95%, 96%, 97%, 98%, or about 99% nucleotide sequence identity to the entire nucleotide sequence of SEQ ID NO:115; or
(ii) the nucleic acid molecule encoding TXNIP comprises SEQ ID NO: 121; or a nucleotide sequence having about 85%, 86%, 87%, 88%, 89%, 90%, 91%, 92%, 93%, 94%, 95%, 96%, 97%, 98%, or about 99% nucleotide sequence identity to the entire nucleotide sequence of SEQ ID NO:121.

6. The composition of any one of claims 1-5, wherein the expression cassette further comprises:
(a) a linker;
(b) an intron;
(c) a post-transcriptional regulatory region;
(d) a Woodchuck hepatitis virus posttranscriptional regulatory element (WPRE); and/or
(e) a polyadenylation signal.

7. The composition of claim 6, wherein:
(a) the linker is a 2A linker; and/or
(b) the polyadenylation signal is a bovine growth hormone polyadenylation signal or and SV40 polyadenylation signal.

8. The composition of any one of claims 1-7, wherein the expression cassette is present in a vector.

9. The composition of claim 8, wherein the vector is an AAV vector selected from the group consisting of AAV2, AAV 8, AAV2/5, and AAV 2/8.

10. An AAV vector particle comprising the composition of any one of claims 1-9.

11. An isolated cell comprising the AAV particle of claim 10.

12. A pharmaceutical composition comprising the AAV composition of any one of claims 8-9 or the particle of claim 10.

13. The pharmaceutical composition of claim 12:
(a) further comprising a viscosity inducing agent; and/or
(b) which is for intraocular administration,
wherein the intraocular administration is selected from the group consisting of intravitreal or subretinal, subvitreal, subconjuctival, sub-tenon, periocular, retrobulbar, suprachoroidal, and/or intrascleral administration.

14. The composition of any one of claims 8-9, the AAV viral particle of claim 10, or the pharmaceutical composition of claim 12 or 13 for use in a method of:
(A) prolonging the viability of a photoreceptor cell compromised by a degenerative ocular disorder, wherein the method comprises contacting said cell with the composition of any one of claims 1-9, the AAV viral particle of claim 10, or the pharmaceutical composition of claim 12 or 13;
(B) treating or preventing a degenerative ocular disorder in a subject;
(C) delaying loss of functional vision in a subject having a degenerative ocular disorder; or
(D) treating or preventing retinitis pigmentosa in a subject.

15. The composition of any one of claims 8-9, the AAV viral particle of claim 10, or the pharmaceutical composition of claim 12 or 13 for use according to claim 14(A)-(C), wherein:
(a) the degenerative ocular disorder is associated with decreased viability of cone cells and/or decreased viability of rod cells;
(b) the degenerative ocular disorder is selected from the group consisting of retinitis pigmentosa, age related macular 5 degeneration, cone rod dystrophy, and rod cone dystrophy;
(c) the degenerative ocular disorder is a genetic disorder;
(d) the degenerative ocular disorder is not associated with blood vessel leakage and/or growth; and/or
(e) the degenerative ocular disorder is retinitis pigmentosa.

16. The composition of any one of claims 8-9, the AAV viral particle of claim 10, or the pharmaceutical composition of claim 12 or 13 for use according to claim 14 or 15, wherein the method further comprises administering to the subject a therapeutically effective amount of a composition according to claim 8 or 9 comprising an adeno-associated virus (AAV) expression cassette, the expression cassette comprising a human bestrophin 1 (hBest1) promoter, a chimeric intron, and a nucleic acid molecule encoding nuclear factor erythroid 2-like 2 (Nrf2), or a pharmaceutical composition comprising a composition according to claim 8 or 9 comprising an adeno-associated virus (AAV) expression cassette, the expression cassette comprising a human red opsin (hRedO) promoter and a nucleic acid molecule encoding transforming growth factor beta 1 (Tgfb 1).

## Patentansprüche

1. Zusammensetzung, umfassend eine Adeno-assoziiertes-Virus(AAV)-Expressionskassette, wobei die Expressionskassette einen Photorezeptor-spezifischen (PR-spezifischen) Promotor und ein Nukleinsäuremolekül umfasst, das für eine C247S-Variante von Thioredoxin-interagierend-5-Protein (TXNIP) codiert, wobei der PRspezifische Promotor ein humaner Red-Opsin(hRedO)-Promotor ist.

2. Zusammensetzung nach Anspruch 1, wobei:
(a) der hRedO-Promotor die Nukleotide 452-2017 von SEQ ID NO:8, die direkt mit den Nukleotiden 4541-5032 von SEQ ID NO:8 verknüpft sind, umfasst; oder eine Nukleotidsequenz mit etwa 85 %, 86 %, 87 %, 88 %, 89 %, 90 %, 91 %, 92 %, 93 %, 94 %, 95 %, 96 %, 97 %, 98 % oder etwa 99 % Nukleotidsequenzidentität mit der gesamten Nukleotidsequenz von Nukleotiden 452-2017 von SEQ ID NO:8, die direkt mit Nukleotiden 4541-5032 von SEQ ID NO:8 verknüpft ist, umfasst, oder
(b) der hRedO-Promotor die Nukleotidsequenz von SEQ ID NO: 16 oder eine Nukleotidsequenz mit etwa 85 %, 86 %, 87 %, 88 %, 89 %, 90 %, 91 %, 92 %, 93 %, 94 %, 95 %, 96 %, 97 %, 98 % oder etwa 99 % Nukleotidsequenzidentität mit der gesamten Nukleotidsequenz von SEQ ID NO: 16 umfasst.

3. Zusammensetzung nach einem der Ansprüche 1-2, wobei das Nukleinsäuremolekül, das TXNIP codiert, umfasst:
(a) Nukleotide 366-1541 von SEQ ID NO:111; oder eine Nukleotidsequenz mit etwa 85 %, 86 %, 87 %, 88 %, 89 %, 90 %, 91 %, 92 %, 93 %, 94 %, 95 %, 96 %, 97 %, 98 % oder etwa 99 % Nukleotidsequenzidentität mit der gesamten Nukleotidsequenz der Nukleotide 366-1541 von SEQ ID NO:111; oder
(b) Nukleotide 162-1172 von SEQ ID NO: 112 oder eine Nukleotidsequenz mit etwa 85 %, 86 %, 87 %, 88 %, 89 %, 90 %, 91 %, 92 %, 93 %, 94 %, 95 %, 96 %, 97 %, 98 % oder etwa 99 % Nukleotidsequenzidentität mit der gesamten Nukleotidsequenz der Nukleotide 162-1172 von SEQ ID NO: 112; oder
(c) Nukleotide 280-1473 von SEQ ID NO: 113; oder eine Nukleotidsequenz mit etwa 85 %, 86 %, 87 %, 88 %, 89 %, 90 %, 91 %, 92 %, 93 %, 94 %, 95 %, 96 %, 97 %, 98 % oder etwa 99 % Nukleotidsequenzidentität mit der gesamten Nukleotidsequenz der Nukleotide 280-1473 von SEQ ID NO: 113; oder
(d) Nukleotide 280-1470 von SEQ ID NO: 114 oder eine Nukleotidsequenz mit etwa 85 %, 86 %, 87 %, 88 %, 89 %, 90 %, 91 %, 92 %, 93 %, 94 %, 95 %, 96 %, 97 %, 98 % oder etwa 99 % Nukleotidsequenzidentität mit der gesamten Nukleotidsequenz der Nukleotide 280-1470 von SEQ ID NO: 114; oder
(e) die Nukleotidsequenz von SEQ ID NO: 120; oder eine Nukleotidsequenz mit etwa 85 %, 86 %, 87 %, 88 %, 89 %, 90 %, 91 %, 92 %, 93 %, 94 %, 95 %, 96 %, 97 %, 98 % oder etwa 99 % Nukleotidsequenzidentität mit der gesamten Nukleotidsequenz von SEQ ID NO: 120.

4. Zusammensetzung nach Anspruch 1 oder 2, wobei das Nukleinsäuremolekül für eine C247S.LL351.352AA-Variante von Thioredoxin-interagierend-5-Protein (TXNIP) codiert.

5. Zusammensetzung nach Anspruch 4, wobei:
(i) das für TXNIP codierende Nukleinsäuremolekül SEQ ID NO: 115; oder eine Nukleotidsequenz mit etwa 85 %, 86 %, 87 %, 88 %, 89 %, 90 %, 91 %, 92 %, 93 %, 94 %, 95 %, 96 %, 97 %, 98 % oder etwa 99 % Nukleotidsequenzidentität mit der gesamten Nukleotidsequenz von SEQ ID NO: 115 umfasst; oder
(ii) das für TXNIP codierende Nukleinsäuremolekül SEQ ID NO: 121; oder eine Nukleotidsequenz mit etwa 85 %, 86 %, 87 %, 88 %, 89 %, 90 %, 91 %, 92 %, 93 %, 94 %, 95 %, 96 %, 97 %, 98 % oder etwa 99 % Nukleotidsequenzidentität mit der gesamten Nukleotidsequenz von SEQ ID NO: 121 umfasst.

6. Zusammensetzung nach einem der Ansprüche 1-5, wobei die Expressionskassette ferner umfasst:
(a) einen Linker;
(b) ein Intron;
(c) eine posttranskriptionale regulatorische Region;
(d) ein posttranskriptionales regulatorisches Woodchuck-Hepatitisvirus-Element (WPRE); und/oder
(e) ein Polyadenylierungssignal.

7. Zusammensetzung nach Anspruch 6, wobei:
(a) der Linker ein 2A-Linker ist; und/oder
(b) das Polyadenylierungssignal ein Rinderwachstumshormon-Polyadenylierungssignal oder/und ein SV40-Polyadenylierungssignal ist.

8. Zusammensetzung nach einem der Ansprüche 1-7, wobei die Expressionskassette in einem Vektor vorhanden ist.

9. Zusammensetzung nach Anspruch 8, wobei der Vektor ein AAV-Vektor ist, der aus der Gruppe ausgewählt ist, die aus AAV2, AAV 8, AAV2/5 und AAV 2/8 besteht.

10. AAV-Vektorpartikel, umfassend die Zusammensetzung nach einem der Ansprüche 1-9.

11. Isolierte Zelle, die das AAV-Partikel nach Anspruch 10 umfasst.

12. Pharmazeutische Zusammensetzung, umfassend die AAV-Zusammensetzung nach einem der Ansprüche 8-9 oder das Partikel nach Anspruch 10.

13. Pharmazeutische Zusammensetzung nach Anspruch 12:
(a) ferner umfassend ein viskositätsinduzierendes Mittel; und/oder
(b) die zur intraokularen Anwendung bestimmt ist, wobei die intraokulare Verabreichung ausgewählt ist aus der Gruppe bestehend aus intravitrealer oder subretinaler, subvitrealer, subkonjuktivaler, subtenonaler, periokularer, retrobulbärer, suprachoroidaler und/oder intraskleraler Verabreichung.

14. Zusammensetzung nach einem der Ansprüche 8-9, AAV-Viruspartikel nach Anspruch 10 oder pharmazeutische Zusammensetzung nach Anspruch 12 oder 13 zur Verwendung in einem Verfahren zur:
(a) Verlängerung der Lebensfähigkeit einer durch eine degenerative Augenerkrankung beeinträchtigten Photorezeptorzelle, wobei das Verfahren das Inkontaktbringen der Zelle mit der Zusammensetzung nach einem der Ansprüche 1-9, dem AAV-Viruspartikel nach Anspruch 10 oder der pharmazeutischen Zusammensetzung nach Anspruch 12 oder 13 umfasst;
(b) Behandlung oder Prävention einer degenerativen Augenerkrankung bei einem Individuum;
(c) Verzögerung des Verlusts des funktionellen Sehvermögens bei einem Individuum mit einer degenerativen Augenerkrankung; oder
(d) Behandlung oder Prävention von Retinitis pigmentosa bei einem Individuum.

15. Zusammensetzung nach einem der Ansprüche 8-9, AAV-Viruspartikel nach Anspruch 10 oder pharmazeutische Zusammensetzung nach Anspruch 12 oder 13 zur Verwendung gemäß Anspruch 14(A)-(C), wobei:
(a) die degenerative Augenerkrankung mit einer verminderten Lebensfähigkeit von Kegelzellen und/oder einer verminderten Lebensfähigkeit von Stabzellen assoziiert ist;
(b) die degenerative Augenerkrankung ausgewählt ist aus der Gruppe bestehend aus Retinitis pigmentosa, altersbedingter Makuladegeneration 5, Kegelstabdystrophie und Stabkegeldystrophie;
(c) die degenerative Augenerkrankung eine genetische Erkrankung ist;
(d) die degenerative Augenerkrankung nicht mit Blutgefäßleckage und/oder -wachstum assoziiert ist; und/oder
(e) die degenerative Augenerkrankung Retinitis pigmentosa ist.

16. Zusammensetzung nach einem der Ansprüche 8-9, AAV-Viruspartikel nach Anspruch 10 oder pharmazeutische Zusammensetzung nach Anspruch 12 oder 13 zur Verwendung gemäß Anspruch 14 oder 15, wobei das Verfahren ferner das Verabreichen einer therapeutisch wirksamen Menge einer Zusammensetzung nach Anspruch 8 oder 9, umfassend eine Adeno-assoziierte Virus(AAV)-Expressionskassette, an das Subjekt umfasst, wobei die Expressionskassette einen humanen Bestrophin 1 (hBest1)-Promotor, ein chimäres Bestintron und ein Nukleinsäuremolekül umfasst, das Nukleärfaktor-Erythroid-2-like 2 (Nrf2) codiert, oder einer pharmazeutischen Zusammensetzung, umfassend eine Zusammensetzung nach Anspruch 8 oder 9, die eine Adeno-assoziiertes-Virus(AAV)-Expressionskassette umfasst, wobei die Expressionskassette einen humanen Red-Opsin(hRedO)-Promotor und ein Nukleinsäuremolekül umfasst, das für Transformierender-Wachstumsfaktor-beta 1 (Tgfb1) codiert.

## Revendications

1. Composition, comprenant une cassette d'expression de virus adéno-associé (AAV), la cassette d'expression comprenant un promoteur spécifique du photorécepteur (spécifique du PR) et une molécule d'acide nucléique codant pour une protéine 5 variant C247S interagissant avec la thiorédoxine (TXNIP), dans laquelle le promoteur spécifique du PR est un promoteur de l'opsine rouge humaine (hRedO).

2. Composition de la revendication 1, dans laquelle :
(a) le promoteur de hRedO comprend les nucléotides 452-2017 de SEQ ID NO:8 directement liés aux nucléotides 4541-5032 de SEQ ID NO:8 ; ou une séquence nucléotidique ayant environ 85 %, 86 %, 87 %, 88 %, 89 %, 90 %, 91 %, 92 %, 93 %, 94 %, 95 %, 96 %, 97 %, 98 %, ou environ 99 % d'identité de séquence nucléotidique avec la séquence nucléotidique entière des nucléotides 452-2017 de SEQ ID NO: 8 directement liés aux nucléotides 4541-5032 de SEQ ID NO: 8, ou
(b) le promoteur de hRedO comprend la séquence nucléotidique de SEQ ID NO: 16, ou une séquence nucléotidique ayant environ 85 %, 86 %, 87 %, 88 %, 89 %, 90 %, 91 %, 92 %, 93 %, 94 %, 95 %, 96 %, 97 %, 98 %, ou environ 99 % d'identité de séquence nucléotidique avec la séquence nucléotidique entière de SEQ ID NO: 16.

3. Composition selon l'une quelconque des revendications 1 à 2, dans laquelle la molécule d'acide nucléique codant pour TXNIP comprend :
(a) les nucléotides 366-1541 de SEQ ID NO: 111 ; ou une séquence nucléotidique ayant environ 85 %, 86 %, 87 %, 88 %, 89 %, 90 %, 91 %, 92 %, 93 %, 94 %, 95 %, 96 %, 97 %, 98 %, ou environ 99 % d'identité de séquence nucléotidique avec la séquence nucléotidique entière des nucléotides 366-1541 de SEQ ID NO: 111 ; ou
(b) les nucléotides 162-1172 de SEQ ID NO: 112, ou une séquence nucléotidique ayant environ 85 %, 86 %, 87 %, 88 %, 89 %, 90 %, 91 %, 92 %, 93 %, 94 %, 95 %, 96 %, 97 %, 98 %, ou environ 99 % d'identité de séquence nucléotidique avec la séquence nucléotidique entière des nucléotides 162-1172 de SEQ ID NO: 112 ; ou
(c) les nucléotides 280-1473 de SEQ ID NO: 113 ; ou une séquence nucléotidique ayant environ 85 %, 86 %, 87 %, 88 %, 89 %, 90 %, 91 %, 92 %, 93 %, 94 %, 95 %, 96 %, 97 %, 98 %, ou environ 99 % d'identité de séquence nucléotidique avec la séquence nucléotidique entière des nucléotides 280-1473 de SEQ ID NO: 113 ; ou
(d) les nucléotides 280-1470 de SEQ ID NO: 114, ou une séquence nucléotidique ayant environ 85 %, 86 %, 87 %, 88 %, 89 %, 90 %, 91 %, 92 %, 93 %, 94 %, 95 %, 96 %, 97 %, 98 %, ou environ 99 % d'identité de séquence nucléotidique avec la séquence nucléotidique entière des nucléotides 280-1470 de SEQ ID NO: 114 ; ou
(e) la séquence nucléotidique de SEQ ID NO: 120 ; ou une séquence nucléotidique ayant environ 85 %, 86 %, 87 %, 88 %, 89 %, 90 %, 91 %, 92 %, 93 %, 94 %, 95 %, 96 %, 97 %, 98 %, ou environ 99 % d'identité de séquence nucléotidique avec la séquence nucléotidique entière de SEQ ID NO: 120.

4. Composition selon la revendication 1 ou 2, dans laquelle la molécule d'acide nucléique code pour une protéine 5 variant C247S .LL351.352AA interagissant avec la thiorédoxine (TXNIP).

5. Composition de la revendication 4, dans laquelle :
(i) la molécule d'acide nucléique codant pour TXNIP comprend SEQ ID NO: 115 ; ou une séquence nucléotidique ayant environ 85 %, 86 %, 87 %, 88 %, 89 %, 90 %, 91 %, 92 %, 93 %, 94 %, 95 %, 96 %, 97 %, 98 %, ou environ 99 % d'identité de séquence nucléotidique avec la séquence nucléotidique entière de SEQ ID NO: 115 ; ou
(ii) la molécule d'acide nucléique codant pour TXNIP comprend SEQ ID NO: 121 ; ou une séquence nucléotidique ayant environ 85 %, 86 %, 87 %, 88 %, 89 %, 90 %, 91 %, 92 %, 93 %, 94 %, 95 %, 96 %, 97 %, 98 %, ou environ 99 % d'identité de séquence nucléotidique avec la séquence nucléotidique entière de SEQ ID NO: 121.

6. Composition selon l'une quelconque des revendications 1 à 5, dans laquelle la cassette d'expression comprend en outre :
(a) un lieur ;
(b) un intron ;
(c) une région régulatrice post-transcriptionnelle ;
(d) un élément régulateur post-transcriptionnel du virus de l'hépatite Woodchuck (WPRE) ; et/ou
(e) un signal de polyadénylation.

7. Composition de la revendication 6, dans laquelle :
(a) le lieur est un lieur 2A ; et/ou
(b) le signal de polyadénylation est un signal de polyadénylation de l'hormone de croissance bovine ou/et un signal de polyadénylation SV40.

8. Composition selon l'une quelconque des revendications 1 à 7, dans laquelle la cassette d'expression est présente dans un vecteur.

9. Composition selon la revendication 8, dans laquelle le vecteur est un vecteur AAV choisi dans le groupe constitué par AAV2, AAV 8, AAV2/5 et AAV 2/8.

10. Particule de vecteur AAV comprenant la composition selon l'une quelconque des revendications 1 à 9.

11. Cellule isolée comprenant la particule AAV de la revendication 10.

12. Composition pharmaceutique comprenant la composition AAV selon l'une quelconque des revendications 8-9 ou la particule selon la revendication 10.

13. Composition pharmaceutique selon la revendication 12 :
(a) comprenant en outre un agent inducteur de viscosité ; et/ou
(b) qui est destiné à une administration intraoculaire, dans laquelle l'administration intraoculaire est choisie dans le groupe constitué par une administration intravitréenne ou sous-rétinienne, sous-vitréenne, sous-conjonctivale, sous-ténonienne, périoculaire, rétrobulbaire, suprachoroïdienne et/ou intrasclérale.

14. Composition selon l'une quelconque des revendications 8 à 9, particule virale AAV selon la revendication 10, ou composition pharmaceutique selon la revendication 12 ou 13 pour une utilisation dans un procédé de :
(A) prolongation de la viabilité d'une cellule de photorécepteur compromise par un trouble oculaire dégénératif, le procédé comprenant la mise en contact de ladite cellule avec la composition selon l'une quelconque des revendications 1 à 9, la particule virale AAV selon la revendication 10, ou la composition pharmaceutique selon la revendication 12 ou 13 ;
(B) traitement ou prévention d'un trouble oculaire dégénératif chez un sujet ;
(C) retardement de la perte de vision fonctionnelle chez un sujet ayant un trouble oculaire dégénératif ; ou
(D) traitement ou prévention de la rétinite pigmentaire chez un sujet.

15. Composition selon l'une quelconque des revendications 8 à 9, particule virale AAV selon la revendication 10, ou composition pharmaceutique selon la revendication 12 ou 13 pour une utilisation selon la revendication 14(A)-(C), dans laquelle :
(a) le trouble oculaire dégénératif est associé à une diminution de la viabilité des cônes et/ou à une diminution de la viabilité des bâtonnets ;
(b) le trouble oculaire dégénératif est choisi dans le groupe constitué de la rétinite pigmentaire, la dégénérescence 5 maculaire liée à l'âge, la dystrophie cône-bâtonnet et la dystrophie bâtonnet-cône ;
(c) le trouble oculaire dégénératif est un trouble génétique ;
(d) le trouble oculaire dégénératif n'est pas associé à une fuite et/ou croissance de vaisseau sanguin ; et/ou
(e) le trouble oculaire dégénératif est la rétinite pigmentaire.

16. Composition selon l'une quelconque des revendications 8 à 9, particule virale AAV selon la revendication 10, ou composition pharmaceutique selon la revendication 12 ou 13 pour une utilisation selon la revendication 14 ou 15, le procédé comprenant en outre l'administration au sujet d'une quantité thérapeutiquement efficace d'une composition selon la revendication 8 ou 9 comprenant une cassette d'expression de virus adéno-associé (AAV), la cassette d'expression comprenant un promoteur de bestrophine 1 humaine (hBest1), un intron chimérique, et une molécule d'acide nucléique codant pour le facteur nucléaire (dérivé de l'érythroïde 2)-like 2 (Nrf2), ou une composition pharmaceutique comprenant une composition selon la revendication 8 ou 9 comprenant une cassette d'expression de virus adéno-associé (AAV), la cassette d'expression comprenant un promoteur d'opsine rouge humaine (hRedO) et une molécule d'acide nucléique codant pour le facteur de croissance transformant bêta 1 (Tgfb1).
